# EUROPEAN PATENT APPLICATION

(11) **EP 4 707 273 A2**
(43) Date of publication of application: **11.03.2026**
(21) Application number: 26151568.8
(22) Date of filing: 27.10.2022
(51) Int. Cl.: C07D 215/14

(54) **POLYCYCLIC COMPOUND AND LIGHT EMITTING DEVICE INCLUDING THE SAME**

(30) Priority: 29.10.2021 KR 20210147289
(62) Divisional of application: 22204056.0
(71) Applicant: Samsung Display Co., Ltd., Yongin-si, Gyeonggi-do 17113 (KR)
(72) Inventor: KIM, Seulong, Cheonan-si (KR); PARK, Eungyoung, Hwaseong-si (KR); SONG, Hajin, Hwaseong-si (KR); LEE, Jongwon, Seongnam-si (KR); HWANG, Jaehoon, Seoul (KR)
(74) Representative: Crow, Martin

(57) **Abstract**

A polycyclic compound represented by Formula 1: 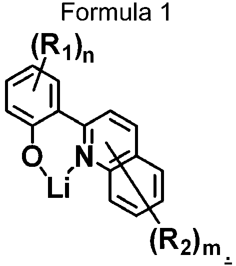 and a light emitting device including a first electrode, a hole transport region on the first electrode, an emission layer on the hole transport region, an electron transport region on the emission layer, and a second electrode on the electron transport region are provided. The electron transport region includes a polycyclic compound represented by Formula 1.

## Description

### CROSS-REFERENCE TO RELATED APPLICATION

This application claims priority to and the benefit of Korean Patent Application No. 10-2021-0147289, filed on October 29, 2021, the entire content of which is hereby incorporated by reference.

### BACKGROUND

Aspects of one or more embodiments of the present disclosure relate to a polycyclic compound and a light emitting device including the same, and for example, to a light emitting device including a plurality of materials such as a polycyclic compound utilized as an electron transport layer material.

Recently, the development of an electroluminescence display apparatus as an image display apparatus is being actively conducted. Unlike a liquid crystal display apparatus, etc., the luminescence display is a self-luminescent display in which holes and electrons injected from a first electrode and a second electrode recombine in an emission layer, and thus a luminescent material including an organic compound in the emission layer emits light to attain display.

### SUMMARY

The invention is defined by the claims.

An aspect of one or more embodiments of the present disclosure is a polycyclic compound represented by Formula 1:

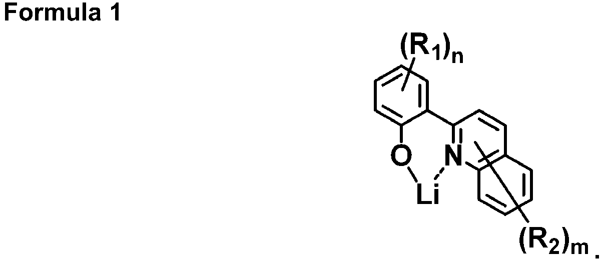

The polycyclic compounds of the invention are capable of improving luminous efficiency and device service life (increases lifetime of device) of a light emitting device.

An aspect of one or more embodiments of the present disclosure provides a light emitting device comprising a first electrode, a hole transport region on the first electrode, an emission layer on the hole transport region, an electron transport region on the emission layer, and a second electrode on the electron transport region, wherein the electron transport region comprises a polycyclic compound described herein.

In Formula 1, R₁ and R₂ may each independently be a hydrogen atom, a deuterium atom, a halogen atom, a substituted or unsubstituted silyl group, a substituted or unsubstituted amine group, a substituted or unsubstituted alkyl group having 1 to 20 carbon atoms, a substituted or unsubstituted alkenyl group having 2 to 20 carbon atoms, a substituted or unsubstituted aryl group having 6 to 30 ring-forming carbon atoms, or a substituted or unsubstituted heteroaryl group having 2 to 30 ring-forming carbon atoms, and optionally may be bonded to an adjacent group to form a ring, and n is an integer of 0 to 4, and m is an integer of 0 to 6.

For example, m may be 0 or 1, preferably 0. For example, n may be 1 or 2, preferably 1.

In an embodiment, the polycyclic compound may be represented by Formula 1-1:

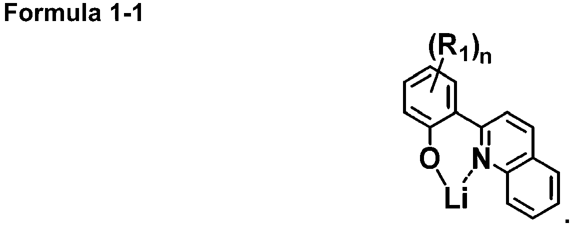

In Formula 1-1 above, R₁ and n may each independently be the same as defined in Formula 1.

In an embodiment, the polycyclic compound may be represented by Formula 1-2:

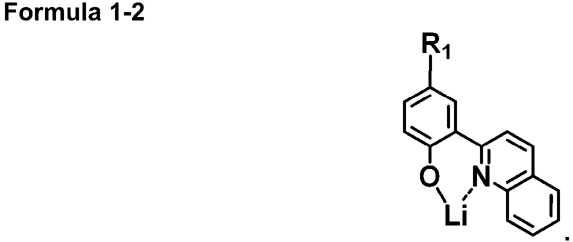

In Formula 1-2, R₁ is the same as defined in Formula 1.

In an embodiment, R₁ may be a substituted or unsubstituted alkyl group having 1 to 20 carbon atoms. For example, R₁ may be a substituted or unsubstituted alkyl group having 1 to 10 carbon atoms or 1 to 5 carbon atoms. For example, R₁ may be an unsubstituted alkyl group having 1 to 10 carbon atoms or 1 to 5 carbon atoms.

In an embodiment, the polycyclic compound comprises at least one compound represented in Compound Group 1:

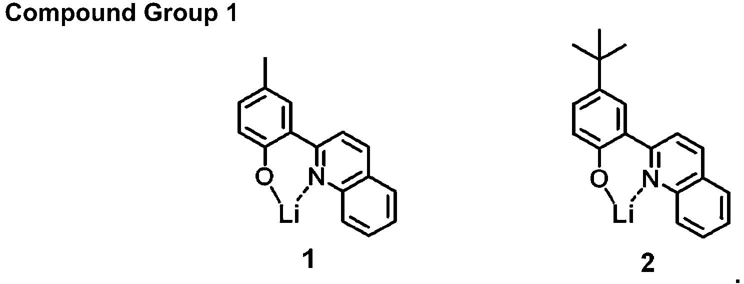

In an embodiment, the electron transport region may further comprise at least one compound represented by Formula 2 and/or Formula 3:

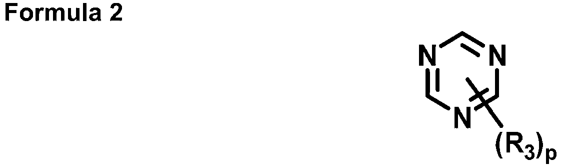

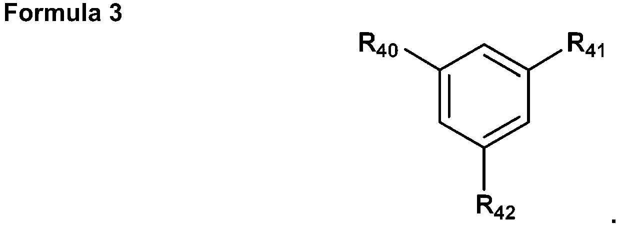

In Formula 2, each R₃ is independently a hydrogen atom, a deuterium atom, a halogen atom, a substituted or unsubstituted silyl group, a substituted or unsubstituted amine group, a substituted or unsubstituted alkyl group having 1 to 20 carbon atoms, a substituted or unsubstituted alkenyl group having 2 to 20 carbon atoms, a substituted or unsubstituted aryl group having 6 to 30 ring-forming carbon atoms, or a substituted or unsubstituted heteroaryl group having 2 to 30 ring-forming carbon atoms, and optionally may be bonded to an adjacent group to form a ring, and p is an integer of 0 to 3.

In Formula 2, each R₃ may independently be a hydrogen atom, a deuterium atom, a halogen atom, a substituted or unsubstituted silyl group, a substituted or unsubstituted amine group, a substituted or unsubstituted alkyl group having 1 to 20 carbon atoms, a substituted or unsubstituted alkenyl group having 2 to 20 carbon atoms, a substituted or unsubstituted aryl group having 6 to 30 ring-forming carbon atoms, or a substituted or unsubstituted heteroaryl group having 2 to 30 ring-forming carbon atoms, and p is an integer of 0 to 3.

In Formula 2, when two R₃s are independently a substituted or unsubstituted silyl group, a substituted or unsubstituted amine group, a substituted or unsubstituted alkyl group having 1 to 20 carbon atoms, a substituted or unsubstituted alkenyl group having 2 to 20 carbon atoms, a substituted or unsubstituted aryl group having 6 to 30 ring-forming carbon atoms, or a substituted or unsubstituted heteroaryl group having 2 to 30 ring-forming carbon atoms, the two R₃s may be bonded together to form a ring.

In Formula 3, R₄₀ to R₄₂ may each independently be a hydrogen atom, a deuterium atom, a halogen atom, a cyano group, a substituted or unsubstituted silyl group, a substituted or unsubstituted thiol group, a substituted or unsubstituted oxy group, a substituted or unsubstituted amine group, a substituted or unsubstituted alkyl group having 1 to 20 carbon atoms, a substituted or unsubstituted alkenyl group having 2 to 20 carbon atoms, a substituted or unsubstituted aryl group having 6 to 30 ring-forming carbon atoms, or a substituted or unsubstituted heteroaryl group having 2 to 30 ring-forming carbon atoms, and optionally may be bonded to an adjacent group to form a ring.

In Formula 3, R₄₀ to R₄₂ may each independently be a hydrogen atom, a deuterium atom, a halogen atom, a cyano group, a substituted or unsubstituted silyl group, a substituted or unsubstituted thiol group, a substituted or unsubstituted oxy group, a substituted or unsubstituted amine group, a substituted or unsubstituted alkyl group having 1 to 20 carbon atoms, a substituted or unsubstituted alkenyl group having 2 to 20 carbon atoms, a substituted or unsubstituted aryl group having 6 to 30 ring-forming carbon atoms, or a substituted or unsubstituted heteroaryl group having 2 to 30 ring-forming carbon atoms.

In Formula 3, when two of R₄₀ to R₄₂ are independently a substituted or unsubstituted silyl group, a substituted or unsubstituted thiol group, a substituted or unsubstituted oxy group, a substituted or unsubstituted amine group, a substituted or unsubstituted alkyl group having 1 to 20 carbon atoms, a substituted or unsubstituted alkenyl group having 2 to 20 carbon atoms, a substituted or unsubstituted aryl group having 6 to 30 ring-forming carbon atoms, or a substituted or unsubstituted heteroaryl group having 2 to 30 ring-forming carbon atoms, the two of R₄₀ to R₄₂ may be bonded together to form a ring.

In an embodiment, the electron transport region may comprise a hole blocking layer disposed on the emission layer, an electron transport layer disposed on the hole blocking layer, and an electron injection layer disposed on the electron transport layer, wherein the electron transport layer may comprise the polycyclic compound described herein.

In an embodiment, the electron transport layer may have a thickness of about 250 Å or more. For example, the electron transport layer may have a thickness of about 300 Å or more, or about 350 Å or more.

In an embodiment, the electron transport layer may have a refractive index of less than about 1.8.

In an embodiment, the emission layer may emit light having a center wavelength of about 430 nm to about 470 nm.

In an embodiment of the present disclosure, a light emitting device comprises a first electrode; a second electrode on the first electrode; and at least one functional layer between the first electrode and the second electrode, wherein the at least one functional layer comprises at least one of a polycyclic compound represented by Formula 1, a compound represented by Formula 2, and/or a compound represented by Formula 3, and the at least one functional layer has a thickness of about 250 Å or more.

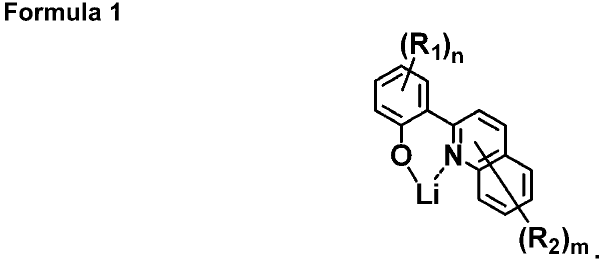

In an embodiment of the present disclosure, a light emitting device comprises a first electrode; a second electrode on the first electrode; and at least one functional layer between the first electrode and the second electrode, wherein the at least one functional layer comprises (i) a polycyclic compound represented by Formula 1, and (ii) a compound represented by Formula 2, and/or a compound represented by Formula 3. The at least one functional layer may be an electron transport region/layer. The at least one functional layer may have a thickness of about 250 Å or more.

In Formula 1, R₁ and R₂ may each independently be a hydrogen atom, a deuterium atom, a halogen atom, a substituted or unsubstituted silyl group, a substituted or unsubstituted amine group, a substituted or unsubstituted alkyl group having 1 to 20 carbon atoms, a substituted or unsubstituted alkenyl group having 2 to 20 carbon atoms, a substituted or unsubstituted aryl group having 6 to 30 ring-forming carbon atoms, or a substituted or unsubstituted heteroaryl group having 2 to 30 ring-forming carbon atoms, and optionally may be bonded to an adjacent group to form a ring, and n is an integer of 0 to 4, and m is an integer of 0 to 6:

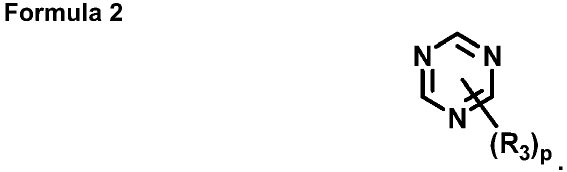

In Formula 2, each R₃ is independently a hydrogen atom, a deuterium atom, a halogen atom, a substituted or unsubstituted silyl group, a substituted or unsubstituted amine group, a substituted or unsubstituted alkyl group having 1 to 20 carbon atoms, a substituted or unsubstituted alkenyl group having 2 to 20 carbon atoms, a substituted or unsubstituted aryl group having 6 to 30 ring-forming carbon atoms, or a substituted or unsubstituted heteroaryl group having 2 to 30 ring-forming carbon atoms, and optionally may be bonded to an adjacent group to form a ring, and p is an integer of 0 to 3:

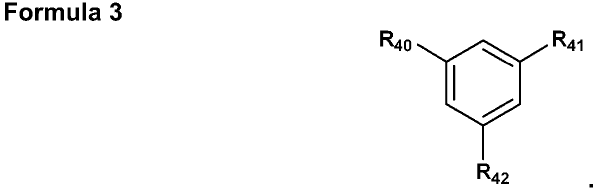

In Formula 3, R₄₀ to R₄₂ may each independently be a hydrogen atom, a deuterium atom, a halogen atom, a cyano group, a substituted or unsubstituted silyl group, a substituted or unsubstituted thiol group, a substituted or unsubstituted oxy group, a substituted or unsubstituted amine group, a substituted or unsubstituted alkyl group having 1 to 20 carbon atoms, a substituted or unsubstituted alkenyl group having 2 to 20 carbon atoms, a substituted or unsubstituted aryl group having 6 to 30 ring-forming carbon atoms, or a substituted or unsubstituted heteroaryl group having 2 to 30 ring-forming carbon atoms, and optionally may be bonded to an adjacent group to form a ring.

In an embodiment, the at least one functional layer may comprise the polycyclic compound described herein and/or the compound represented by Formula 2.

In an embodiment, the at least one functional layer may comprise the polycyclic compound described herein and/or the compound represented by Formula 3.

In an embodiment, the at least one functional layer may comprise a hole transport layer, an emission layer, and/or an electron transport layer, and the electron transport layer may comprise the polycyclic compound described herein, and/or at least one of the compounds represented by Formula 2 or the compounds represented by Formula 3.

In an embodiment, the at least one functional layer may comprise a hole transport layer, an emission layer, and/or an electron transport layer, and the electron transport layer may comprise the polycyclic compound described herein, and at least one of the compounds represented by Formula 2 or the compounds represented by Formula 3.

In an embodiment, the electron transport layer may have a thickness of about 250 Å or more.

In an embodiment, the electron transport layer may have a refractive index of less than about 1.8.

In an embodiment, in the polycyclic compound described herein, R₁ may be a substituted or unsubstituted alkyl group having 1 to 20 carbon atoms, R₂ may be a hydrogen atom, and n may be 1.

### BRIEF DESCRIPTION OF THE DRAWINGS

The above and other aspects and features of certain embodiments of the disclosure will be more apparent from the following description taken in conjunction with the accompanying drawings, in which:
FIG. 1 is a plan view illustrating a display apparatus according to an embodiment of the present disclosure;
FIG. 2 is a cross-sectional view of a display apparatus according to an embodiment of the present disclosure;
FIG. 3 is a cross-sectional view schematically illustrating a light emitting device according to an embodiment of the present disclosure;
FIG. 4 is a cross-sectional view schematically illustrating a light emitting device according to an embodiment of the present disclosure;
FIG. 5 is a cross-sectional view schematically illustrating a light emitting device according to an embodiment of the present disclosure;
FIG. 6 is a cross-sectional view schematically illustrating a light emitting device according to an embodiment of the present disclosure;
FIG. 7 is a cross-sectional view of a display apparatus according to an embodiment of the present disclosure;
FIG. 8 is a cross-sectional view of a display apparatus according to an embodiment of the present disclosure;
FIG. 9 is a cross-sectional view illustrating a display apparatus according to an embodiment of the present disclosure; and
FIG. 10 is a cross-sectional view illustrating a display apparatus according to an embodiment of the present disclosure.

### DETAILED DESCRIPTION

The present disclosure may be modified in many alternate suitable forms, and thus specific embodiments will be exemplified in the drawings and described in more detail. It should be understood, however, that it is not intended to limit the present disclosure to the particular forms disclosed, but rather, is intended to cover all modifications and alternatives falling within the scope of the present disclosure.

When explaining each of drawings, like reference numbers are utilized for referring to like elements, and duplicative descriptions thereof may not be provided. In the accompanying drawings, the dimensions of each structure may be exaggeratingly illustrated for clarity of the present disclosure. It will be understood that, although the terms "first", "second", etc. may be utilized herein to describe one or more suitable elements, these elements should not be limited by these terms. These terms are only utilized to distinguish one element from another. For example, a first element could be termed a second element, and, similarly, a second element could be termed a first element, without departing from the scope of the present disclosure. As utilized herein, the singular forms, "a," "an," and "the" are intended to include the plural forms as well, unless the context clearly indicates otherwise.

In the present application, it will be understood that the terms "include," "have" etc., specify the presence of a feature, a fixed number, a step, an operation, an element, a component, or a combination thereof disclosed in the specification, but do not exclude the possibility of presence or addition of one or more other features, fixed numbers, steps, operations, elements, components, or combination thereof.

In the present application, when a part such as a layer, a film, a region, or a plate is referred to as being "on" or "above" another part, it can be directly on the other part, or an intervening part may also be present. In contrast, when a part such as a layer, a film, a region, or a plate is referred to as being "under" or "below" another part, it can be directly under the other part, or an intervening part may also be present. In some embodiments, it will be understood that when a part is referred to as being "on" another part, it can be disposed on the other part, or disposed under the other part as well.

In the specification, the term "substituted or unsubstituted" may refer to substituted or unsubstituted with at least one substituent selected from the group consisting of a deuterium atom, a halogen atom, a cyano group, a nitro group, an amino group, a silyl group, an oxy group, a thio group, a sulfinyl group, a sulfonyl group, a carbonyl group, a boron group, a phosphine oxide group, a phosphine sulfide group, an alkyl group, an alkenyl group, an alkynyl group, an alkoxy group, a hydrocarbon ring group, an aryl group, and a heterocyclic group. In some embodiments, each of the substituents exemplified above may be substituted or unsubstituted. For example, a biphenyl group may be interpreted as an aryl group or a phenyl group substituted with a phenyl group.

In the specification, the phrase "bonded to an adjacent group to form a ring" may indicate that one is bonded to an adjacent group to form a substituted or unsubstituted hydrocarbon ring, or a substituted or unsubstituted heterocycle. The hydrocarbon ring includes an aliphatic hydrocarbon ring and an aromatic hydrocarbon ring. The heterocycle includes an aliphatic heterocycle and an aromatic heterocycle. The hydrocarbon ring and the heterocycle may be monocyclic or polycyclic. In some embodiments, the rings formed by being bonded to each other may be connected to another ring to form a spiro structure.

In the specification, the term "adjacent group" may refer to a substituent substituted for an atom which is directly linked to an atom substituted with a corresponding substituent, another substituent substituted for an atom which is substituted with a corresponding substituent, or a substituent sterically positioned at the nearest position to a corresponding substituent. For example, two methyl groups in 1,2-dimethylbenzene may be interpreted as "adjacent groups" to each other and two ethyl groups in 1,1-diethylcyclopentane may be interpreted as "adjacent groups" to each other. In some embodiments, two vinyl groups in 1,2-divinylbenzene may be interpreted as "adjacent groups" to each other.

In the specification, examples of the halogen atom may include a fluorine atom, a chlorine atom, a bromine atom, or an iodine atom.

In the specification, the alkyl group may be a linear, branched or cyclic type or kind. The number of carbons in the alkyl group is 1 to 50, 1 to 30, 1 to 20, 1 to 10, or 1 to 6. Examples of the alkyl group may include a methyl group, an ethyl group, an n-propyl group, an isopropyl group, an n-butyl group, an s-butyl group, a t-butyl group, an i-butyl group, a 2-ethylbutyl group, a 3,3-dimethylbutyl group, an n-pentyl group, an i-pentyl group, a neopentyl group, a t-pentyl group, a cyclopentyl group, a 1-methylpentyl group, a 3-methylpentyl group, a 2-ethylpentyl group, a 4-methyl-2-pentyl group, an n-hexyl group, a 1-methylhexyl group, a 2-ethylhexyl group, a 2-butylhexyl group, a cyclohexyl group, a 4-methylcyclohexyl group, a 4-t-butylcyclohexyl group, an n-heptyl group, a 1-methylheptyl group, a 2,2-dimethylheptyl group, a 2-ethylheptyl group, a 2-butylheptyl group, an n-octyl group, a t-octyl group, a 2-ethyloctyl group, a 2-butyloctyl group, a 2-hexyloctyl group, a 3,7-dimethyloctyl group, a cyclooctyl group, an n-nonyl group, an n-decyl group, an adamantyl group, a 2-ethyldecyl group, a 2-butyldecyl group, a 2-hexyldecyl group, a 2-octyldecyl group, an n-undecyl group, an n-dodecyl group, a 2-ethyldodecyl group, a 2-butyldodecyl group, a 2-hexyldocecyl group, a 2-octyldodecyl group, an n-tridecyl group, an n-tetradecyl group, an n-pentadecyl group, an n-hexadecyl group, a 2-ethylhexadecyl group, a 2-butylhexadecyl group, a 2-hexylhexadecyl group, a 2-octylhexadecyl group, an n-heptadecyl group, an n-octadecyl group, an n-nonadecyl group, an n-eicosyl group, a 2-ethyleicosyl group, a 2-butyleicosyl group, a 2-hexyleicosyl group, a 2-octyleicosyl group, an n-henicosyl group, an n-docosyl group, an n-tricosyl group, an n-tetracosyl group, an n-pentacosyl group, an n-hexacosyl group, an n-heptacosyl group, an n-octacosyl group, an n-nonacosyl group, an n-triacontyl group, etc., but the embodiment of the present disclosure is not limited thereto.

In the specification, a cycloalkyl group may refer to a cyclic alkyl group. The number of carbons in the cycloalkyl group is 3 to 50, 3 to 30, 3 to 20, or 3 to 10. Examples of the cycloalkyl group may include a cyclopropyl group, a cyclobutyl group, a cyclopentyl group, a cyclohexyl group, a 4-methylcyclohexyl group, a 4-t-butylcyclohexyl group, a cycloheptyl group, a cyclooctyl group, a cyclononyl group, a cyclodecyl group, a norbornyl group, a 1-adamantyl group, a 2-adamantyl group, an isobornyl group, a bicycloheptyl group, etc., but the embodiment of the present disclosure is not limited thereto.

In the specification, the alkyl group may include an aryl alkyl group. The aryl alkyl group may refer to that an aryl group is bonded to the alkyl group defined above. Examples of the aryl alkyl group may include a toluyl group, a chrysyl group, a cumenyl group, a mesityl group, a benzyl group, a phenethyl group, a styryl group, etc., but the embodiment of the present disclosure is not limited thereto.

In the specification, an alkenyl group refers to a hydrocarbon group including at least one carbon double bond in the middle or terminal of an alkyl group having 2 or more carbon atoms. The alkenyl group may be linear or branched. The carbon number is not specifically limited, but is 2 to 30, 2 to 20 or 2 to 10. Examples of the alkenyl group include a vinyl group, a 1-butenyl group, a 1-pentenyl group, a 1,3-butadienyl aryl group, a styrenyl group, a styrylvinyl group, etc., without limitation.

In the specification, an alkynyl group refers to a hydrocarbon group including at least one carbon triple bond in the middle or terminal of an alkyl group having 2 or more carbon atoms. The alkynyl group may be linear or branched. The carbon number is not limited, but is 2 to 30, 2 to 20 or 2 to 10. Examples of the alkynyl group include an ethynyl group, a propynyl group, etc., without limitation.

The hydrocarbon ring group herein refers to any suitable functional group or substituent derived from an aliphatic hydrocarbon ring. The hydrocarbon ring group may be a saturated hydrocarbon ring group having 5 to 20 ring-forming carbon atoms.

In the disclosure, an aryl group refers to any functional group or substituent derived from an aromatic hydrocarbon ring. The aryl group may be a monocyclic aryl group or a polycyclic aryl group. The number of ring-forming carbon atoms in the aryl group may be 6 to 30, 6 to 20, or 6 to 15. Examples of the aryl group may include a phenyl group, a naphthyl group, a fluorenyl group, an anthracenyl group, a phenanthryl group, a biphenyl group, a terphenyl group, a quaterphenyl group, a quinquephenyl group, a sexiphenyl group, a triphenylenyl group, a pyrenyl group, a benzofluoranthenyl group, a chrysenyl group, etc., but the embodiment of the present disclosure is not limited thereto.

In the disclosure, the fluorenyl group may be substituted, and two substituents may be bonded to each other to form a spiro structure. Examples of embodiments in which the fluorenyl group is substituted are as follows. However, the embodiment of the present disclosure is not limited thereto.

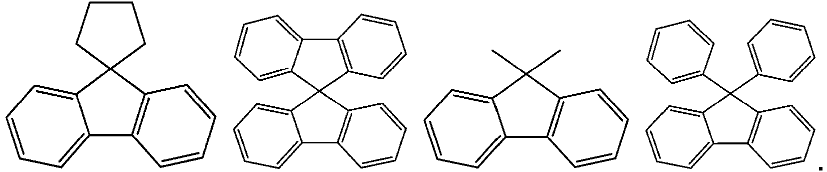

The heterocyclic group herein refers to any suitable functional group or substituent derived from a ring including at least one of B, O, N, P, Si, or Se as a heteroatom. The heterocyclic group includes an aliphatic heterocyclic group and/or an aromatic heterocyclic group. The aromatic heterocyclic group may be a heteroaryl group. The aliphatic heterocycle and the aromatic heterocycle may be monocyclic or polycyclic.

In the disclosure, the heterocyclic group may include at least one of B, O, N, P, Si or S as a heteroatom. When the heterocyclic group includes two or more heteroatoms, the two or more heteroatoms may be the same as or different from each other. The heterocyclic group may be a monocyclic heterocyclic group or a polycyclic heterocyclic group and has the concept including a heteroaryl group. The number of ring-forming carbon atoms in the heterocyclic group may be 2 to 30, 2 to 20, or 2 to 10.

In the disclosure, the aliphatic heterocyclic group may include one or more selected from among B, O, N, P, Si, and S as a heteroatom. The number of ring-forming carbon atoms in the aliphatic heterocyclic group may be 2 to 30, 2 to 20, or 2 to 10. Examples of the aliphatic heterocyclic group may include an oxirane group, a thiirane group, a pyrrolidine group, a piperidine group, a tetrahydrofuran group, a tetrahydrothiophene group, a thiane group, a tetrahydropyran group, a 1,4-dioxane group, etc., but the embodiment of the present disclosure is not limited thereto.

The heteroaryl group herein may include at least one of B, O, N, P, Si, or S as a heteroatom. When the heteroaryl group contains two or more heteroatoms, the two or more heteroatoms may be the same as or different from each other. The heteroaryl group may be a monocyclic heteroaryl group or polycyclic heteroaryl group. The number of ring-forming carbon atoms in the heteroaryl group may be 2 to 30, 2 to 20, or 2 to 10. Examples of the heteroaryl group may include a thiophene group, a furan group, a pyrrole group, an imidazole group, a pyridine group, a pyrimidine group, a bipyridine group, a triazine group, a triazole group, an acridyl group, a pyridazine group, a pyrazinyl group, a quinoline group, a quinazoline group, a quinoxaline group, a phenoxazine group, a phthalazine group, a pyrido pyridine group, a pyrido pyrazine group, a pyrazino pyrazine group, an isoquinoline group, an indole group, a carbazole group, an N-arylcarbazole group, an N-heteroarylcarbazole group, an N-alkylcarbazole group, a benzoxazole group, a benzimidazole group, a benzothiazole group, a benzocarbazole group, a benzothiophene group, a dibenzothiophene group, a thienothiophene group, a benzofuran group, a phenanthroline group, a thiazole group, an isoxazole group, an oxazole group, an oxadiazole group, a thiadiazole group, a phenothiazine group, a dibenzosilole group, a dibenzofuran group, etc., but the embodiment of the present disclosure is not limited thereto.

In the disclosure, the above description of the aryl group may be applied to an arylene group except that the arylene group is a divalent group. The above description of the heteroaryl group may be applied to a heteroarylene group except that the heteroarylene group is a divalent group.

In the disclosure, a silyl group includes an alkylsilyl group and an arylsilyl group. Examples of the silyl group may include a trimethylsilyl group, a triethylsilyl group, a t-butyldimethylsilyl group, a triisopropylsilyl group, a vinyldimethylsilyl group, a propyldimethylsilyl group, a triphenylsilyl group, a diphenylsilyl group, a phenylsilyl group, etc., but the embodiment of the present disclosure is not limited thereto.

In the disclosure, a thiol group may include an alkyl thiol group and an aryl thiol group. The thiol group may refer to a sulfur atom that is bonded to the alkyl group or the aryl group as defined above. Examples of the thiol group may include a methylthiol group, an ethylthiol group, a propylthiol group, a pentylthiol group, a hexylthiol group, an octylthiol group, a dodecylthiol group, a cyclopentylthiol group, a cyclohexylthiol group, a phenylthiol group, a naphthylthiol group, but the embodiment of the present disclosure is not limited thereto.

In the disclosure, an oxy group may refer to an oxygen atom that is bonded to the alkyl group or the aryl group as defined above. The oxy group may include an alkoxy group and/or an aryl oxy group. The alkoxy group may be a linear chain, a branched chain or a ring chain. The number of carbon atoms in the alkoxy group is not limited, but may be, for example, 1 to 20 or 1 to 10. The number of ring-forming carbon atoms in the aryloxy group may be, for example, 6 to 30, 6 to 20, or 6 to 15. Examples of the oxy group may include methoxy, ethoxy, n-propoxy, isopropoxy, butoxy, pentyloxy, hexyloxy, octyloxy, nonyloxy, decyloxy, phenoxy, benzyloxy, etc., but the embodiment of the present disclosure is not limited thereto.

The boron group herein may refer to a boron atom that is bonded to the alkyl group or the aryl group as defined above. The boron group includes an alkyl boron group and/or an aryl boron group. Examples of the boron group may include a trimethylboron group, a triethylboron group, a t-butyldimethylboron group, a triphenylboron group, a diphenylboron group, a phenylboron group, etc., but the embodiment of the present disclosure is not limited thereto.

In the disclosure, the number of carbon atoms in an amine group is not limited, but may be 1 to 30. The amine group may include an alkyl amine group and/or an aryl amine group. Examples of the amine group may include a methylamine group, a dimethylamine group, a phenylamine group, a diphenylamine group, a naphthylamine group, a 9-methyl-anthracenylamine group, a triphenylamine group, etc., but the embodiment of the present disclosure is not limited thereto.

In the disclosure, the alkyl group selected from among an alkylthiol group, an alkylsulfoxy group, an alkylaryl group, an alkylamino group, an alkyl boron group, an alkyl silyl group, and an alkyl amine group is the same as the examples of the alkyl group described above.

In the disclosure, the aryl group selected from among an aryloxy group, an arylthiol group, an arylsulfoxy group, an arylamino group, an aryl boron group, an aryl silyl group, an aryl amine group and an aryl alkyl group is the same as the examples of the aryl group described above.

In the disclosure, a direct linkage may refer to a single bond.

In some embodiments, 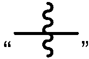 or "-* " herein refers to a position to be connected.

Hereinafter, embodiments of the present disclosure will be described with reference to the accompanying drawings.

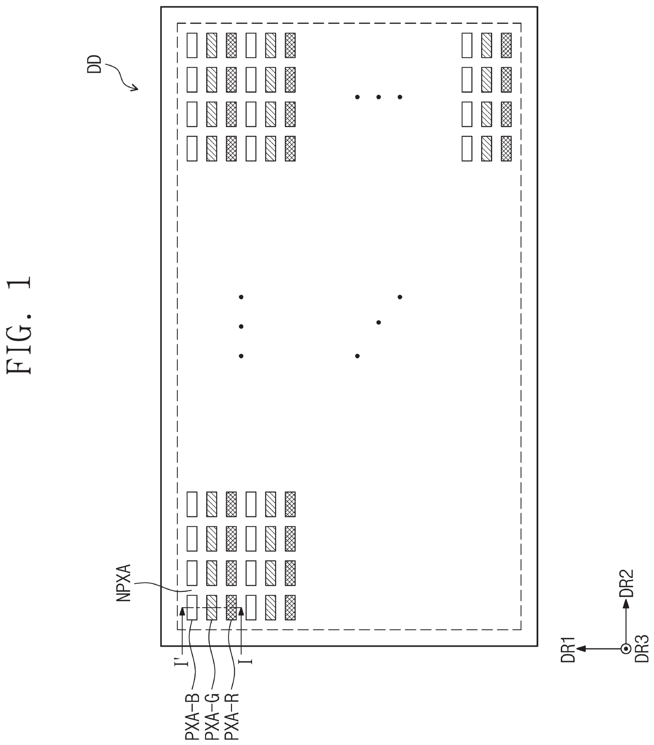

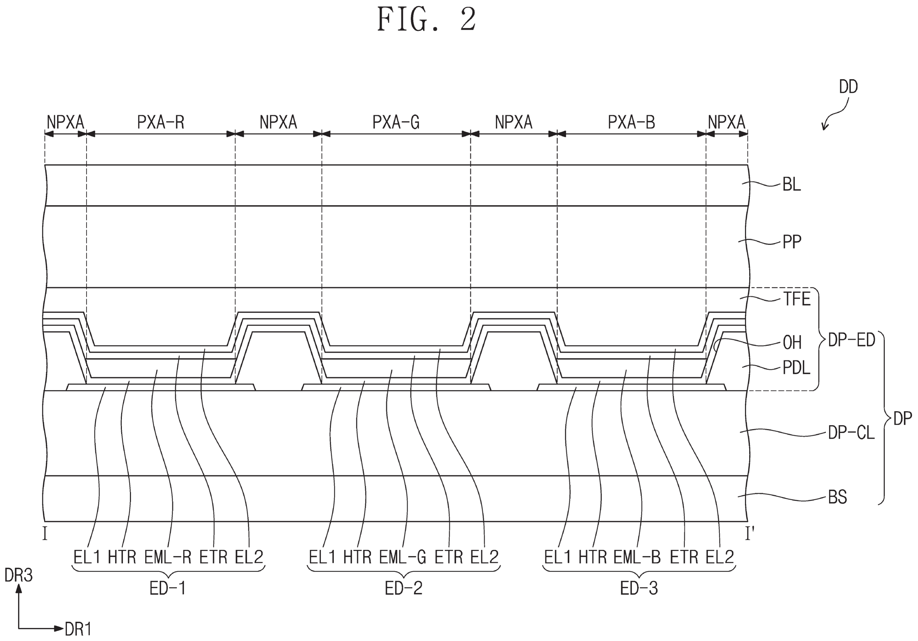

FIG. 1 is a plan view illustrating an embodiment of a display apparatus DD. FIG. 2 is a cross-sectional view of the display apparatus DD of the embodiment. FIG. 2 is a cross-sectional view illustrating a part taken along line I-I' of FIG. 1.

The display apparatus DD may include a display panel DP and an optical layer PP disposed on the display panel DP. The display panel DP includes light emitting devices ED-1, ED-2, and ED-3. The display apparatus DD may include a plurality of light emitting devices ED-1, ED-2, and ED-3. The optical layer PP may be on the display panel DP and control reflected light in the display panel DP due to external light. The optical layer PP may include, for example, a polarization layer or a color filter layer. In some embodiments, the optical layer PP may not be provided (e.g., may be excluded) from the display apparatus DD of an embodiment.

A base substrate BL may be on the optical layer PP. The base substrate BL may be a member which provides a base surface on which the optical layer PP disposed. The base substrate BL may be a glass substrate, a metal substrate, a plastic substrate, etc. However, the embodiment of the present disclosure is not limited thereto, and the base substrate BL may be an inorganic layer, an organic layer, and/or a composite material layer. In some embodiments, the base substrate BL may not be provided (e.g., may be excluded).

The display apparatus DD according to an embodiment may further include a filling layer. The filling layer may be between a display device layer DP-ED and the base substrate BL. The filling layer may be an organic material layer. The filling layer may include at least one of an acrylic-based resin, a silicone-based resin, or an epoxy-based resin.

The display panel DP may include a base layer BS, a circuit layer DP-CL provided on the base layer BS, and the display device layer DP-ED. The display device layer DP-ED may include a pixel defining film PDL, the light emitting devices ED-1, ED-2, and ED-3 between portions of the pixel defining film PDL, and an encapsulation layer TFE on the light emitting devices ED-1, ED-2, and ED-3.

The base layer BS may be a member which provides a base surface on which the display device layer DP-ED is disposed. The base layer BS may be a glass substrate, a metal substrate, a plastic substrate, etc. However, the embodiment of the present disclosure is not limited thereto, and the base layer BS may be an inorganic layer, an organic layer, and/or a composite material layer.

In an embodiment, the circuit layer DP-CL is disposed on the base layer BS, and the circuit layer DP-CL may include a plurality of transistors. Each of the transistors may include a control electrode, an input electrode, and an output electrode. For example, the circuit layer DP-CL may include a switching transistor and a driving transistor in order to drive the light emitting devices ED-1, ED-2, and ED-3 of the display device layer DP-ED.

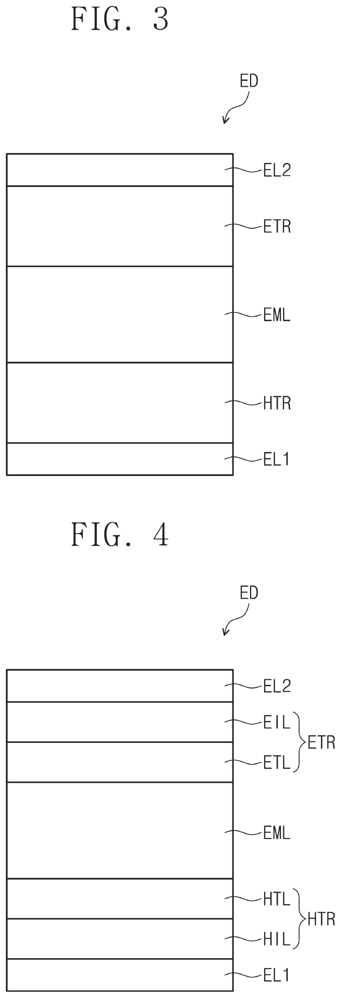

Each of the light emitting devices ED-1, ED-2, and ED-3 may have a structure of a light emitting device ED of an embodiment according to FIGS. 3 to 6, which will be described later. Each of the light emitting devices ED-1, ED-2 and ED-3 may include a first electrode EL1, a hole transport region HTR, emission layers EML-R, EML-G and EML-B, an electron transport region ETR, and a second electrode EL2.

FIG. 2 illustrates an embodiment in which the emission layers EML-R, EML-G, and EML-B of the light emitting devices ED-1, ED-2, and ED-3 are in openings OH defined in the pixel defining film PDL, and the hole transport region HTR, the electron transport region ETR, and the second electrode EL2 are provided as a common layer in the entire light emitting devices ED-1, ED-2, and ED-3. However, the embodiment of the present disclosure is not limited thereto, and the hole transport region HTR and the electron transport region ETR in an embodiment may be provided by being patterned inside the opening hole OH defined in the pixel defining film PDL. For example, the hole transport region HTR, the emission layers EML-R, EML-G, and EML-B, and the electron transport region ETR of the light emitting devices ED-1, ED-2, and ED-3 in an embodiment may be provided by being patterned in an inkjet printing method.

The encapsulation layer TFE may cover the light emitting devices ED-1, ED-2 and ED-3. The encapsulation layer TFE may seal the display device layer DP-ED. The encapsulation layer TFE may be a thin film encapsulation layer. The encapsulation layer TFE may be formed by laminating one layer or a plurality of layers. The encapsulation layer TFE includes at least one insulation layer. The encapsulation layer TFE according to an embodiment may include at least one inorganic film (hereinafter, an encapsulation-inorganic film). The encapsulation layer TFE according to an embodiment may also include at least one organic film (hereinafter, an encapsulation-organic film) and at least one encapsulation-inorganic film.

The encapsulation-inorganic film protects (reduces moisture/oxygen) the display device layer DP-ED from moisture/oxygen, and the encapsulation-organic film protects the display device layer DP-ED from foreign substances such as dust particles. The encapsulation-inorganic film may include silicon nitride, silicon oxynitride, silicon oxide, titanium oxide, aluminum oxide, and/or the like, but the embodiment of the present disclosure is not limited thereto. The encapsulation-organic film may include an acrylic-based compound, an epoxy-based compound, and/or the like. The encapsulation-organic film may include a photopolymerizable organic material, but the embodiment of the present disclosure is not limited thereto.

The encapsulation layer TFE may be on the second electrode EL2 and may be disposed filling the opening hole OH.

Referring to FIGS. 1 and 2, the display apparatus DD may include a non-light emitting region NPXA and light emitting regions PXA-R, PXA-G and PXA-B. The light emitting regions PXA-R, PXA-G and PXA-B may be regions in which light generated by the respective light emitting devices ED-1, ED-2 and ED-3 is emitted. The light emitting regions PXA-R, PXA-G, and PXA-B may be spaced apart from each other on a plane.

Each of the light emitting regions PXA-R, PXA-G, and PXA-B may be a region divided by the pixel defining film PDL. The non-light emitting regions NPXA may be regions between the adjacent light emitting regions PXA-R, PXA-G, and PXA-B, which correspond to portions of the pixel defining film PDL. In some embodiments, in the disclosure, the light emitting regions PXA-R, PXA-G, and PXA-B may respectively correspond to pixels. The pixel defining film PDL may divide the light emitting devices ED-1, ED-2, and ED-3. The emission layers EML-R, EML-G and EML-B of the light emitting devices ED-1, ED-2 and ED-3 may be disposed in openings OH defined in the pixel defining film PDL and separated from each other.

The light emitting regions PXA-R, PXA-G and PXA-B may be divided into a plurality of groups according to the color of light generated from the light emitting devices ED-1, ED-2 and ED-3. In the display apparatus DD of an embodiment shown in FIGS. 1 and 2, three light emitting regions PXA-R, PXA-G, and PXA-B which emit red light, green light, and blue light, respectively are illustrated. For example, the display apparatus DD of an embodiment may include the red light emitting region PXA-R, the green light emitting region PXA-G, and the blue light emitting region PXA-B that are separated from each other.

In the display apparatus DD according to an embodiment, the plurality of light emitting devices ED-1, ED-2 and ED-3 may emit light beams having wavelengths different from each other. For example, in an embodiment, the display apparatus DD may include a first light emitting device ED-1 that emits red light, a second light emitting device ED-2 that emits green light, and a third light emitting device ED-3 that emits blue light. For example, the red light emitting region PXA-R, the green light emitting region PXA-G, and the blue light emitting region PXA-B of the display apparatus DD may correspond to the first light emitting device ED-1, the second light emitting device ED-2, and the third light emitting device ED-3, respectively.

However, the embodiment of the present disclosure is not limited thereto, and the first to third light emitting devices ED-1, ED-2, and ED-3 may emit light beams in substantially the same wavelength range or at least one light emitting device may emit a light beam in a wavelength range different from the others. For example, the first to third light emitting devices ED-1, ED-2, and ED-3 may all emit blue light.

The light emitting regions PXA-R, PXA-G, and PXA-B in the display apparatus DD according to an embodiment may be arranged in a stripe form. Referring to FIG. 1, the plurality of red light emitting regions PXA-R may be arranged with each other along a second direction DR2, the plurality of green light emitting regions PXA-G may be arranged with each other along the second direction DR2, and the plurality of blue light emitting regions PXA-B may be arranged along the second direction DR2. In addition, one red light emitting region PXA-R, one green light emitting region PXA-G, and one blue light emitting region PXA-B may be alternately arranged with each other in this order along a first direction DR1. (DR3 is a third direction which is perpendicular or normal to the plane defined by the first direction DR1 and the second direction DR2).

FIGS. 1 and 2 illustrate that all the light emitting regions PXA-R, PXA-G, and PXA-B have substantially similar area, but the embodiment of the present disclosure is not limited thereto. Thus, the light emitting regions PXA-R, PXA-G, and PXA-B may have different areas from each other according to the wavelength range of the emitted light. In this embodiment, the areas of the light emitting regions PXA-R, PXA-G, and PXA-B may refer to areas when viewed in a plane defined by the first direction DR1 and the second direction DR2.

In some embodiments, an arrangement form of the light emitting regions PXA-R, PXA-G, and PXA-B is not limited to the feature illustrated in FIG. 1, and the order in which the red light emitting region PXA-R, the green light emitting region PXA-G, and the blue light emitting region PXA-B are arranged may be provided in one or more suitable combinations according to the characteristics of display quality required in the display apparatus DD. For example, the arrangement form of the light emitting regions PXA-R, PXA-G, and PXA-B may be a pentile (PENTILE^{®}) arrangement form (for example, an RGBG matrix, an RGBG structure, or RGBG matrix structure) or a diamond (e.g., Diamond Pixel^{™}) arrangement form. PENTILE^{®} is a duly registered trademark of Samsung Display Co., Ltd. Diamond Pixel^{™} is the atoms of Samsung's OLED displays, consisting of red, blue, and green (RGB) screen dots in the shape of diamonds.

In some embodiments, the areas of the light emitting regions PXA-R, PXA-G, and PXA-B may be different from each other. For example, in an embodiment, the area of the green light emitting region PXA-G may be smaller than that of the blue light emitting region PXA-B, but the embodiment of the present disclosure is not limited thereto.

Hereinafter, FIGS. 3 to 6 are cross-sectional views schematically illustrating light emitting devices according to embodiments. The light emitting devices ED according to embodiments may each include a first electrode EL1, a second electrode EL2, and at least one functional layer between the first electrode EL1 and the second electrode EL2. For example, each of the light emitting devices ED according to embodiments may include a first electrode EL1, a hole transport region HTR, an emission layer EML, an electron transport region ETR, and a second electrode EL2 that are sequentially stacked.

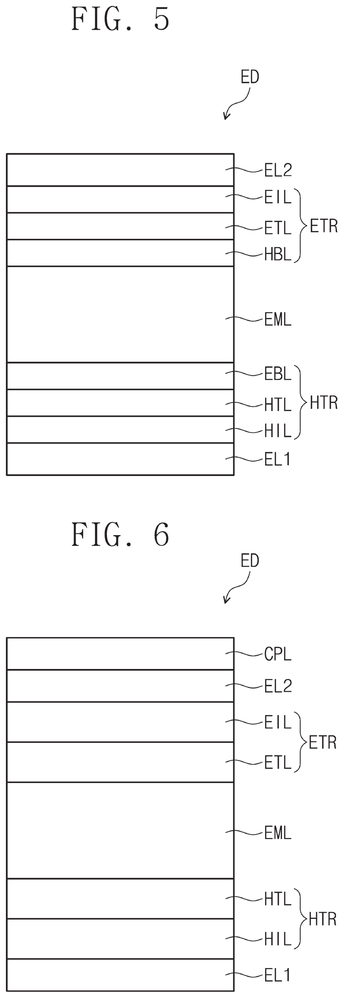

Compared to FIG. 3, FIG. 4 illustrates a cross-sectional view of a light emitting device ED of an embodiment, in which a hole transport region HTR includes a hole injection layer HIL and a hole transport layer HTL, and an electron transport region ETR includes an electron injection layer EIL and an electron transport layer ETL. Compared to FIG. 3, FIG. 5 illustrates a cross-sectional view of a light emitting device ED of an embodiment, in which a hole transport region HTR includes a hole injection layer HIL, a hole transport layer HTL, and an electron blocking layer EBL, and an electron transport region ETR includes an electron injection layer EIL, an electron transport layer ETL, and a hole blocking layer HBL. Compared to FIG. 4, FIG. 6 illustrates a cross-sectional view of a light emitting device ED of an embodiment including a capping layer CPL disposed on a second electrode EL2.

In the light emitting device ED according to an embodiment, the first electrode EL1 has conductivity (e.g., is a conductor). The first electrode EL1 may be formed of a metal material, a metal alloy, and/or a conductive compound. The first electrode EL1 may be an anode or a cathode. However, the embodiment of the present disclosure is not limited thereto. In some embodiments, the first electrode EL1 may be a pixel electrode. The first electrode EL1 may be a transmissive electrode, a transflective electrode, or a reflective electrode. The first electrode EL1 may include Ag, Mg, Cu, Al, Pt, Pd, Au, Ni, Nd, Ir, Cr, Li, Ca, LiF, Mo, Ti, W, In, Sn, Zn, a compound or compounds of one or more of the foregoing elements, a combination or combinations of two or more of the foregoing elements or compounds, a mixture or mixtures of two or more of the foregoing elements or compounds, and/or an oxide or oxides thereof.

When the first electrode EL1 is the transmissive electrode, the first electrode EL1 may include a transparent metal oxide such as indium tin oxide (ITO), indium zinc oxide (IZO), zinc oxide (ZnO), and/or indium tin zinc oxide (ITZO). When the first electrode EL1 is the transflective electrode or the reflective electrode, the first electrode EL1 may include Ag, Mg, Cu, Al, Pt, Pd, Au, Ni, Nd, Ir, Cr, Li, Ca, LiF/Ca (a stacked structure of LiF and Ca), LiF/Al (a stacked structure of LiF and Al), Mo, Ti, W, and/or a compound or mixture thereof (e.g., a mixture of Ag and Mg). In some embodiments, the first electrode EL1 may have a multilayer structure including a reflective film or a transflective film formed of the above-described materials, and a transparent conductive film formed of ITO, IZO, ZnO, ITZO, etc. For example, the first electrode EL1 may have a three-layer structure of ITO/Ag/ITO, but the embodiment of the present disclosure is not limited thereto. The embodiment of the present disclosure is not limited thereto, and the first electrode EL1 may include the above-described metal materials, combinations of at least two metal materials of the above-described metal materials, oxides of the above-described metal materials, and/or the like. The thickness of the first electrode EL1 may be from about 700 Å to about 10,000 Å. For example, the thickness of the first electrode EL1 may be from about 1,000 Å to about 3,000 Å.

The hole transport region HTR is provided on the first electrode EL1. The hole transport region HTR may have a single layer formed of a single material, a single layer formed of a plurality of different materials, or a multilayer structure including a plurality of layers formed of a plurality of different materials.

The hole transport region HTR may include at least one of a hole injection layer HIL, a hole transport layer HTL, a buffer layer or an emission-auxiliary layer, or an electron blocking layer EBL.

For example, the hole transport region HTR may have a single layer structure of the hole injection layer HIL or the hole transport layer HTL, or may have a single layer structure formed of a hole injection material and a hole transport material. In some embodiments, the hole transport region HTR may have a single layer structure formed of a plurality of different materials, or a structure in which a hole injection layer HIL/hole transport layer HTL, a hole injection layer HIL/hole transport layer HTL/buffer layer, a hole injection layer HIL/buffer layer, a hole transport layer HTL/buffer layer, or a hole injection layer HIL/hole transport layer HTL/electron blocking layer EBL are stacked in order from the first electrode EL1, but the embodiment of the present disclosure is not limited thereto.

The thickness of the hole transport region HTR may be, for example, from about 50 Å to about 15,000 Å. The hole transport region HTR may be formed utilizing one or more suitable methods such as a vacuum deposition method, a spin coating method, a cast method, a Langmuir-Blodgett (LB) method, an inkjet printing method, a laser printing method, and/or a laser induced thermal imaging (LITI) method.

The hole transport region HTR may include a compound represented by Formula H-1:

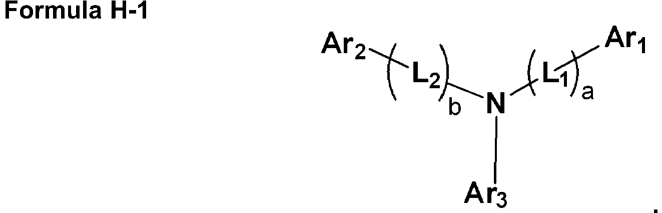

In Formula H-1 above, L₁ and L₂ may each independently be a direct linkage, a substituted or unsubstituted arylene group having 6 to 30 ring-forming carbon atoms, or a substituted or unsubstituted heteroarylene group having 2 to 30 ring-forming carbon atoms. a and b may each independently be an integer of 0 to 10. In some embodiments, when a or b is an integer of 2 or greater, a plurality of L₁s and L₂s may each independently be a substituted or unsubstituted arylene group having 6 to 30 ring-forming carbon atoms, or a substituted or unsubstituted heteroarylene group having 2 to 30 ring-forming carbon atoms.

In Formula H-1, Ar₁ and Ar₂ may each independently be a substituted or unsubstituted aryl group having 6 to 30 ring-forming carbon atoms, or a substituted or unsubstituted heteroaryl group having 2 to 30 ring-forming carbon atoms. In some embodiments, in Formula H-1, Ar₃ may be a substituted or unsubstituted aryl group having 6 to 30 ring-forming carbon atoms.

The compound represented by Formula H-1 above may be a monoamine compound. In some embodiments, the compound represented by Formula H-1 above may be a diamine compound in which at least one among Ar₁ to Ar₃ includes the amine group as a substituent. In some embodiments, the compound represented by Formula H-1 above may be a carbazole-based compound including a substituted or unsubstituted carbazole group in at least one of Ar₁ or Ar₂, or a fluorene-based compound including a substituted or unsubstituted fluorene group in at least one of Ar₁ or Ar₂.

The compound represented by Formula H-1 may be represented by any one among the compounds of Compound Group H. However, the compounds listed in Compound Group H are examples, and the compounds represented by Formula H-1 are not limited to those represented by Compound Group H:

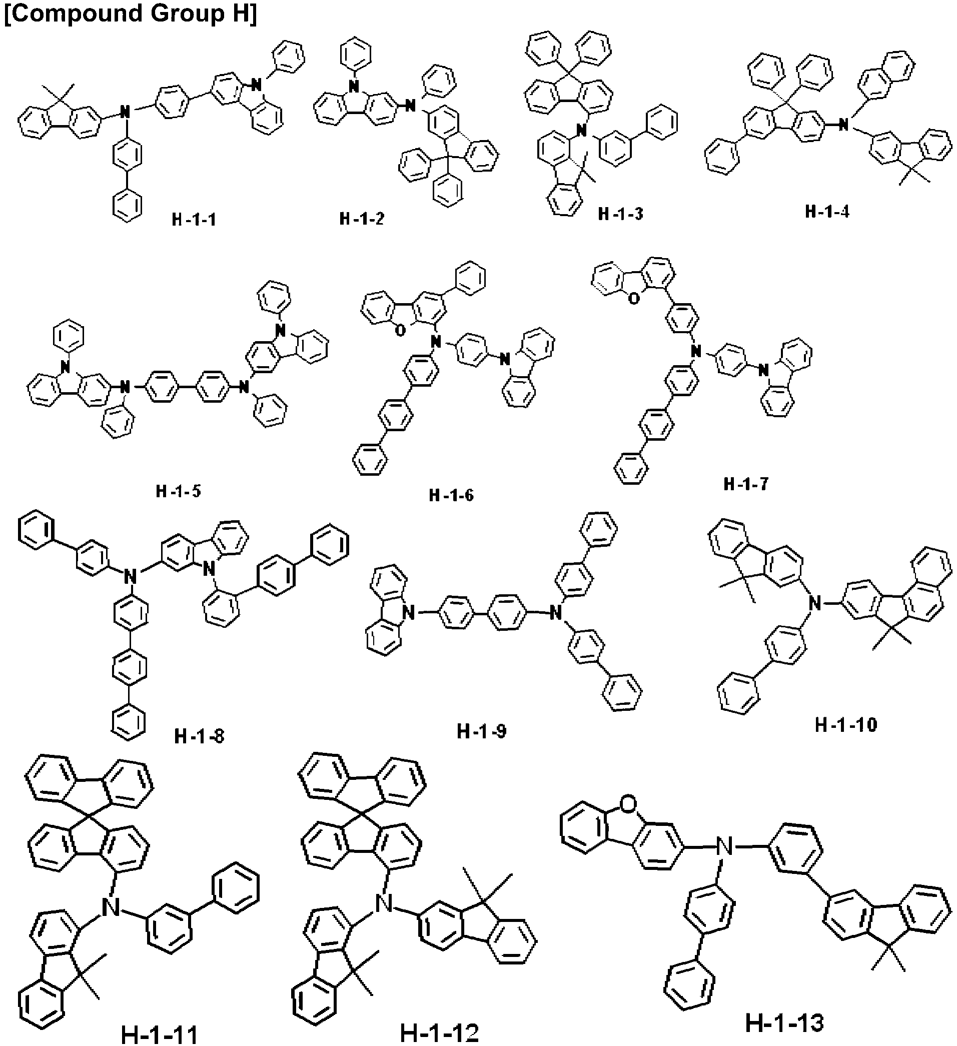

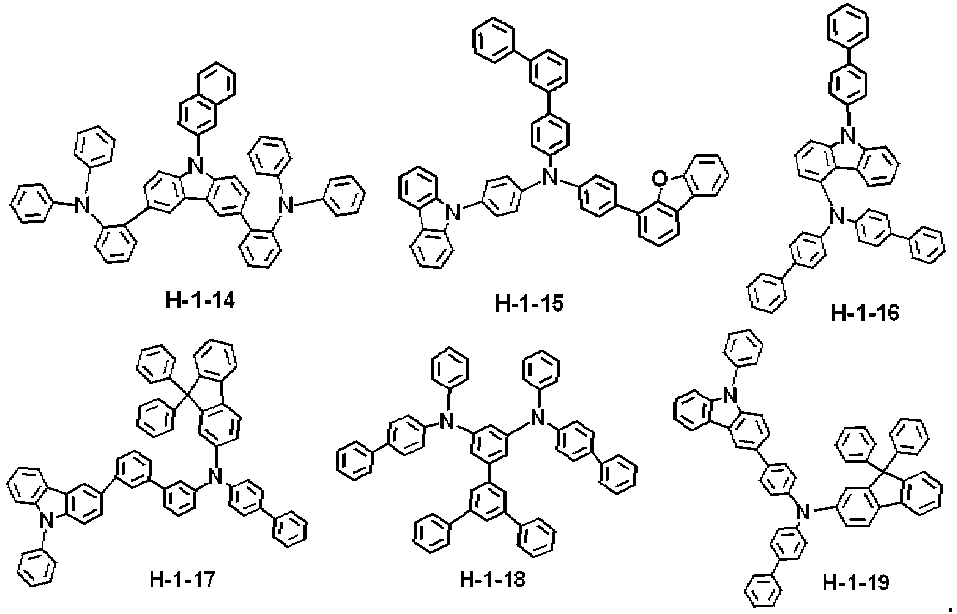

The hole transport region HTR may further include a phthalocyanine compound such as copper phthalocyanine; N¹,N^{1'}-([1,1'-biphenyl]-4,4'-diyl)bis(N¹-phenyl-N⁴,N⁴-di-m-tolylbenzene-1,4-diamine) (DNTPD), 4,4',4"-[tris(3-methylphenyl)phenylamino] triphenylamine (m-MTDATA), 4,4', 4"-tris(N,N-diphenylamino)triphenylamine (TDATA), 4,4',4"-tris[N(2-naphthyl)-N-phenylamino]-triphenylamine (2-TNATA), poly(3,4-ethylenedioxythiophene)/poly(4-styrenesulfonate) (PEDOT/PSS), polyaniline/dodecylbenzenesulfonic acid (PANI/DBSA), polyaniline/camphor sulfonic acid (PANI/CSA), polyaniline/poly(4-styrenesulfonate) (PANI/PSS), N,N'-di(naphthalene-I-yl)-N,N'-diphenyl-benzidine (NPB), triphenylamine-containing polyetherketone (TPAPEK), 4-isopropyl-4'-methyldiphenyliodonium [tetrakis(pentafluorophenyl)borate], dipyrazino[2,3-f: 2',3'-h]quinoxaline-2,3,6,7,10,11-hexacarbonitrile (HATCN), and/or the like.

The hole transport region HTR may include a carbazole-based derivative such as N-phenyl carbazole and polyvinyl carbazole, a fluorene-based derivative, N,N'-bis(3-methylphenyl)-N,N'-diphenyl-[1,1-biphenyl]-4,4'-diamine (TPD), a triphenylamine-based derivative such as 4,4',4"-tris(N-carbazolyl)triphenylamine (TCTA), N,N'-di(naphthalene-I-yl)-N,N'-diphenyl-benzidine (NPB), 4,4'-cyclohexylidene bis[N,N-bis(4-methylphenyl]benzenamine] (TAPC), 4,4'-bis[N,N'-(3-tolyl)amino]-3,3'-dimethylbiphenyl (HMTPD), 1,3-bis(N-carbazolyl)benzene (mCP), and/or the like.

In some embodiments, the hole transport region HTR may include 9-(4-tert-butylphenyl)-3,6-bis(triphenylsilyl)-9H-carbazole (CzSi), 9-phenyl-9H-3,9'-bicarbazole (CCP), 1,3-bis(1,8-dimethyl-9H-carbazol-9-yl)benzene (mDCP), etc.

The hole transport region HTR may include the above-described compounds of the hole transport region in at least one of a hole injection layer HIL, a hole transport layer HTL, or an electron blocking layer EBL.

The thickness of the hole transport region HTR may be from about 100 Å to about 10,000 Å, for example, from about 100 Å to about 5,000 Å. When the hole transport region HTR includes the hole injection layer HIL, the hole injection layer HIL may have, for example, a thickness of about 30 Å to about 1,000 Å. When the hole transport region HTR includes the hole transport layer HTL, the hole transport layer HTL may have a thickness of about 30 Å to about 1,000 Å. For example, when the hole transport region HTR includes the electron blocking layer EBL, the electron blocking layer EBL may have a thickness of about 10 Å to about 1,000 Å. When the thicknesses of the hole transport region HTR, the hole injection layer HIL, the hole transport layer HTL and the electron blocking layer EBL satisfy the above-described ranges, satisfactory (suitable) hole transport properties may be achieved without a substantial increase in a driving voltage.

The hole transport region HTR may further include a charge generating material to increase conductivity in addition to the above-described materials. The charge generating material may be dispersed substantially uniformly or non-uniformly in the hole transport region HTR. The charge generating material may be, for example, a p-dopant. The p-dopant may include at least one of a halogenated metal compound, a quinone derivative, a metal oxide, or a cyano group-containing compound, but the embodiment of the present disclosure is not limited thereto. For example, the p-dopant may include a metal halide compound such as Cul and Rbl, a quinone derivative such as tetracyanoquinodimethane (TCNQ) and 2,3,5,6-tetrafluoro-7,7'8,8-tetracyanoquinodimethane (F4-TCNQ), a metal oxide such as tungsten oxide and molybdenum oxide, a cyano group-containing compound such as dipyrazino[2,3-f: 2',3'-h] quinoxaline-2,3,6,7,10,11-hexacarbonitrile (HATCN), 4-[[2,3-bis[cyano-(4-cyano-2,3,5,6-tetrafluorophenyl)methylidene]cyclopropylidene]-cya nomethyl]-2,3,5,6-tetrafluorobenzonitrile (NDP9), etc., but the embodiment of the present disclosure is not limited thereto.

As described above, the hole transport region HTR may further include at least one of the buffer layer or the electron blocking layer EBL in addition to the hole injection layer HIL and the hole transport layer HTL. The buffer layer may compensate for a resonance distance according to the wavelength of light emitted from the emission layer EML and may thus increase light emission efficiency. A material that may be contained in the hole transport region HTR may be utilized as a material to be contained in the buffer layer. The electron blocking layer EBL is a layer that serves to prevent or reduce the electron injection from the electron transport region ETR to the hole transport region HTR.

The emission layer EML is provided on the hole transport region HTR. The emission layer EML may have a thickness of, for example, about 100 Å to about 1,000 Å or about 100 Å to about 300 Å. The emission layer EML may have a single layer formed of a single material, a single layer formed of a plurality of different materials, or a multilayer structure having a plurality of layers formed of a plurality of different materials.

In the light emitting device ED of an embodiment, the emission layer EML may include an anthracene derivative, a pyrene derivative, a fluoranthene derivative, a chrysene derivative, a dehydrobenzanthracene derivative, or a triphenylene derivative. For example, the emission layer EML may include the anthracene derivative or the pyrene derivative.

In each light emitting device ED of embodiments illustrated in FIGS. 3 to 6, the emission layer EML may further include a suitable host and dopant other than the above-described host and dopant, and the emission layer EML may include a compound represented by Formula E-1. The compound represented by Formula E-1 may be utilized as a fluorescent host material.

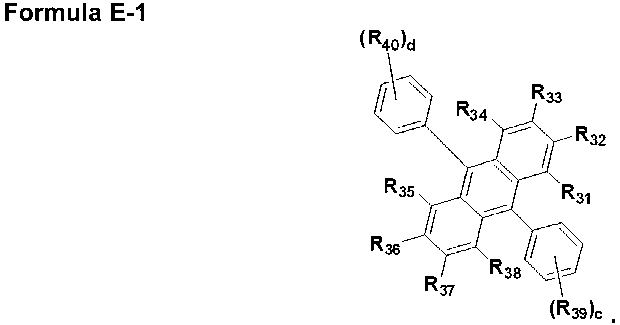

In Formula E-1, R₃₁ to R₄₀ may each independently be a hydrogen atom, a deuterium atom, a halogen atom, a substituted or unsubstituted silyl group, a substituted or unsubstituted thiol group, a substituted or unsubstituted oxy group, a substituted or unsubstituted alkyl group having 1 to 10 carbon atoms, a substituted or unsubstituted alkenyl group having 2 to 10 carbon atoms, a substituted or unsubstituted aryl group having 6 to 30 ring-forming carbon atoms, or a substituted or unsubstituted heteroaryl group having 2 to 30 ring-forming carbon atoms, or may be bonded to an adjacent group to form a ring. In some embodiments, R₃₁ to R₄₀ may be bonded to an adjacent group to form a saturated hydrocarbon ring or an unsaturated hydrocarbon ring, a saturated heterocycle, or an unsaturated heterocycle.

In Formula E-1, c and d may each independently be an integer of 0 to 5.

Formula E-1 may be represented by any one among Compound E1 to Compound E19:

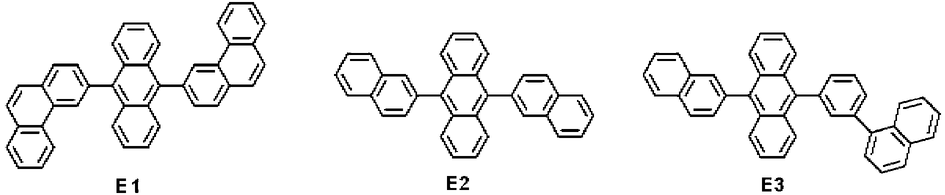

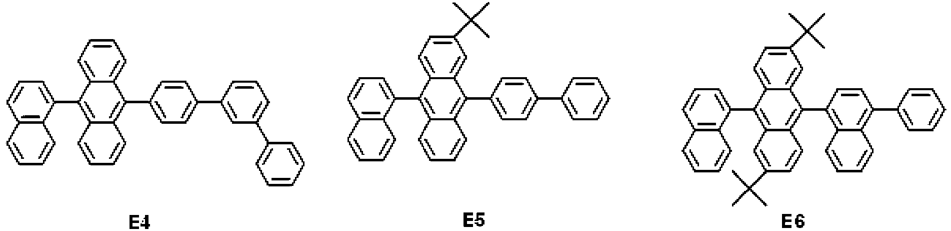

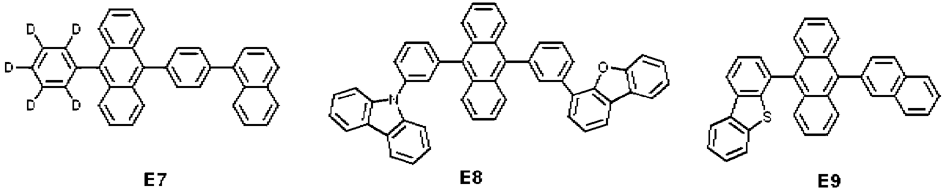

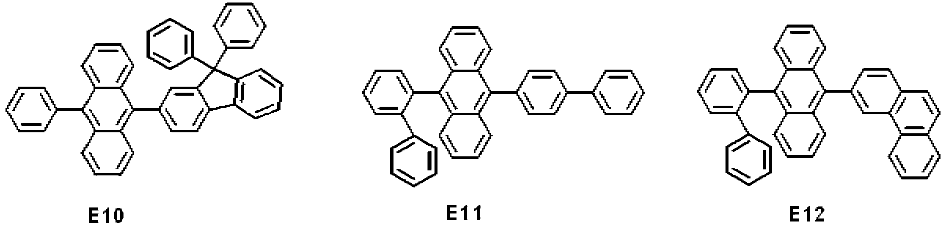

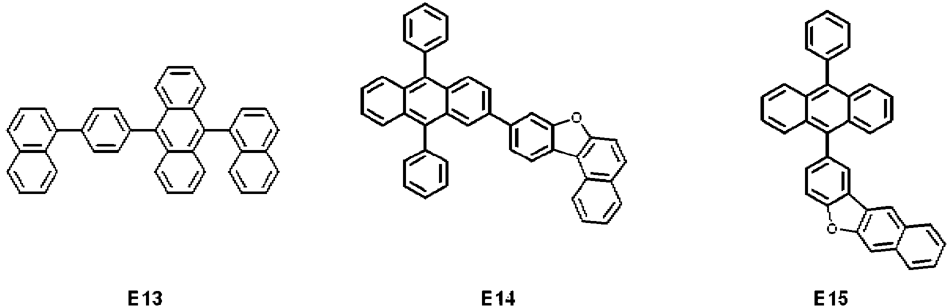

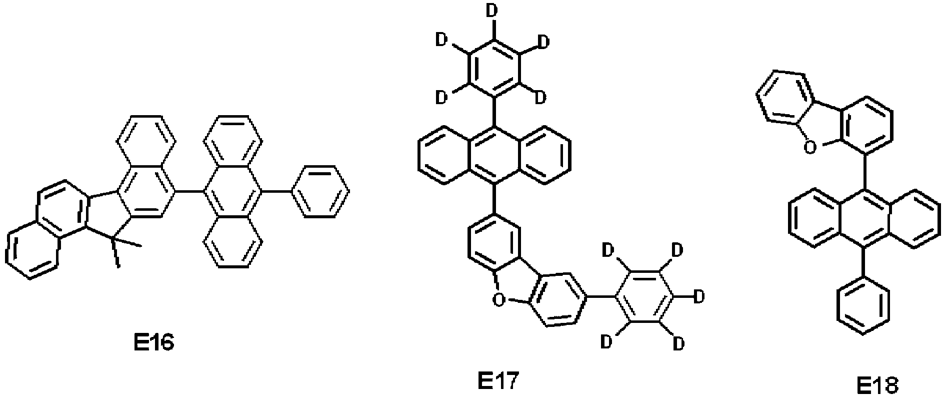

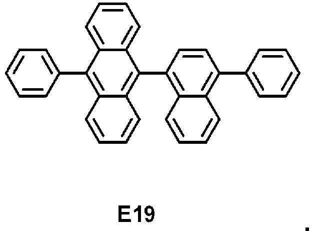

In an embodiment, the emission layer EML may include a compound represented by Formula E-2a or Formula E-2b. The compound represented by Formula E-2a or Formula E-2b may be utilized as a phosphorescent host material.

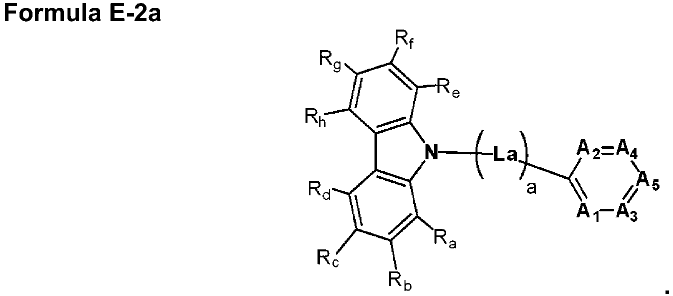

In Formula E-2a, a may be an integer of 0 to 10, Lₐ may be a direct linkage, a substituted or unsubstituted arylene group having 6 to 30 ring-forming carbon atoms, or a substituted or unsubstituted heteroarylene group having 2 to 30 ring-forming carbon atoms. In some embodiments, when a is an integer of 2 or more, a plurality of Lₐs may each independently be a substituted or unsubstituted arylene group having 6 to 30 ring-forming carbon atoms, or a substituted or unsubstituted heteroarylene group having 2 to 30 ring-forming carbon atoms.

In some embodiments, in Formula E-2a, A₁ to A₅ may each independently be N or CRᵢ. Rₐ to Rᵢ may each independently be a hydrogen atom, a deuterium atom, a substituted or unsubstituted amine group, a substituted or unsubstituted thiol group, a substituted or unsubstituted oxy group, a substituted or unsubstituted alkyl group having 1 to 20 carbon atoms, a substituted or unsubstituted alkenyl group having 2 to 20 carbon atoms, a substituted or unsubstituted aryl group having 6 to 30 ring-forming carbon atoms, or a substituted or unsubstituted heteroaryl group having 2 to 30 ring-forming carbon atoms, and/or may be bonded to an adjacent group to form a ring. Rₐ to Rᵢ may be bonded to an adjacent group to form a hydrocarbon ring or a heterocycle containing N, O, S, etc. as a ring-forming atom.

In some embodiments, in Formula E-2a, two or three selected from among A₁ to A₅ may be N, and the rest (those that are not N) may be CRᵢ.

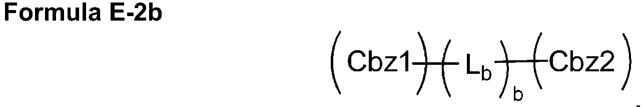

In Formula E-2b, Cbz1 and Cbz2 may each independently be an unsubstituted carbazole group, or a carbazole group substituted with an aryl group having 6 to 30 ring-forming carbon atoms. L_{b} is a direct linkage, a substituted or unsubstituted arylene group having 6 to 30 ring-forming carbon atoms, or a substituted or unsubstituted heteroarylene group having 2 to 30 ring-forming carbon atoms. In some embodiments, b is an integer of 0 to 10, and when b is an integer of 2 or more, a plurality of L_{b}s may each independently be a substituted or unsubstituted arylene group having 6 to 30 ring-forming carbon atoms, or a substituted or unsubstituted heteroarylene group having 2 to 30 ring-forming carbon atoms.

The compound represented by Formula E-2a or Formula E-2b may be represented by any one among the compounds of Compound Group E-2. However, the compounds listed in Compound Group E-2 are examples, and the compound represented by Formula E-2a or Formula E-2b is not limited to those represented in Compound Group E-2.

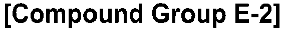

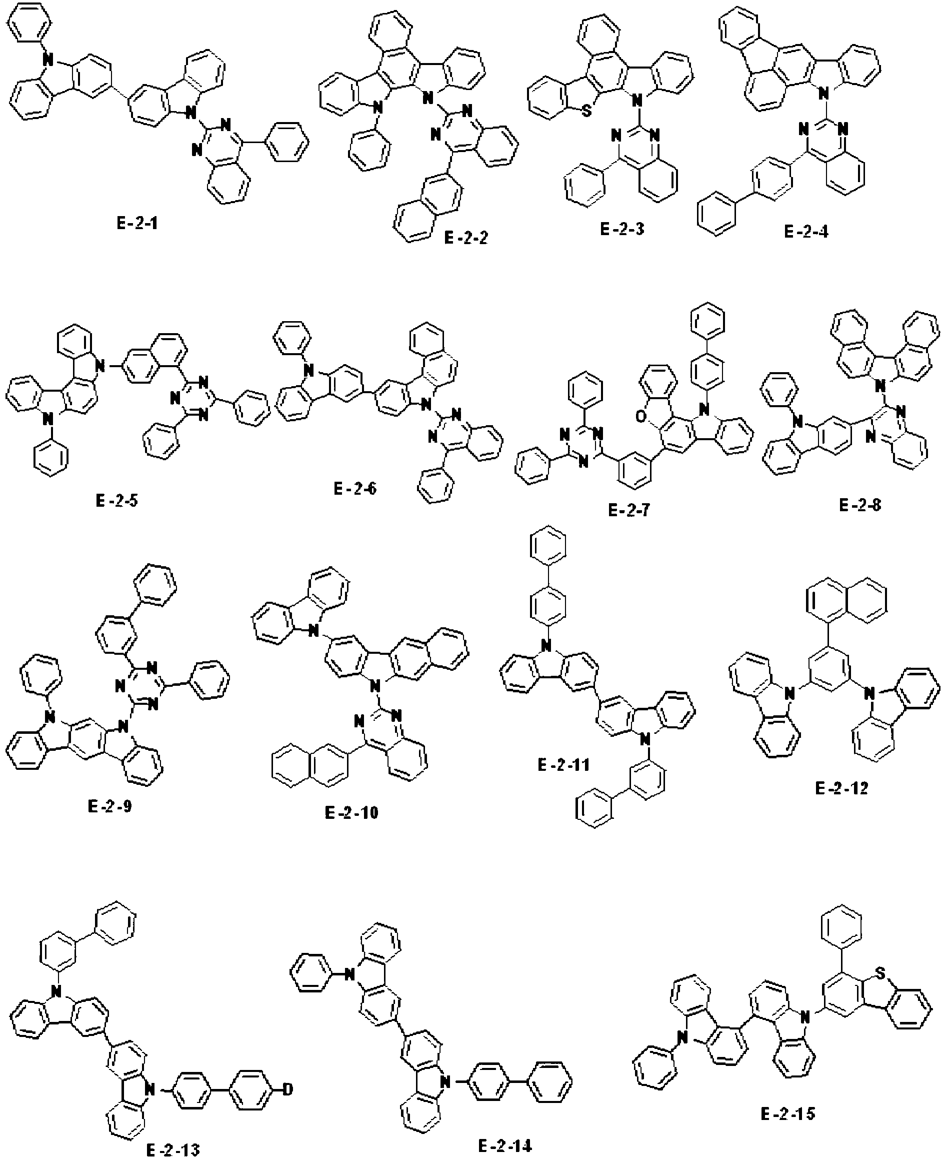

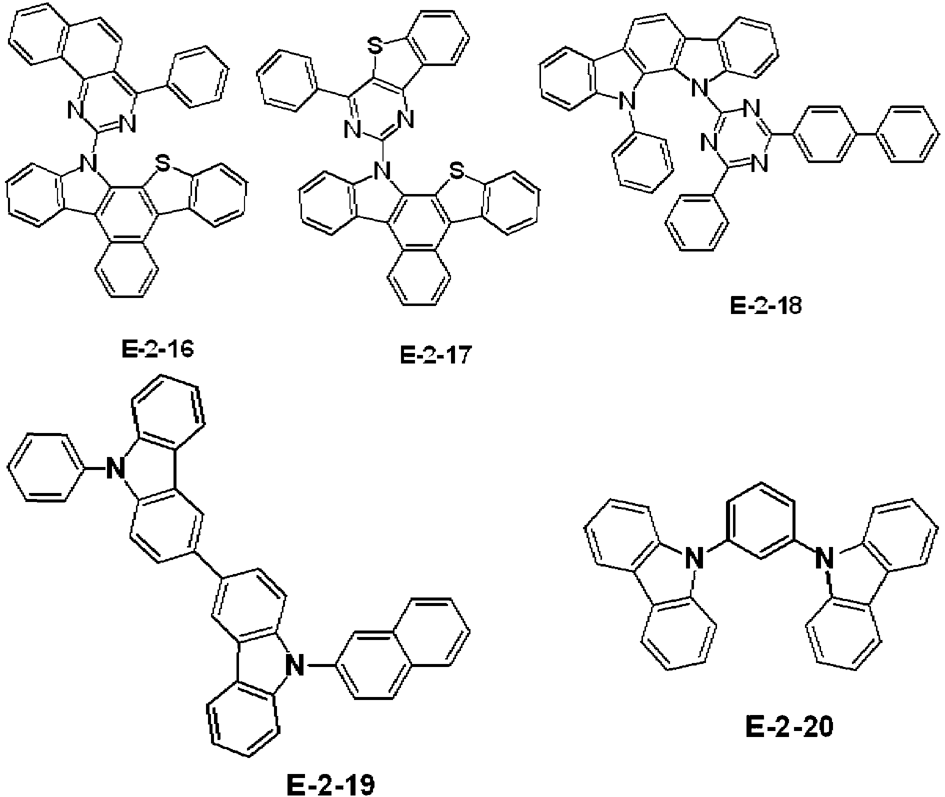

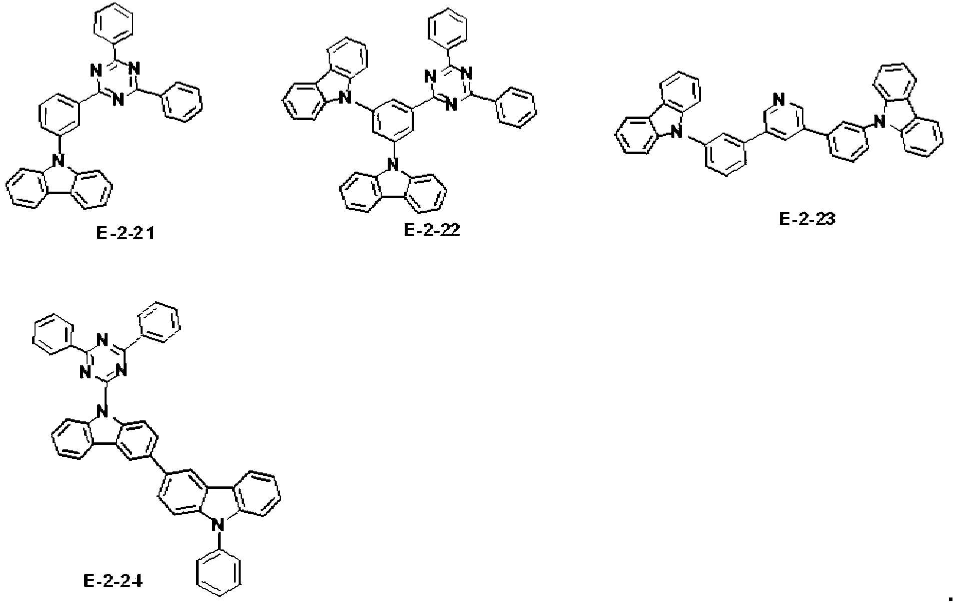

The emission layer EML may further include a general material suitable in the art as a host material. For example, the emission layer EML may include, as a host material, at least one of bis(4-(9H-carbazol-9-yl)phenyl)diphenylsilane (BCPDS), (4-(1-(4-(diphenylamino)phenyl)cyclohexyl)phenyl)diphenyl-phosphine oxide (POPCPA), bis[2-(diphenylphosphino)phenyl]ether oxide (DPEPO), 4,4'-bis(N-carbazolyl)-1,1'-biphenyl (CBP), 1,3-bis(carbazol-9-yl)benzene (mCP), 2,8-bis(diphenylphosphoryl)dibenzo[b,d]furan (PPF), 4,4',4"-tris(carbazol-9-yl)-triphenylamine (TCTA), or 1,3,5-tris(1-phenyl-1H-benzo[d]imidazole-2-yl)benzene (TPBi). However, the embodiment of the present disclosure is not limited thereto. For example, tris(8-hydroxyquinolino)aluminum (Alq₃), 9,10-di(naphthalene-2-yl)anthracene (ADN), 2-tert-butyl-9,10-di(naphth-2-yl)anthracene (TBADN), distyrylarylene (DSA), 4,4'-bis(9-carbazolyl)-2,2'-dimethyl-biphenyl (CDBP), 2-methyl-9,10-bis(naphthalen-2-yl)anthracene (MADN), hexaphenyl cyclotriphosphazene (CP1), 1,4-bis(triphenylsilyl)benzene (UGH2), hexaphenylcyclotrisiloxane (DPSiO₃), octaphenylcyclotetra siloxane (DPSiO₄), etc. may be utilized as a host material.

The emission layer EML may further include a compound represented by Formula M-a or Formula M-b. The compound represented by Formula M-a or Formula M-b may be utilized as a phosphorescence dopant material.

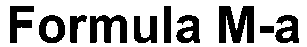

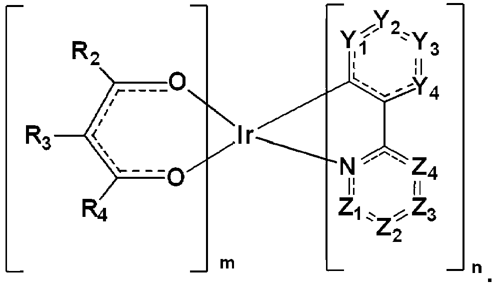

In Formula M-a above, Y₁ to Y₄ and Z₁ to Z₄ may each independently be CR₁ or N, R₁ to R₄ may each independently be a hydrogen atom, a deuterium atom, a substituted or unsubstituted amine group, a substituted or unsubstituted thiol group, a substituted or unsubstituted oxy group, a substituted or unsubstituted alkyl group having 1 to 20 carbon atoms, a substituted or unsubstituted alkenyl group having 2 to 20 carbon atoms, a substituted or unsubstituted aryl group having 6 to 30 ring-forming carbon atoms, or a substituted or unsubstituted heteroaryl group having 2 to 30 ring-forming carbon atoms, and/or may be bonded to an adjacent group to form a ring. In Formula M-a, m is 0 or 1, and n is 2 or 3. In Formula M-a, when m is 0, n is 3, and when m is 1, n is 2.

The compound represented by Formula M-a may be utilized as a phosphorescent dopant.

The compound represented by Formula M-a may be represented by any one among Compound M-a1 to Compound M-a25. However, Compounds M-a1 to M-a25 are examples, and the compound represented by Formula M-a is not limited to those represented by Compounds M-a1 to M-a25.

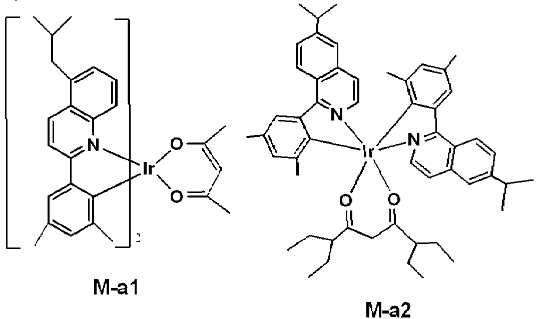

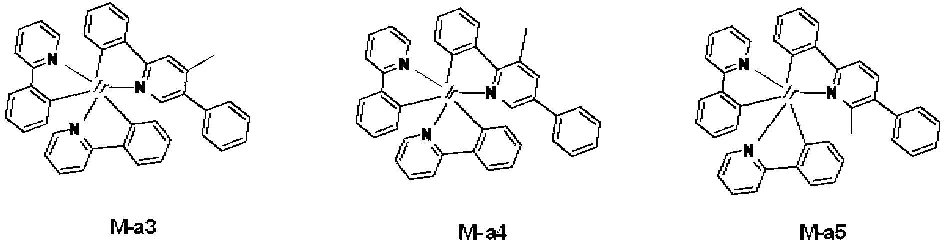

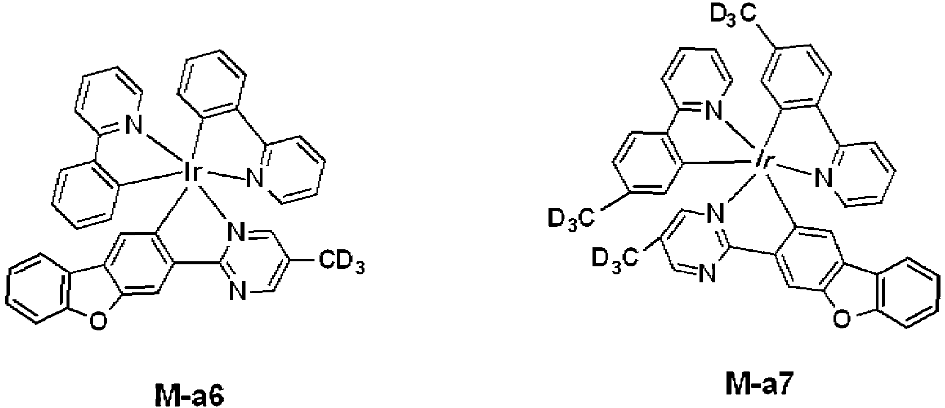

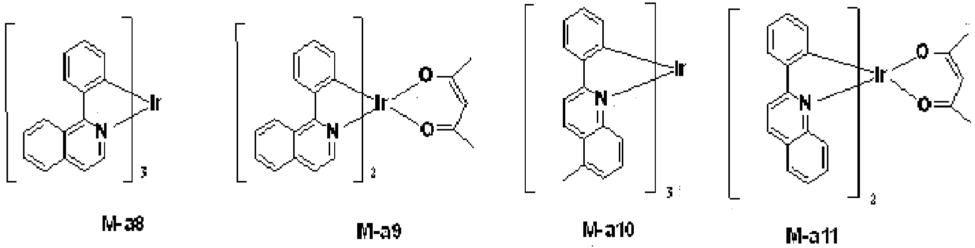

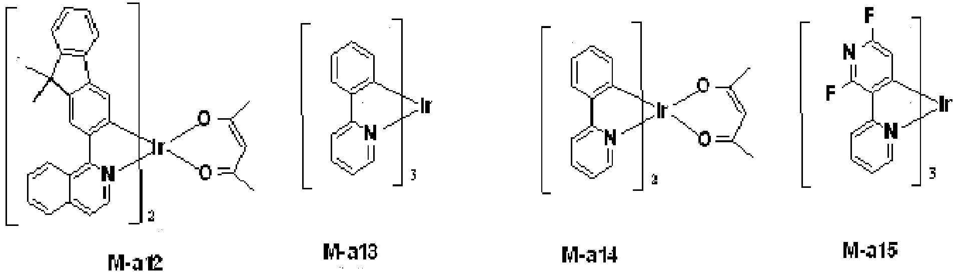

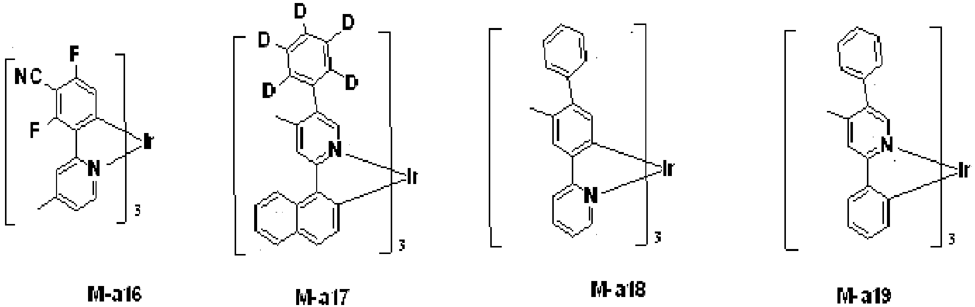

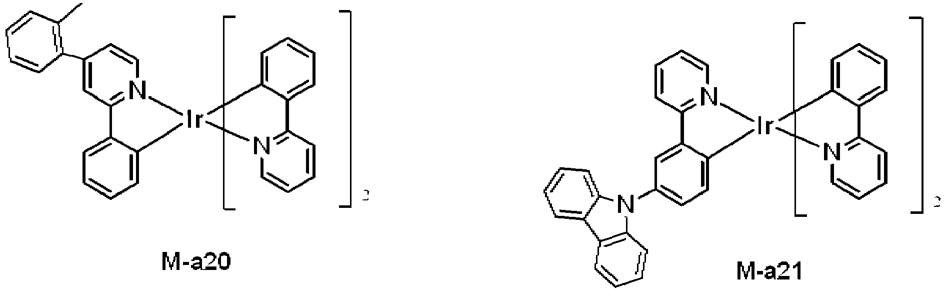

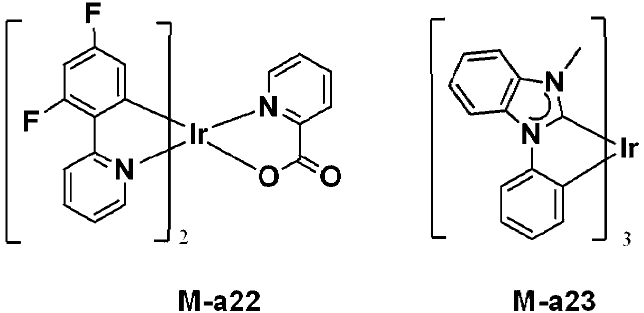

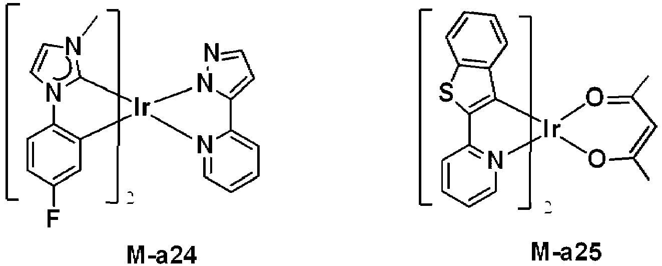

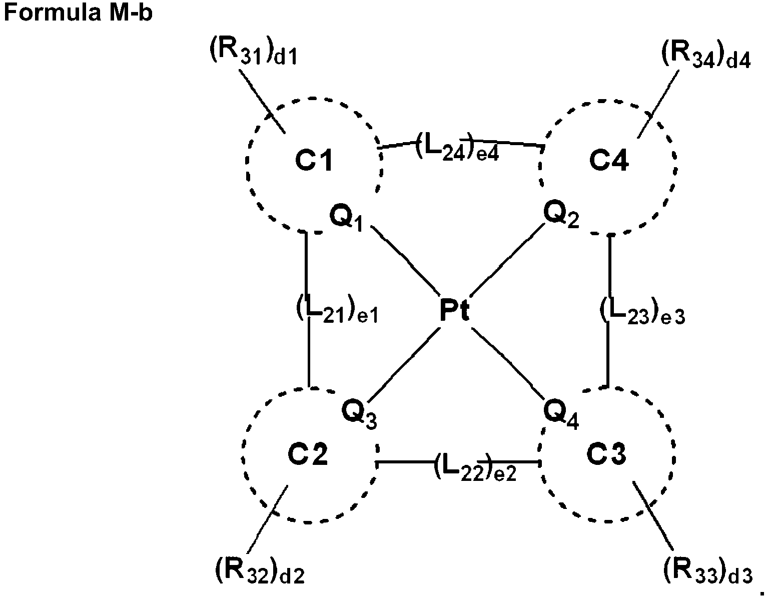

In Formula M-b, Q₁ to Q₄ may each independently be C or N, and C1 to C4 may each independently be a substituted or unsubstituted hydrocarbon ring having 5 to 30 ring-forming carbon atoms, or a substituted or unsubstituted heterocycle having 2 to 30 ring-forming carbon atoms. L₂₁ to L₂₄ may each independently be a direct linkage, 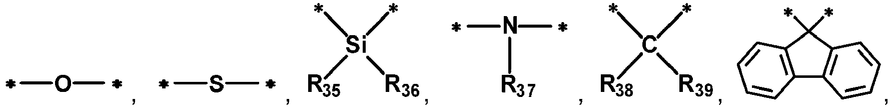 a substituted or unsubstituted divalent alkyl group having 1 to 20 carbon atoms, a substituted or unsubstituted arylene group having 6 to 30 ring-forming carbon atoms, or a substituted or unsubstituted heteroarylene group having 2 to 30 ring-forming carbon atoms, and e1 to e4 may each independently be 0 or 1. R₃₁ to R₃₉ may each independently be a hydrogen atom, a deuterium atom, a halogen atom, a cyano group, a substituted or unsubstituted amine group, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms, and/or are bonded to an adjacent group to form a ring, and d1 to d4 may each independently be an integer of 0 to 4.

The compound represented by Formula M-b may be utilized as a blue phosphorescence dopant or a green phosphorescence dopant.

The compound represented by Formula M-b may be represented by any one among the compounds below (i.e., M-b-1 to M-b-11). However, the compounds below are examples, and the compound represented by Formula M-b is not limited to those represented by the compounds below.

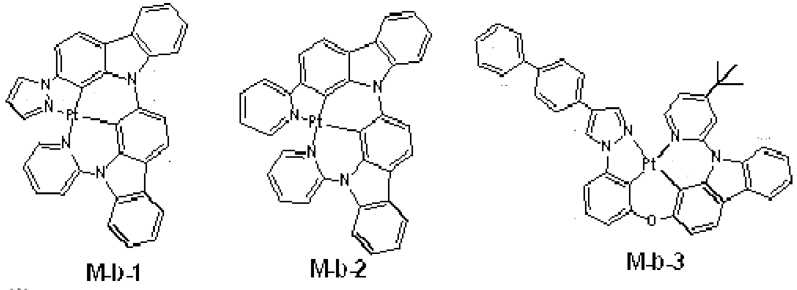

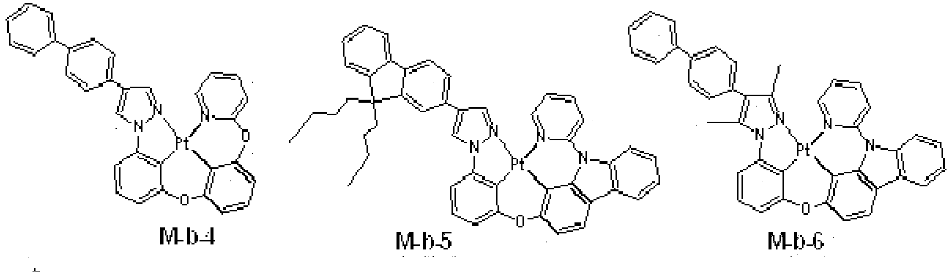

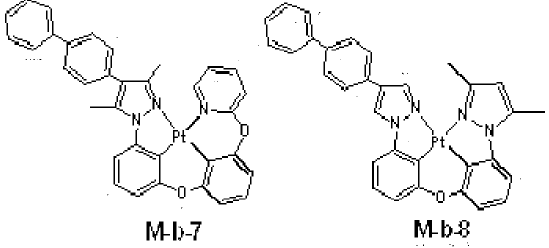

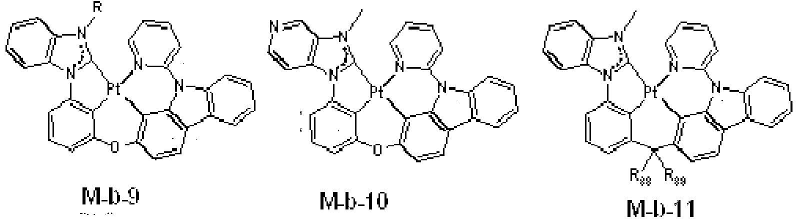

In the compounds, R, R₃₈, and R₃₉ may each independently be a hydrogen atom, a deuterium atom, a halogen atom, a cyano group, a substituted or unsubstituted amine group, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms.

The emission layer EML may further include a compound represented by any one among Formula F-a to Formula F-c. The compound represented by Formula F-a or Formula F-c may be utilized as a fluorescence dopant material.

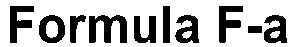

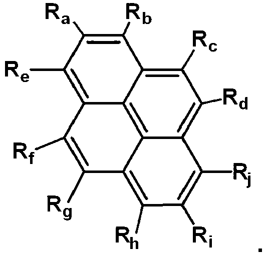

In Formula F-a, two selected from among Rₐ to Rⱼ may each independently be substituted with *-NAr₁Ar₂. The substituents, which are not substituted with *-NAr₁Ar₂, among Rₐ to Rⱼ may each independently be a hydrogen atom, a deuterium atom, a halogen atom, a cyano group, a substituted or unsubstituted amine group, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms. In *-NAr₁Ar₂, Ar₁ and Ar₂ may each independently be a substituted or unsubstituted aryl group having 6 to 30 ring-forming carbon atoms, or a substituted or unsubstituted heteroaryl group having 2 to 30 ring-forming carbon atoms. For example, at least one of Ar₁ or Ar₂ may be a heteroaryl group containing O or S as a ring-forming atom.

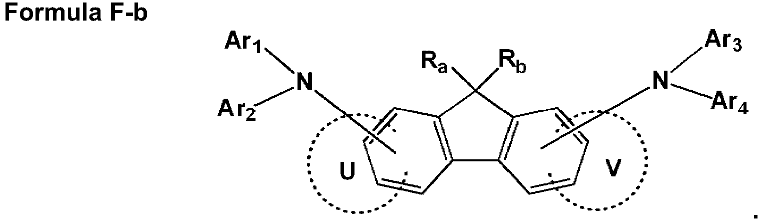

In Formula F-b, Ar₁ to Ar₄ may each independently be a substituted or unsubstituted aryl group having 6 to 30 ring-forming carbon atoms, or a substituted or unsubstituted heteroaryl group having 2 to 30 ring-forming carbon atoms, or may be bonded to an adjacent group to form a ring.

In Formula F-b, Rₐ and R_{b} may each independently be a hydrogen atom, a deuterium atom, a substituted or unsubstituted alkyl group having 1 to 20 carbon atoms, a substituted or unsubstituted alkenyl group having 2 to 20 carbon atoms, a substituted or unsubstituted aryl group having 6 to 30 ring-forming carbon atoms, or a substituted or unsubstituted heteroaryl group having 2 to 30 ring-forming carbon atoms, or may be bonded to an adjacent group to form a ring.

In Formula F-b, U and V may each independently be a substituted or unsubstituted hydrocarbon ring having 5 to 30 ring-forming carbon atoms, or a substituted or unsubstituted heterocycle having 2 to 30 ring-forming carbon atoms.

In Formula F-b, the number of rings represented by U and V may each independently be 0 or 1. For example, in Formula F-b, when the number of U or V is 1, one ring constitutes a fused ring at a portion indicated by U or V, and when the number of U or V is 0, a ring indicated by U or V does not exist. For example, when the number of U is 0 and the number of V is 1, or when the number of U is 1 and the number of V is 0, the fused ring having a fluorene core in Formula F-b may be a cyclic compound having four rings. In some embodiments, when each number of U and V is 0, the fused ring in Formula F-b may be a cyclic compound having three rings. In some embodiments, when each number of U and V is 1, the fused ring having a fluorene core in Formula F-b may be a cyclic compound having five rings.

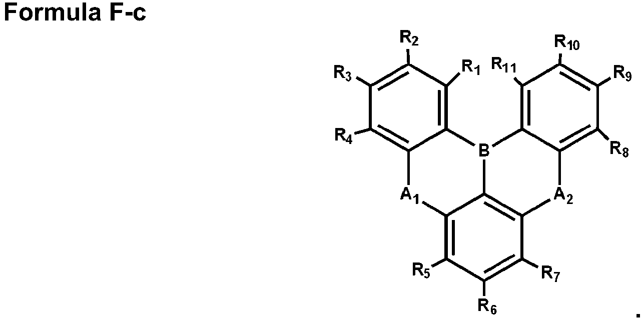

In Formula F-c, A₁ and A₂ may each independently be O, S, Se, or NRₘ, and Rₘ may be a hydrogen atom, a deuterium atom, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms. R₁ to R₁₁ may each independently be a hydrogen atom, a deuterium atom, a halogen atom, a cyano group, a substituted or unsubstituted amine group, a substituted or unsubstituted boryl group, a substituted or unsubstituted oxy group, a substituted or unsubstituted thiol group, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms, and/or are bonded to an adjacent group to form a ring.

In Formula F-c, A₁ and A₂ may each independently be bonded to substituents of an adjacent ring to form a condensed ring. For example, when A₁ and A₂ may each independently be NRₘ, A₁ may be bonded to R₄ or R₅ to form a ring. In some embodiments, A₂ may be bonded to R₇ or R₈ to form a ring.

In an embodiment, the emission layer EML may further include, as a suitable dopant material, styryl derivatives (e.g., 1,4-bis[2-(3-N-ethylcarbazoryl)vinyl]benzene (BCzVB), 4-(di-p-tolylamino)-4'-[(di-p-tolylamino)styryl]stilbene (DPAVB), and N-(4-((E)-2-(6-((E)-4-(diphenylamino)styryl)naphthalen-2-yl)vinyl)phenyl)-N-phenylbenz enamine (N-BDAVBi), 4,4'-bis[2-(4-(N,N-diphenylamino)phenyl)vinyl]biphenyl(DPAVBi), perylene and the derivatives thereof (e.g., 2,5,8,11-tetra-t-butylperylene (TBP)), pyrene and the derivatives thereof (e.g., 1,1-dipyrene, 1,4-dipyrenylbenzene, 1,4-bis(N,N-diphenylamino)pyrene), etc.

The emission layer EML may further include a suitable phosphorescence dopant material. For example, a metal complex containing iridium (Ir), platinum (Pt), osmium (Os), aurum (Au), titanium (Ti), zirconium (Zr), hafnium (Hf), europium (Eu), terbium (Tb), or thulium (Tm) may be utilized as a phosphorescent dopant. For example, iridium(III) bis(4,6-difluorophenylpyridinato-N,C2')picolinate (Flrpic), bis(2,4-difluorophenylpyridinato)-tetrakis(1-pyrazolyl)borate iridium(III) (Fir6), or platinum octaethyl porphyrin (PtOEP) may be utilized as a phosphorescent dopant. However, the embodiment of the present disclosure is not limited thereto.

The emission layer EML may include a quantum dot material. The core of the quantum dot may be selected from among a Group II-VI compound, a Group III-VI compound, a Group I-III-IV compound, a Group III-V compound, a Group III-II-V compound, a Group IV-VI compound, a Group IV element, a Group IV compound, and one or more combinations thereof.

The Group II-VI compound may be selected from the group including (e.g., consisting of) a binary compound selected from the group including (e.g., consisting of) CdSe, CdTe, CdS, ZnS, ZnSe, ZnTe, ZnO, HgS, HgSe, HgTe, MgSe, MgS, and one or more mixtures thereof, a ternary compound selected from the group including (e.g., consisting of) CdSeS, CdSeTe, CdSTe, ZnSeS, ZnSeTe, ZnSTe, HgSeS, HgSeTe, HgSTe, CdZnS, CdZnSe, CdZnTe, CdHgS, CdHgSe, CdHgTe, HgZnS, HgZnSe, HgZnTe, MgZnSe, MgZnS, and one or more mixtures thereof, and a quaternary compound selected from the group including (e.g., consisting of) HgZnTeS, CdZnSeS, CdZnSeTe, CdZnSTe, CdHgSeS, CdHgSeTe, CdHgSTe, HgZnSeS, HgZnSeTe, HgZnSTe, and one or more mixtures thereof.

The Group III-VI compound may include a binary compound such as In₂S₃ and/or In₂Se₃, a ternary compound such as InGaS₃ and/or InGaSe₃, or one or more combinations thereof.

The Group I-III-VI compound may be selected from a ternary compound selected from the group including (e.g., consisting of) AgInS, AgInS₂, CuInS, CuInS₂, AgGaS₂, CuGaS₂ CuGaO₂, AgGaO₂, AgAlO₂, and one or more mixtures thereof, and a quaternary compound such as AgInGaS₂ and/or CuInGaS₂.

The Group III-V compound may be selected from the group including (e.g., consisting of) a binary compound selected from the group including (e.g., consisting of) GaN, GaP, GaAs, GaSb, AlN, AlP, AlAs, AlSb, InN, InP, InAs, InSb, and one or more mixtures thereof, a ternary compound selected from the group including (e.g., consisting of) GaNP, GaNAs, GaNSb, GaPAs, GaPSb, AINP, AlNAs, AlNSb, AlPAs, AlPSb, InGaP, InAlP, InNP, InNAs, InNSb, InPAs, InPSb, and one or more mixtures thereof, and a quaternary compound selected from the group including (e.g., consisting of) GaAlNP, GaAINAs, GaAINSb, GaAlPAs, GaAlPSb, GalnNP, GalnNAs, GalnNSb, GalnPAs, GalnPSb, InAlNP, InAINAs, InAlNSb, InAIPAs, InAlPSb, and one or more mixtures thereof. In some embodiments, the Group III-V compound may further include a Group II metal. For example, InZnP, etc. may be selected as a Group III-II-V compound.

The Group IV-VI compound may be selected from the group including (e.g., consisting of) a binary compound selected from the group including (e.g., consisting of) SnS, SnSe, SnTe, PbS, PbSe, PbTe, and one or more mixtures thereof, a ternary compound selected from the group including (e.g., consisting of) SnSeS, SnSeTe, SnSTe, PbSeS, PbSeTe, PbSTe, SnPbS, SnPbSe, SnPbTe, and one or more mixtures thereof, and a quaternary compound selected from the group including (e.g., consisting of) SnPbSSe, SnPbSeTe, SnPbSTe, and one or more mixtures thereof. The Group IV element may be selected from the group including (e.g., consisting of) Si, Ge, and one or more mixtures thereof. The Group IV compound may be a binary compound selected from the group including (e.g., consisting of) SiC, SiGe, and one or more mixtures thereof.

In this embodiment, a binary compound, a ternary compound, or a quaternary compound may be present in particles in a substantially uniform concentration distribution, or may be present in substantially the same particle in a partially different concentration distribution. In some embodiments, a core/shell structure in which one quantum dot surrounds another quantum dot may also be possible. The core/shell structure may have a concentration gradient in which the concentration of elements present in the shell decreases toward the core.

In some embodiments, a quantum dot may have the above-described core-shell structure including a core containing nanocrystals and a shell around (e.g., surrounding) the core. The shell of the quantum dot may serve as a protection layer to prevent or reduce the chemical deformation of the core so as to maintain semiconductor properties, and/or a charging layer to impart electrophoresis properties to the quantum dot. The shell may be a single layer or a multilayer. An example of the shell of the quantum dot may include a metal or non-metal oxide, a semiconductor compound, or one or more combinations thereof.

For example, the metal or non-metal oxide may be a binary compound such as SiO₂, Al₂O₃, TiO₂, ZnO, MnO, Mn₂O₃, Mn₃O₄, CuO, FeO, Fe₂O₃, Fe₃O₄, CoO, Co₃O₄, and/or NiO, or a ternary compound such as MgAl₂O₄, CoFe₂O₄, NiFe₂O₄, and/or CoMn₂O₄, but the embodiment of the present disclosure is not limited thereto.

Also, the semiconductor compound may be, for example, CdS, CdSe, CdTe, ZnS, ZnSe, ZnTe, ZnSeS, ZnTeS, GaAs, GaP, GaSb, HgS, HgSe, HgTe, InAs, InP, InGaP, InSb, AlAs, AlP, AlSb, etc., but the embodiment of the present disclosure is not limited thereto.

The quantum dot may have a full width of half maximum (FWHM) of a light emission wavelength spectrum of about 45 nm or less, about 40 nm or less, and about 30 nm or less, and color purity or color reproducibility may be improved in the above range. In some embodiments, light emitted through such a quantum dot may be emitted in all directions, and thus a wide viewing angle may be improved (increased).

In some embodiments, although the form of a quantum dot is not limited as long as it is a form commonly utilized in the art, For example, a quantum dot in the form of substantially spherical, pyramidal, multi-arm, or cubic nanoparticles, nanotubes, nanowires, nanofibers, nanoparticles, etc. may be utilized.

The quantum dot may control the color of emitted light according to the particle size thereof. Accordingly, the quantum dot may have one or more suitable light emission colors such as blue, red, and/or green.

In an embodiment, the emission layer EML may include two different hosts, a sensitizer, and a dopant. For example, the emission layer EML may include a phosphorescent sensitizer and a thermally activated delayed fluorescence (TADF) sensitizer as the sensitizer.

For example, the emission layer EML may include a hole transport host and an electron transport host. In the light emitting device ED of an embodiment, the hole transport host and the electron transport host may form an exciplex. A triplet energy of the exciplex formed by the hole transport host and the electron transport host may correspond to T1 that is a gap between a lowest unoccupied molecular orbital (LUMO) energy level of the electron transport host and a highest occupied molecular orbital (HOMO) energy level of the hole transport host.

In an organic light emitting device of an embodiment, the lowest excited triplet energy level (T1) of the exciplex formed by the hole transport host and the electron transport host may be about 2.4 eV to about 3.0 eV.

In some embodiments, the lowest excited triplet energy level (T1) of the exciplex may be a value smaller than an energy gap of each host material. Therefore, the exciplex may have the lowest excited tirplet energy level (T1) of about 3.0 eV or less that is an energy gap between the hole transport host and the electron transport host.

In some embodiments, the emission layer EML of the light emitting device ED may be to emit blue light. For example, the emission layer EML of the light emitting device ED of an embodiment may be to emit blue light having a center wavelenth of about 490 nm to about 470 nm. However, the embodiment of the present disclosure is not limited thereto, and the emission layer EML may emit blue light having a center wavelength greater than about 470 nm, or may emit green light or red light.

In each light emitting device ED of embodiments illustrated in FIGS. 3 to 6, the electron transport region ETR is provided on the emission layer EML. The electron transport region ETR may include at least one of the hole blocking layer HBL, the electron transport layer ETL, or the electron injection layer EIL, but the embodiment of the present disclosure is not limited thereto.

The electron transport region ETR may have a single layer formed of a single material, a single layer formed of a plurality of different materials, or a multilayer structure including a plurality of layers formed of a plurality of different materials.

For example, the electron transport region ETR may have a single layer structure of the electron injection layer EIL or the electron transport layer ETL, and may have a single layer structure formed of an electron injection material and an electron transport material. In some embodiments, the electron transport region ETR may have a single layer structure formed of a plurality of different materials, or may have a structure in which an electron transport layer ETL/electron injection layer EIL, a hole blocking layer HBL/electron transport layer ETL/electron injection layer EIL are stacked in order from the emission layer EML, but the embodiment of the present disclosure is not limited thereto. The electron transport region ETR may have a thickness, for example, from about 1,000 Å to about 1,500 Å.

The electron transport region ETR may be formed by utilizing one or more suitable methods such as a vacuum deposition method, a spin coating method, a cast method, a Langmuir-Blodgett (LB) method, an inkjet printing method, a laser printing method, a laser induced thermal imaging (LITI) method, etc.

In an embodiment, the electron transport region ETR includes the polycyclic compound represented by Formula 1.

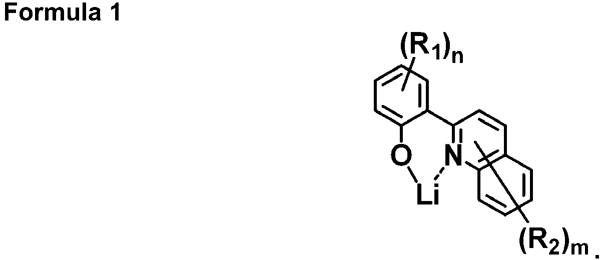

In Formula 1, R₁ and R₂ may each independently be a hydrogen atom, a deuterium atom, a halogen atom, a substituted or unsubstituted silyl group, a substituted or unsubstituted amine group, a substituted or unsubstituted alkyl group having 1 to 20 carbon atoms, a substituted or unsubstituted alkenyl group having 2 to 20 carbon atoms, a substituted or unsubstituted aryl group having 6 to 30 ring-forming carbon atoms, or a substituted or unsubstituted heteroaryl group having 2 to 30 ring-forming carbon atoms, and optionally may be bonded to an adjacent group to form a ring. For example, R₁ may be a substituted or unsubstituted alkyl group having 1 to 20 carbon atoms, and R₂ may be a hydrogen atom.

n is an integer of 0 to 4. For example, n may be 1.

m is an integer of 0 to 6. For example, m may be 0. The structure in which m is 0 may be the same as the structure in which m is an integer of 1 or more, and at least one R₂ is a hydrogen atom.

In the present disclosure, the electron transport region ETR includes the polycyclic compound represented by Formula 1 containing a quinoline skeleton. For example, the polycyclic compound represented by Formula 1 has a structure in which a lithium ion (Li⁺) is linked both (e.g., simultaneously) to a nitrogen atom contained in the quinoline and to an oxygen atom linked to a phenyl group linked to a carbon atom contained in the quinoline to thereby form a ring.

In an embodiment, the polycyclic compound represented by Formula 1 may be represented by Formula 1-1:

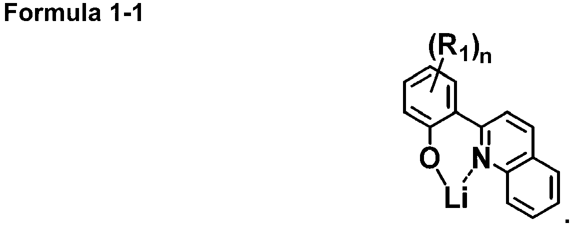

Formula 1-1 is the embodiment in which m is 0 in Formula 1. Formula 1-1 may be the same as the embodiment in Formula 1, in which m is 6, and each of six R₂'s is a hydrogen atom.

In an embodiment, the polycyclic compound represented by Formula 1 may be represented by Formula 1-2:

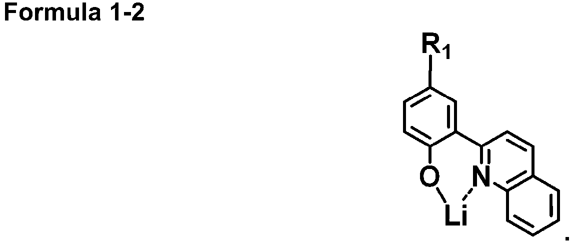

Formula 1-2 is an embodiment in which in Formula 1, n is 1 and m is 0. Formula 1-2 may be the same as the embodiment in which in Formula 1, n is 1, m is 6, and each of six R₂s is a hydrogen atom.

In an embodiment, the polycyclic compound represented by Formula 1 may include at least one among the compounds represented in Compound Group 1:

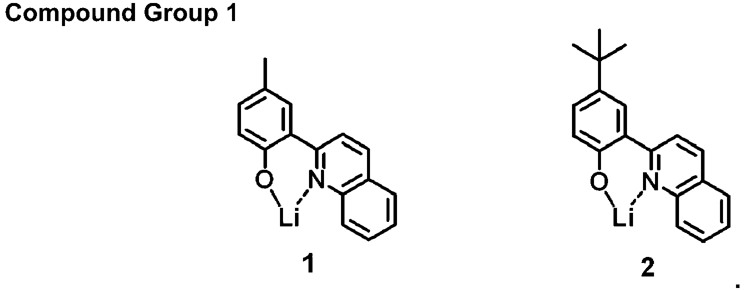

The polycyclic compound represented by Formula 1 has a low refractive index. For example, the polycyclic compound represented by Formula 1 has a refractive index of less than about 1.7 with respect to light having a wavelength of about 450 nm. For example, the polycyclic compound represented by Formula 1 may have a refractive index of about 1.6 to about 1.7 (but exclusive of 1.7, i.e., less than 1.7) with respect to light having a wavelength of about 450 nm. In the disclosure, the term "refractive index of a compound" is to be understood to refer to a refractive index of a single layer composed of the compound.

In Compound Group 1, the refractive index of Compound 1 is about 1.65, and the refractive index of Compound 2 is about 1.63. Referring to the fact that 8-hydroxyl-lithium quinolate (Liq), which is a suitable compound, has a refractive index of about 1.71 with respect to light having a wavelength of about 450 nm, the polycyclic compound represented by Formula 1 may be a material having a refractive index relatively lower than the Liq (i.e., 8-hydroxyl-lithium quinolate).

The electron transport region ETR may include the polycyclic compound represented by Formula 1, which is a low refractive material, and thus may have a refractive index of less than about 1.8. For example, the electron transport region ETR may have a refractive index of less than about 1.8 with respect to light having a wavelength of about 450 nm.

For example, the electron transport layer ETL included in the electron transport region ETR may include the polycyclic compound represented by Formula 1, and thus may have a refractive index of less than about 1.8. For example, the electron transport layer ETL has a refractive index of less than about 1.8 with respect to light having a wavelength of about 450 nm. The refractive index of the electron transport layer ETL may be about 1.7 to about 1.8 (but exclusive of 1.8, i.e., less than 1.8). The light emitting device ED including the electron transport layer ETL having a refractive index of about 1.8 or more may have a decrease in the luminous efficiency compared to the light emitting device ED including the electron transport layer ETL having a refractive index of less than about 1.8. This will be described in more detail below.

The electron transport region ETR may further include at least one among compounds represented by Formula 2 and Formula 3. The compounds represented by Formula 2 or Formula 3 may be electron transport materials.

The electron transport region ETR of an embodiment may include a low refractive material represented by Formula 1 and an electron transport material represented by Formula 2 or Formula 3.

For example, the electron transport region ETR may include the polycyclic compound represented by Formula 1 and the compound represented by Formula 2.

In some embodiments, the electron transport region ETR may include the polycyclic compound represented by Formula 1 and the compound represented by Formula 3.

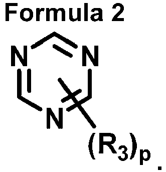

In Formula 2, each R₃ is independently a hydrogen atom, a deuterium atom, a halogen atom, a substituted or unsubstituted silyl group, a substituted or unsubstituted amine group, a substituted or unsubstituted alkyl group having 1 to 20 carbon atoms, a substituted or unsubstituted alkenyl group having 2 to 20 carbon atoms, a substituted or unsubstituted aryl group having 6 to 30 ring-forming carbon atoms, or a substituted or unsubstituted heteroaryl group having 2 to 30 ring-forming carbon atoms, and optionally may be bonded to an adjacent group to form a ring. For example, each R₃ may independently be a substituted or unsubstituted aryl group having 6 to 30 ring-forming carbon atoms, or a substituted or unsubstituted heteroaryl group having 2 to 30 ring-forming carbon atoms. For example, each R₃ may independently be a substituted or unsubstituted phenyl group, or a substituted or unsubstituted carbazole group. When R₃ is provided in plurality, a plurality of R₃'s may be the same as each other or at least one may be different from the others.

p is an integer of 0 to 3. For example, p may be 3.

In an embodiment, the compound represented by Formula 2 above may be represented by Compound A. The refractive index of Compound A with respect to light having a wavelength of about 450 nm is about 1.88.

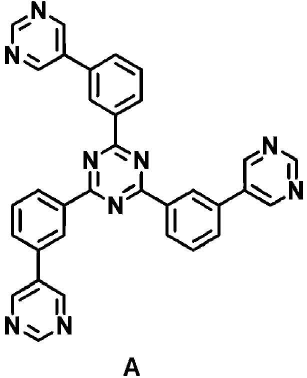

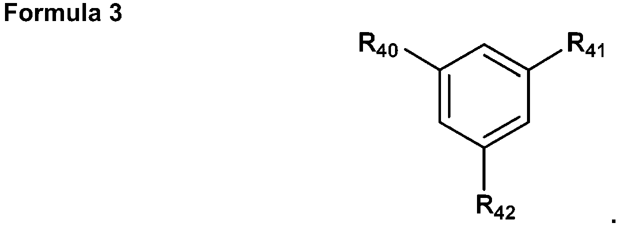

In Formula 3, R₄₀ to R₄₂ may each independently be a hydrogen atom, a deuterium atom, a halogen atom, a cyano group, a substituted or unsubstituted silyl group, a substituted or unsubstituted thiol group, a substituted or unsubstituted oxy group, a substituted or unsubstituted amine group, a substituted or unsubstituted alkyl group having 1 to 20 carbon atoms, a substituted or unsubstituted alkenyl group having 2 to 20 carbon atoms, a substituted or unsubstituted aryl group having 6 to 30 ring-forming carbon atoms, or a substituted or unsubstituted heteroaryl group having 2 to 30 ring-forming carbon atoms, and optionally may be are bonded to an adjacent group to form a ring. For example, R₄₀ to R₄₂ may each independently be a substituted or unsubstituted aryl group having 6 to 30 ring-forming carbon atoms, or a substituted or unsubstituted heteroaryl group having 2 to 30 ring-forming carbon atoms. R₄₀ to R₄₂ may be different from each other, or at least two may be the same as each other.

In an embodiment, the compound represented by Formula 3 above may be represented by Compound B. The refractive index of Compound B with respect to light having a wavelength of about 450 nm is about 1.82.

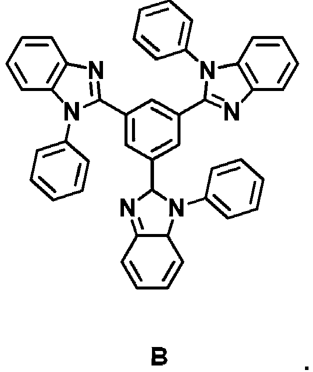

The electron transport region ETR may further include a suitable compound. For example, the electron transport region ETR may include a compound represented by Formula ET-1:

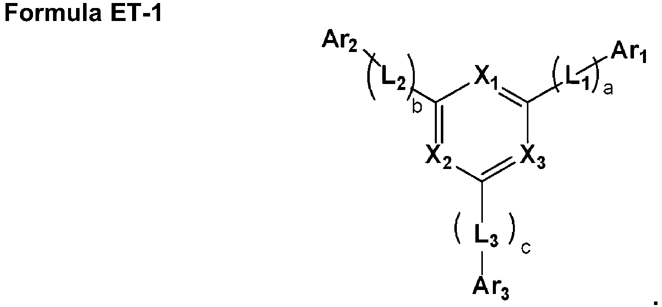

In Formula ET-1, at least one among X₁ to X₃ is N, and the rest are CRₐ. Rₐ may be a hydrogen atom, a deuterium atom, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms. Ar₁ to Ar₃ may each independently be a hydrogen atom, a deuterium atom, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms.

In Formula ET-1, a to c may each independently be an integer of 0 to 10. In Formula ET-1, L₁ to L₃ may each independently be a direct linkage, a substituted or unsubstituted arylene group having 6 to 30 ring-forming carbon atoms, or a substituted or unsubstituted heteroarylene group having 2 to 30 ring-forming carbon atoms. In some embodiments, when a to c are an integer of 2 or more, L₁ to L₃ may each independently be a substituted or unsubstituted arylene group having 6 to 30 ring-forming carbon atoms, or a substituted or unsubstituted heteroarylene group having 2 to 30 ring-forming carbon atoms.

The electron transport region ETR may include an anthracene-based compound. However, the embodiment of the present disclosure is not limited thereto, and the electron transport region ETR may include, for example, tris(8-hydroxyquinolinato)aluminum (Alq₃), 1,3,5-tri[(3-pyridyl)-phen-3-yl]benzene, 2,4,6-tris(3'-(pyridin-3-yl)biphenyl-3-yl)-1,3,5-triazine, 2-(4-(N-phenylbenzoimidazol-1-yl)phenyl)-9,10-dinaphthylanthracene, 1,3,5-tri(1-phenyl-1H-benzo[d]imidazol-2-yl)benzene (TPBi), 2,9-dimethyl-4,7-diphenyl-1,10-phenanthroline (BCP), 4,7-diphenyl-1,10-phenanthroline (Bphen), 3-(4-biphenylyl)-4-phenyl-5-tert-butylphenyl-1,2,4-triazole (TAZ), 4-(naphthalen-1-yl)-3,5-diphenyl-4H-1,2,4-triazole (NTAZ), 2-(4-biphenylyl)-5-(4-tert-butylphenyl)-1,3,4-oxadiazole (tBu-PBD), bis(2-methyl-8-quinolinolato-N1,O8)-(1,1'-biphenyl-4-olato)aluminum (BAlq), berylliumbis(benzoquinolin-10-olate (Bebq₂), 9,10-di(naphthalene-2-yl)anthracene (ADN), 1,3-Bis[3,5-di(pyridin-3-yl)phenyl]benzene (BmPyPhB), or one or more mixtures thereof.

The electron transport region ETR may include at least one among Compound ET1 to Compound ET36:

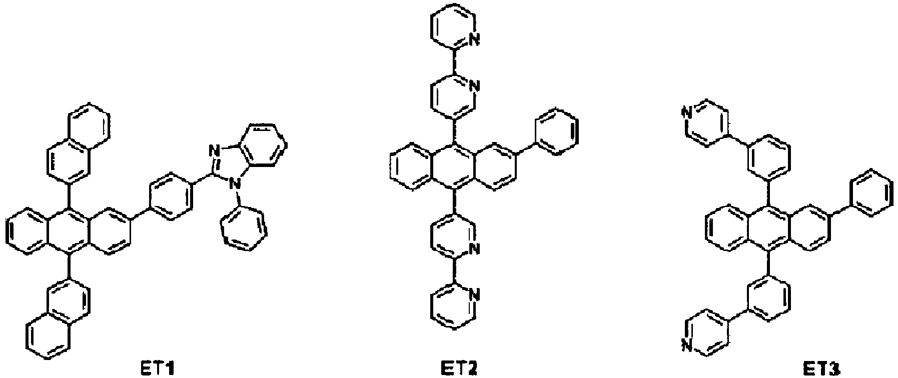

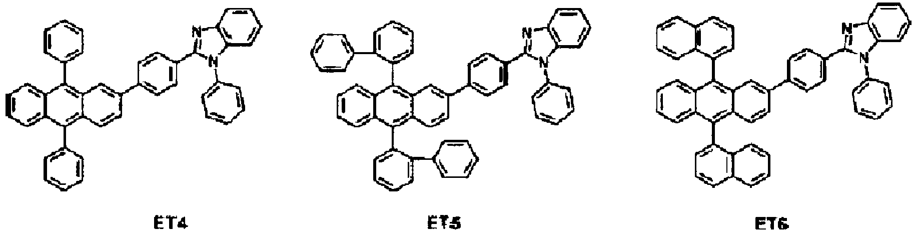

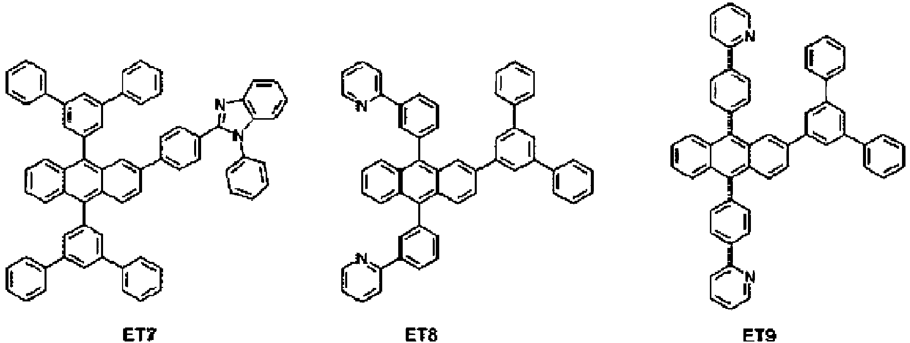

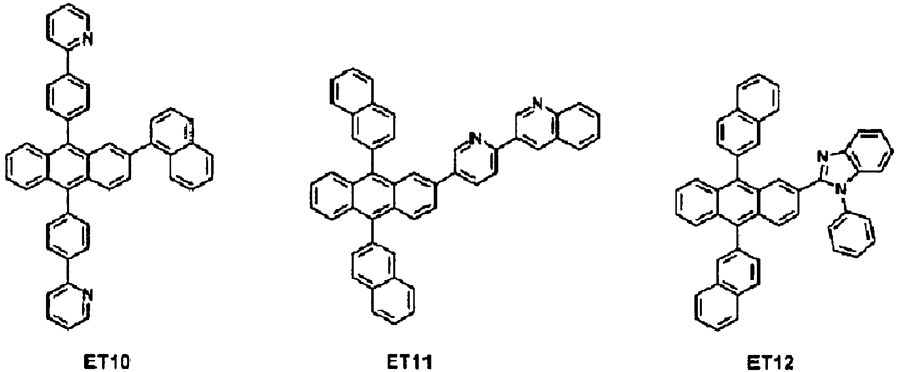

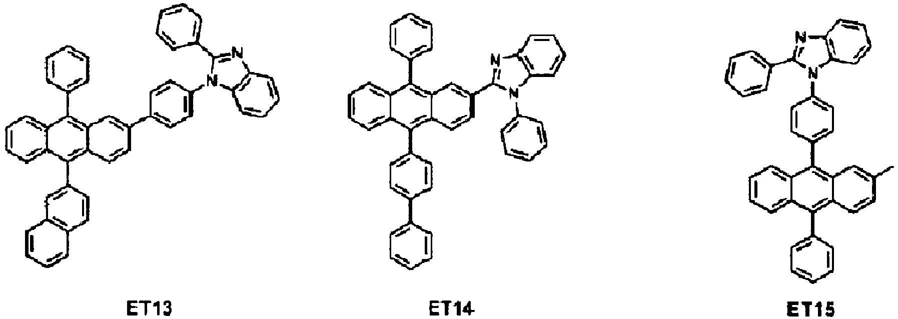

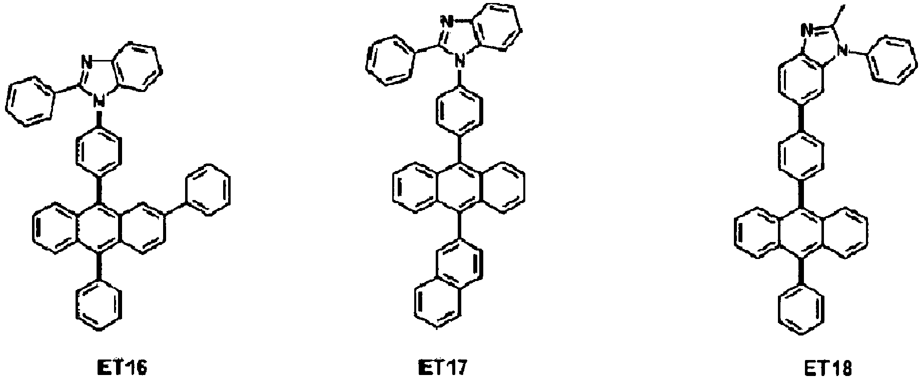

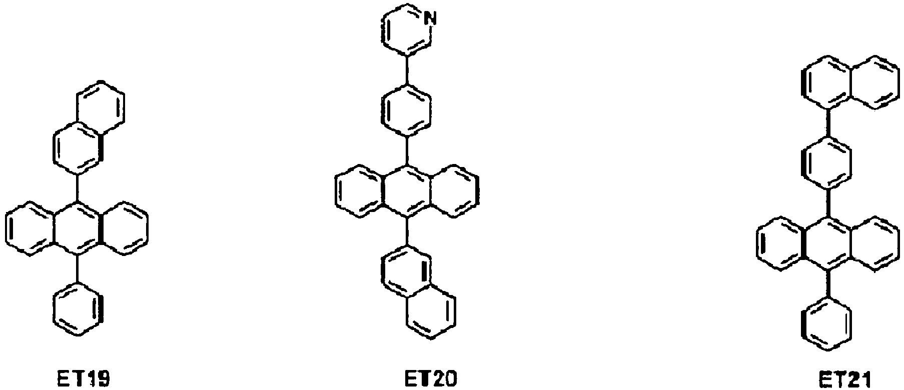

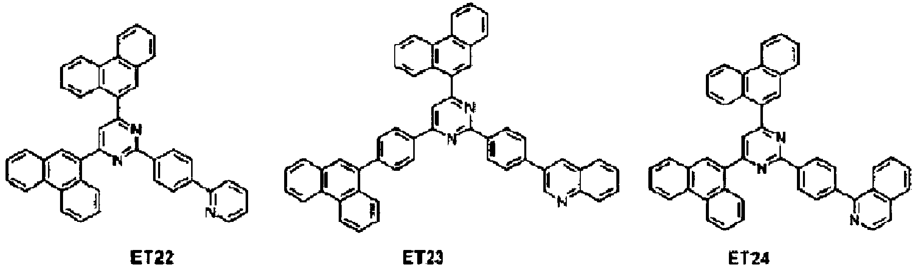

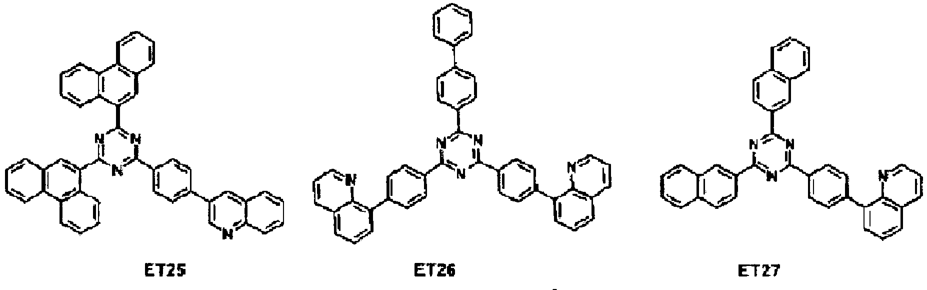

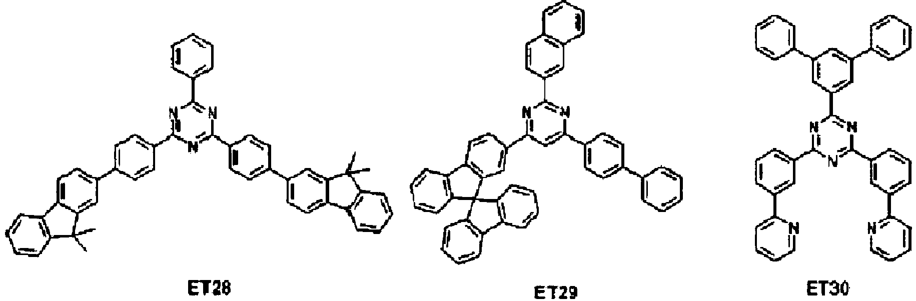

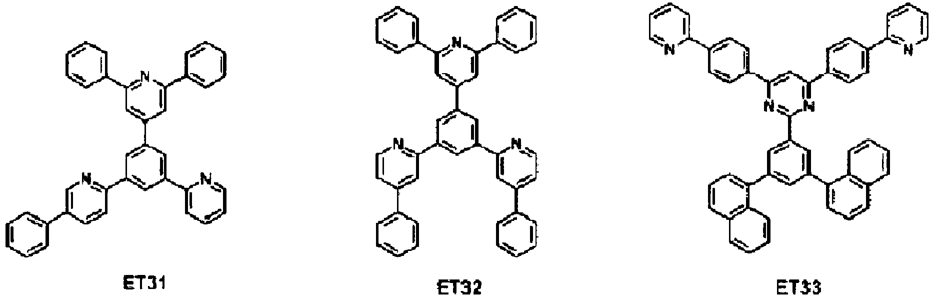

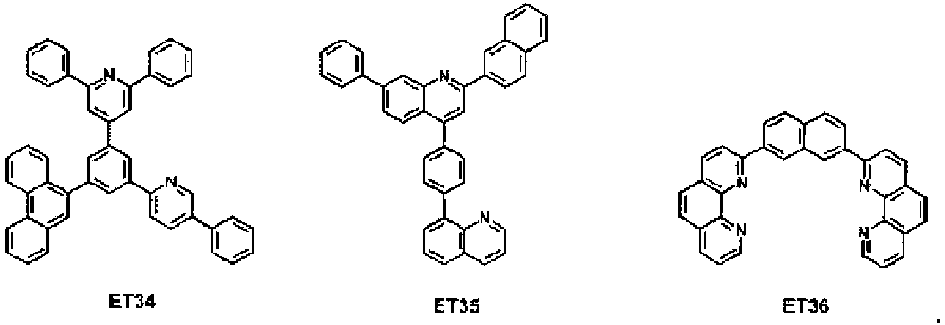

In some embodiments, the electron transport regions ETR may include a metal halide such as LiF, NaCl, CsF, RbCl, Rbl, Cul, and/or KI, a lanthanide metal such as Yb, and a co-deposited material of the metal halide and the lanthanide metal. For example, the electron transport region ETR may include KI:Yb, Rbl:Yb, LiF:Yb, etc. as a co-deposited material. In some embodiments, the electron transport region ETR may be formed utilizing a metal oxide such as Li₂O, BaO, etc., but the embodiment of the present disclosure is not limited thereto. The electron transport region ETR may also be formed of a mixture material of an electron transport material and an insulating organometallic salt. The organometallic salt may be a material having an energy band gap of about 4 eV or more. For example, the organometallic salt may include, for example, a metal acetate, a metal benzoate, a metal acetoacetate, a metal acetylacetonate, and/or a metal stearate.

The electron transport region ETR may further include at least one of 2,9-dimethyl-4,7-diphenyl-1,10-phenanthroline (BCP), diphenyl(4-(triphenylsilyl)phenyl)phosphine oxide (TSPO1), or 4,7-diphenyl-1,10-phenanthroline (Bphen) in addition to the above-described materials, but the embodiment of the present disclosure is not limited thereto.

The electron transport region ETR may include the above-described compounds of the hole transport region in at least one of the electron injection layer EIL, the electron transport layer ETL, or the hole blocking layer HBL.

In an embodiment, the electron transport region ETR includes the electron transport layer ETL, and the electron transport layer ETL may include the polycyclic compound represented by Formula 1. For example, the electron transport layer ETL includes the polycyclic compound represented by Formula 1, and includes at least one of the compound represented by Formula 2 or the compound represented by Formula 3.

For example, the electron transport layer ETL may include the polycyclic compound represented by Formula 1 and the compound represented by Formula 2.

For example, the electron transport layer ETL may include the polycyclic compound represented by Formula 1 and the compound represented by Formula 3.

The electron transport layer ETL of an embodiment includes the polycylic compound represented by Formula 1, and thus has a refractive index of less than about 1.8 with respect to light having a wavelength of about 450 nm. For example, the refractive index of the electron transport layer ETL may be about 1.7 to about 1.8 (but exclusive of 1.8, i.e., less than 1.8).

The electron transport layer ETL has a thickness of about 250 Å or more. For example, the thickness of the electron transport layer ETL may satisfy a value of about 250 Å to about 1,000 Å. For example, the thickness of the electron transport layer ETL may be from about 250 Å to about 500 Å. When the thickness of the electron transport layer ETL satisfies the aforementioned range, satisfactory (suitable) electron transport characteristics may be obtained without a substantial increase in a driving voltage.

When the electron transport region ETR includes the electron injection layer EIL, the electron injection layer EIL may have a thickness of about 1 Å to about 100 Å, for example, about 3 Å to about 90 Å. When the thickness of the electron injection layer EIL satisfies the above-described range, satisfactory electron injection characteristics may be obtained without a substantial increase in a driving voltage.

The second electrode EL2 may be provided on the electron transport region ETR. The second electrode EL2 may be a common electrode. The second electrode EL2 may be a cathode or an anode, but the embodiment of the present disclosure is not limited thereto. For example, when the first electrode EL1 is an anode, the second electrode EL2 may be a cathode, and when the first electrode EL1 is a cathode, the second electrode EL2 may be an anode. The second electrode may include at least one selected from among Ag, Mg, Cu, Al, Pt, Pd, Au, Ni, Nd, Ir, Cr, Li, Ca, LiF, Mo, Ti, W, In, Sn, Zn, compounds comprising one or more of the foregoing elements, combinations of two or more of the foregoing elements or compounds, mixtures of two or more of the foregoing elements or compounds, and oxides thereof.

The second electrode EL2 may be a transmissive electrode, a transflective electrode, or a reflective electrode. When the second electrode EL2 is the transmissive electrode, the second electrode EL2 may be formed of a transparent metal oxide, for example, indium tin oxide (ITO), indium zinc oxide (IZO), zinc oxide (ZnO), indium tin zinc oxide (ITZO), etc.

When the second electrode EL2 is the transflective electrode or the reflective electrode, the second electrode EL2 may include Ag, Mg, Cu, Al, Pt, Pd, Au, Ni, Nd, Ir, Cr, Li, Ca, LiF/Ca, LiF/Al, Mo, Ti, Yb, W, or compounds or mixtures thereof (e.g., AgMg, AgYb, or MgAg). In some embodiments, the second electrode EL2 may have a multilayer structure including a reflective film or a transflective film formed of the above-described materials, and/or a transparent conductive film formed of ITO, IZO, ZnO, ITZO, etc. For example, the second electrode EL2 may include one or more of the above-described metal materials, combinations of at least two metal materials of the above-described metal materials, oxides of the above-described metal materials, and/or the like.

The second electrode EL2 may be connected with an auxiliary electrode. When the second electrode EL2 is connected with the auxiliary electrode, the resistance of the second electrode EL2 may be decreased.

In some embodiments, a capping layer CPL may further be on the second electrode EL2 of the light emitting device ED. The capping layer CPL may include a multilayer or a single layer.

In an embodiment, the capping layer CPL may be an organic layer and/or an inorganic layer. For example, when the capping layer CPL contains an inorganic material, the inorganic material may include an alkaline metal compound (for example, LiF), an alkaline earth metal compound (for example, MgF₂), SiON, SiNₓ, SiOy, etc.

For example, when the capping layer CPL contains an organic material, the organic material may include α-NPD, NPB, TPD, m-MTDATA, Alq₃, CuPc, N4,N4,N4',N4'-tetra(biphenyl-4-yl)biphenyl-4,4'-diamine (TPD15), 4,4',4"-tris(carbazol sol-9-yl)triphenylamine (TCTA), etc., or may include an epoxy resin, or an acrylate such as a methacrylate. However, the embodiment of the present disclosure is not limited thereto, and the capping layer CPL may include at least one among Compounds P1 to P5:

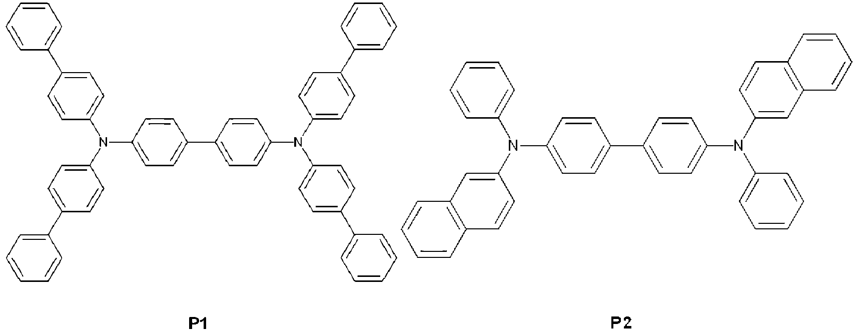

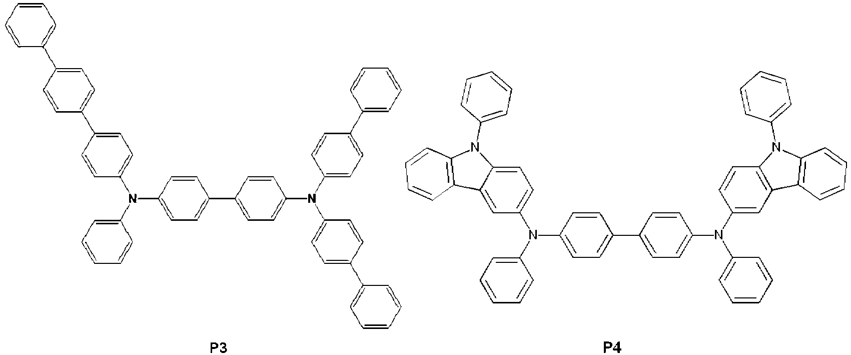

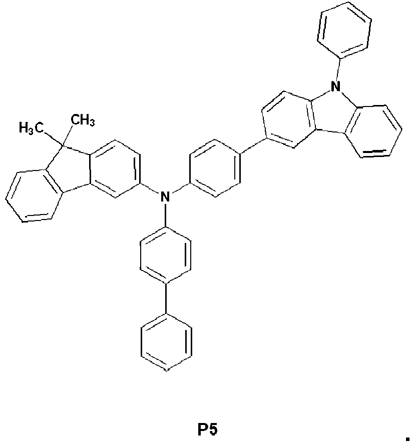

In some embodiments, the refractive index of the capping layer CPL may be about 1.6 or more. For example, the refractive index of the capping layer CPL may be about 1.6 or more with respect to light in a wavelength range of about 550 nm to about 660 nm.

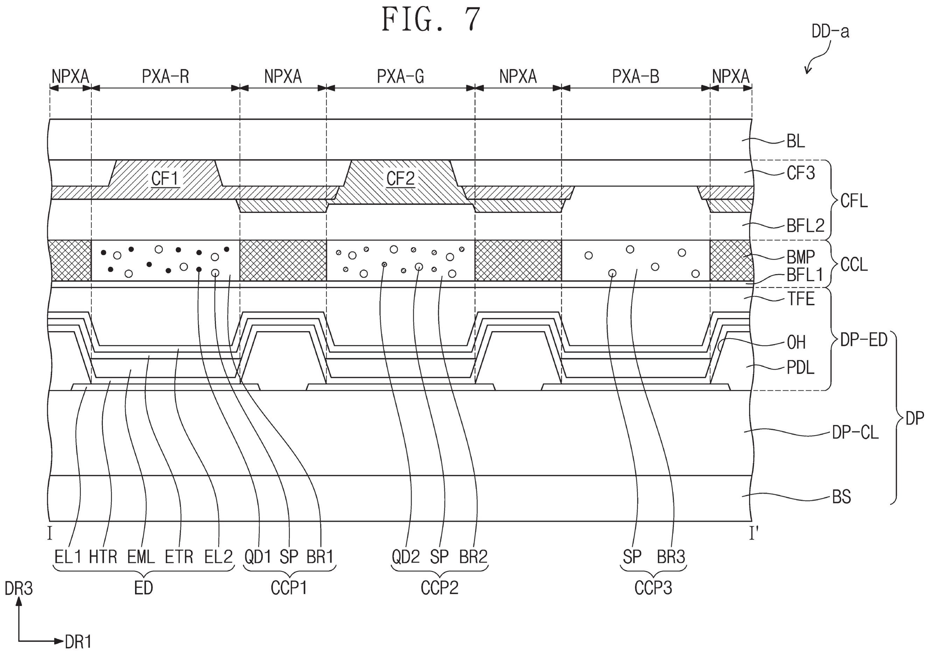

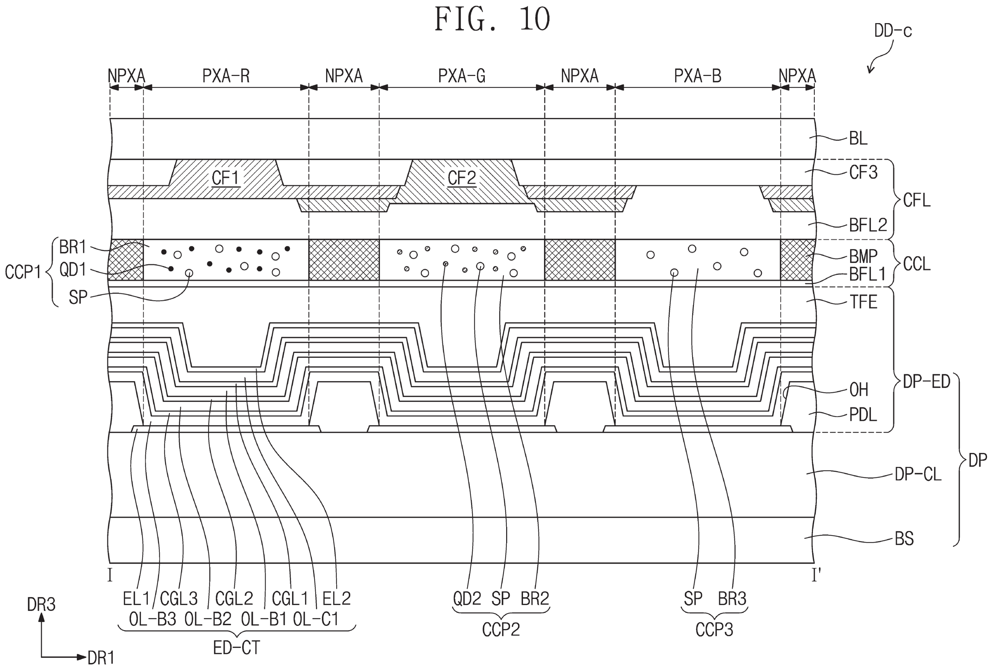

FIGS. 7 to 10 each are a cross-sectional view of a display device according to an embodiment of the present disclosure. Hereinafter, in describing the display apparatuses of embodiments with reference to FIGS. 7 to 10, the duplicated features which have been described in FIGS. 1 to 6 may not be described again, but their differences will be primarily described.

Referring to FIG. 7, the display apparatus DD-a according to an embodiment may include a display panel DP including a display device layer DP-ED, a light control layer CCL on the display panel DP, and a color filter layer CFL.

In an embodiment illustrated in FIG. 7, the display panel DP may include a base layer BS, a circuit layer DP-CL provided on the base layer BS, and the display device layer DP-ED, and the display device layer DP-ED may include a light emitting device ED.

The light emitting device ED may include a first electrode EL1, a hole transport region HTR on the first electrode EL1, an emission layer EML on the hole transport region HTR, an electron transport region ETR on the emission layer EML, and a second electrode EL2 on the electron transport region ETR. In some embodiments, the structures of the light emitting devices of FIGS. 3 to 6 as described above may be equally applied to the structure of the light emitting device ED illustrated in FIG. 7.

The electron transport region ETR of the light emitting device ED included in the display apparatus Dd-a according to an embodiment may include the above-described polycyclic compound of an embodiment.

Referring to FIG. 7, the emission layer EML may be disposed in an opening OH defined in a pixel defining film PDL. For example, the emission layer EML which is divided by the pixel defining film PDL and provided corresponding to each light emitting regions PXA-R, PXA-G, and PXA-B may emit light in substantially the same wavelength range. In the display apparatus DD of an embodiment, the emission layer EML may emit blue light. In some embodiments, the emission layer EML may be provided as a common layer in the entire light emitting regions PXA-R, PXA-G, and PXA-B.

The light control layer CCL may be disposed on the display panel DP. The light control layer CCL may include a light conversion body. The light conversion body may be a quantum dot, a phosphor, and/or the like. The light conversion body may emit provided light by converting the wavelength thereof. For example, the light control layer CCL may be a layer containing the quantum dot or a layer containing the phosphor.

The light control layer CCL may include a plurality of light control parts CCP1, CCP2 and CCP3. The light control parts CCP1, CCP2, and CCP3 may be spaced apart (separated) from each other.

Referring to FIG. 7, divided patterns BMP may be disposed between the light control parts CCP1, CCP2 and CCP3 which are spaced apart from each other, but the embodiment of the present disclosure is not limited thereto. FIG. 7 illustrates that the divided patterns BMP do not overlap the light control parts CCP1, CCP2 and CCP3, but at least a portion of the edges of the light control parts CCP1, CCP2 and CCP3 may overlap the divided patterns BMP.

The light control layer CCL may include a first light control part CCP1 containing a first quantum dot QD1 which converts first color light provided from the light emitting device ED into second color light, a second light control part CCP2 containing a second quantum dot QD2 which converts the first color light into third color light, and a third light control part CCP3 which transmits the first color light.

In an embodiment, the first light control part CCP1 may provide red light that is the second color light, and the second light control part CCP2 may provide green light that is the third color light. The third light control part CCP3 may provide blue light by transmitting the blue light that is the first color light provided from the light emitting device ED. For example, the first quantum dot QD1 may be a red quantum dot, and the second quantum dot QD2 may be a green quantum dot. The same as described above may be applied with respect to the quantum dots QD1 and QD2.

In some embodiments, the light control layer CCL may further include a scatterer SP. The first light control part CCP1 may include the first quantum dot QD1 and the scatterer SP, the second light control part CCP2 may include the second quantum dot QD2 and the scatterer SP, and the third light control part CCP3 may not include (e.g., may exclude) any quantum dot but may include the scatterer SP.

The scatterer SP may be inorganic particles. For example, the scatterer SP may include at least one of TiO₂, ZnO, Al₂O₃, SiO₂, or hollow silica. The scatterer SP may include any one selected from among TiO₂, ZnO, Al₂O₃, SiO₂, and hollow silica, or may be a mixture or mixtures of at least two materials selected from among TiO₂, ZnO, Al₂O₃, SiO₂, and hollow silica.

The first light control part CCP1, the second light control part CCP2, and the third light control part CCP3 each may include base resins BR1, BR2, and BR3 in which the quantum dots QD1 and QD2 and the scatterer SP are dispersed. In an embodiment, the first light control part CCP1 may include the first quantum dot QD1 and the scatterer SP dispersed in a first base resin BR1, the second light control part CCP2 may include the second quantum dot QD2 and the scatterer SP dispersed in a second base resin BR2, and the third light control part CCP3 may include the scatterer SP dispersed in a third base resin BR3. The base resins BR1, BR2, and BR3 are media in which the quantum dots QD1 and QD2 and the scatterer SP are dispersed, and may be formed of one or more suitable resin compositions, which may be generally referred to as a binder. For example, the base resins BR1, BR2, and BR3 may be acrylic-based resins, urethane-based resins, silicone-based resins, epoxy-based resins, etc. The base resins BR1, BR2, and BR3 may be transparent resins. In an embodiment, the first base resin BR1, the second base resin BR2, and the third base resin BR3 may be the same as or different from each other.

The light control layer CCL may include a barrier layer BFL1. The barrier layer BFL1 may serve to prevent or reduce the penetration of moisture and/or oxygen (hereinafter, referred to as 'moisture/oxygen'). The barrier layer BFL1 may be disposed on the light control parts CCP1, CCP2, and CCP3 to block or reduce the light control parts CCP1, CCP2 and CCP3 from being exposed to moisture/oxygen. In some embodiments, the barrier layer BFL1 may cover the light control parts CCP1, CCP2, and CCP3. In some embodiments, the barrier layer BFL2 may be provided between the color filter layer CFL and the light control parts CCP1, CCP2, and CCP3.

The barrier layers BFL1 and BFL2 may include at least one inorganic layer. For example, the barrier layers BFL1 and BFL2 may include an inorganic material. For example, the barrier layers BFL1 and BFL2 may include a silicon nitride, an aluminum nitride, a zirconium nitride, a titanium nitride, a hafnium nitride, a tantalum nitride, a silicon oxide, an aluminum oxide, a titanium oxide, a tin oxide, a cerium oxide, a silicon oxynitride, a metal thin film which secures a transmittance, etc. In some embodiments, the barrier layers BFL1 and BFL2 may further include an organic film. The barrier layers BFL1 and BFL2 may be formed of a single layer or a plurality of layers.

In the display apparatus DD of an embodiment, the color filter layer CFL may be disposed on the light control layer CCL. For example, the color filter layer CFL may be directly disposed on the light control layer CCL. In this embodiment, the barrier layer BFL2 may not be provided.

The color filter layer CFL may include color filters CF1, CF2, and CF3. The color filter layer CFL may include a first filter CF1 configured to transmit the second color light, a second filter CF2 configured to transmit the third color light, and a third filter CF3 configured to transmit the first color light. For example, the first filter CF1 may be a red filter, the second filter CF2 may be a green filter, and the third filter CF3 may be a blue filter. The filters CF1, CF2, and CF3 each may include a polymeric photosensitive resin and/or a pigment or dye. The first filter CF1 may include a red pigment or dye, the second filter CF2 may include a green pigment or dye, and the third filter CF3 may include a blue pigment or dye. In some embodiments, of the present disclosure is not limited thereto, and the third filter CF3 may not include (e.g., may exclude) a pigment or dye. The third filter CF3 may include a polymeric photosensitive resin and may not include (e.g., may exclude) a pigment or dye. The third filter CF3 may be transparent. The third filter CF3 may be formed of a transparent photosensitive resin.

Furthermore, in an embodiment, the first filter CF1 and the second filter CF2 may be a yellow filter. The first filter CF1 and the second filter CF2 may not be separated but be provided as one filter. The first to third filters CF1, CF2, and CF3 may be disposed corresponding to the red light emitting region PXA-R, the green light emitting region PXA-G, and the blue light emitting region PXA-B, respectively.

In some embodiments, the color filter layer CFL may include a light shielding part. The color filter layer CFL may include a light shielding part disposed to overlap at the boundaries of neighboring filters CF1, CF2, and CF3. The light shielding part may be a black matrix. The light shielding part may include an organic light shielding material and/or an inorganic light shielding material containing a black pigment or dye. The light shielding part may separate boundaries between the adjacent filters CF1, CF2, and CF3. In some embodiments, the light shielding part may be formed of a blue filter.

A base substrate BL may be disposed on the color filter layer CFL. The base substrate BL may be a member which provides a base surface in which the color filter layer CFL, the light control layer CCL, and/or the like are disposed. The base substrate BL may be a glass substrate, a metal substrate, a plastic substrate, etc. However, the embodiment of the present disclosure is not limited thereto, and the base substrate BL may be an inorganic layer, an organic layer, and/or a composite material layer. In some embodiments, the base substrate BL may not be provided.

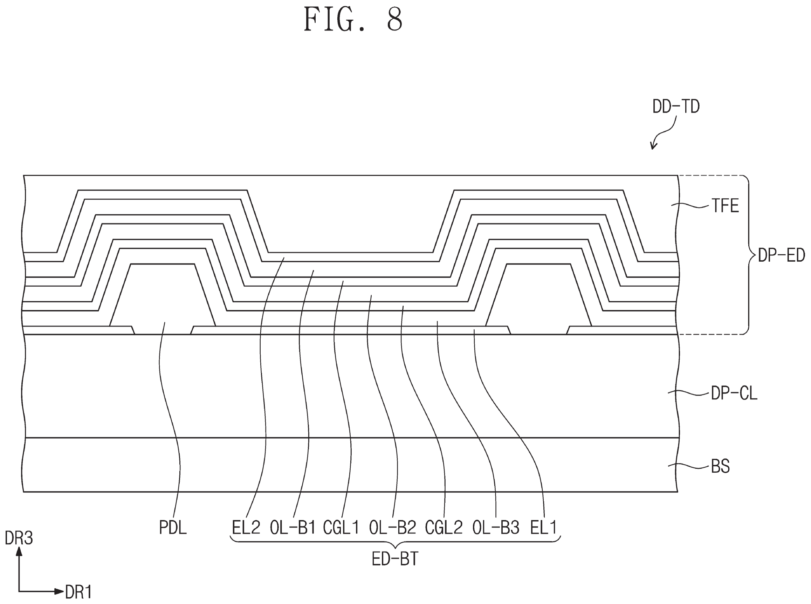

FIG. 8 is a cross-sectional view illustrating a portion of a display apparatus according to an embodiment of the present disclosure. FIG. 8 illustrates a cross-sectional view of a part corresponding to the display panel DP of FIG. 7. In the display apparatus DD-TD of an embodiment, the light emitting device ED-BT may include a plurality of light emitting structures OL-B1, OL-B2, and OL-B3. The light emitting device ED-BT may include a first electrode EL1 and a second electrode EL2 which face each other, and the plurality of light emitting structures OL-B1, OL-B2, and OL-B3 sequentially stacked in the thickness direction between the first electrode EL1 and the second electrode EL2 (e.g., stacked from the first electrode EL1). The light emitting structures OL-B1, OL-B2, and OL-B3 each may include an emission layer EML (FIG. 7) and may also include a hole transport region HTR and an electron transport region ETR disposed with the emission layer EML (FIG. 7) located therebetween.

For example, the light emitting device ED-BT included in the display apparatus DD-TD of an embodiment may be a light emitting device having a tandem structure and including a plurality of emission layers.

In an embodiment illustrated in FIG. 8, all light beams respectively emitted from the light emitting structures OL-B1, OL-B2, and OL-B3 may be blue light. However, the embodiment of the present disclosure is not limited thereto, and the light beams respectively emitted from the light emitting structures OL-B1, OL-B2, and OL-B3 may have wavelength ranges different from each other. For example, the light emitting device ED-BT including the plurality of light emitting structures OL-B1, OL-B2, and OL-B3 which emit light beams having wavelength ranges different from each other may emit white light.

A charge generation layers CGL1 and CGL2 may be disposed between two of the neighboring light emitting structures OL-B1, OL-B2, and OL-B3. The charge generation layers CGL1 and CGL2 may include a p-type or kind charge generation layer and/or an n-type or kind charge generation layer.

At least one among the light emitting structures OL-B1, OL-B2, and OL-B3 included in the display apparatus DD-TD of an embodiment may contain the above-described polycyclic compound of an embodiment.

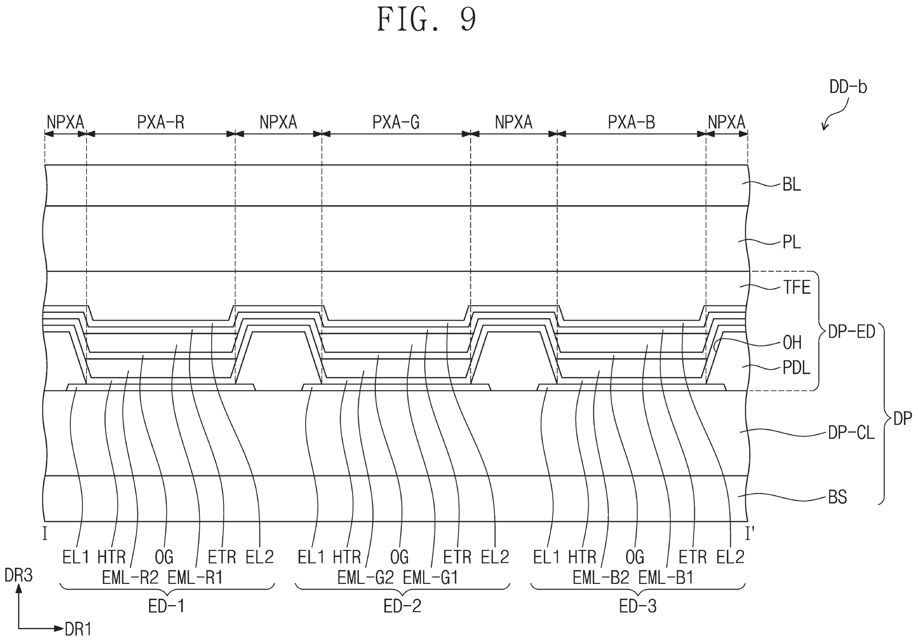

Referring to FIG. 9, the display apparatus DD-b according to an embodiment may include light emitting devices ED-1, ED-2, and ED-3 in which two emission layers are stacked. Compared to the display apparatus DD of an embodiment illustrated in FIG. 2, an embodiment illustrated in FIG. 9 has a difference in that the first to third light emitting devices ED-1, ED-2, and ED-3 each include two emission layers stacked in the thickness direction. In each of the first to third light emitting devices ED-1, ED-2, and ED-3, the two emission layers may emit light in substantially the same wavelength region.

The first light emitting device ED-1 may include a first red emission layer EML-R1 and a second red emission layer EML-R2. The second light emitting device ED-2 may include a first green emission layer EML-G1 and a second green emission layer EML-G2. In some embodiments, the third light emitting device ED-3 may include a first blue emission layer EML-B1 and a second blue emission layer EML-B2. An emission auxiliary part OG may be disposed between the first red emission layer EML-R1 and the second red emission layer EML-R2, between the first green emission layer EML-G1 and the second green emission layer EML-G2, and between the first blue emission layer EML-B1 and the second blue emission layer EML-B2.

The emission auxiliary part OG may include a single layer or a multilayer. The emission auxiliary part OG may include a charge generation layer. For example, the emission auxiliary part OG may include an electron transport region, a charge generation layer, and a hole transport region that are sequentially stacked. The emission auxiliary part OG may be provided as a common layer in the whole of the first to third light emitting devices ED-1, ED-2, and ED-3. However, the embodiment of the present disclosure is not limited thereto, and the emission auxiliary part OG may be provided by being patterned within the openings OH defined in the pixel defining film PDL.

The first red emission layer EML-R1, the first green emission layer EML-G1, and the first blue emission layer EML-B1 may be disposed between the hole transport region HTR and the emission auxiliary part OG. The second red emission layer EML-R2, the second green emission layer EML-G2, and the second blue emission layer EML-B2 may be disposed between the emission auxiliary part OG and the electron transport region ETR.

For example, the first light emitting device ED-1 may include the first electrode EL1, the hole transport region HTR, the second red emission layer EML-R2, the emission auxiliary part OG, the first red emission layer EML-R1, the electron transport region ETR, and the second electrode EL2 that are sequentially stacked. The second light emitting device ED-2 may include the first electrode EL1, the hole transport region HTR, the second green emission layer EML-G2, the emission auxiliary part OG, the first green emission layer EML-G1, the electron transport region ETR, and the second electrode EL2 that are sequentially stacked. The third light emitting device ED-3 may include the first electrode EL1, the hole transport region HTR, the second blue emission layer EML-B2, the emission auxiliary part OG, the first blue emission layer EML-B1, the electron transport region ETR, and the second electrode EL2 that are sequentially stacked.

In some embodiments, an optical auxiliary layer PL may be disposed on the display device layer DP-ED. The optical auxiliary layer PL may include a polarizing layer. The optical auxiliary layer PL may be disposed on the display panel DP and control reflected light in the display panel DP due to external light. Unlike the configuration illustrated, the optical auxiliary layer PL in the display apparatus according to an embodiment may not be provided.

Unlike FIGS. 8 and 9, FIG. 10 illustrates that a display apparatus DD-c includes four light emitting structures OL-B1, OL-B2, OL-B3, and OL-C1. A light emitting device ED-CT may include a first electrode EL1 and a second electrode EL2 which face each other, and first to fourth light emitting structures OL-B1, OL-B2, OL-B3, and OL-C1 that are sequentially stacked in the thickness direction between the first electrode EL1 and the second electrode EL2. Charge generation layers CGL1, CGL2, and CGL3 may be disposed between the first to fourth light emitting structures OL-B1, OL-B2, OL-B3, and OL-C1. Among the four light emitting structures, the first to third light emitting structures OL-B1, OL-B2, and OL-B3 may be to emit blue light, and the fourth light emitting structure OL-C1 may be to emit green light. However, the embodiment of the present disclosure is not limited thereto, and the first to fourth light emitting structures OL-B1, OL-B2, OL-B3, and OL-C1 may be to emit light beams in different wavelength regions.

The charge generation layers CGL1, CGL2, and CGL3 disposed between adjacent light emitting structures OL-B1, OL-B2, OL-B3, and OL-C1 may include a p-type or kind charge generation layer and/or an n-type or kind charge generation layer.

At least one among the light emitting structures OL-B1, OL-B2, OL-B3, and OL-C1 included in the display apparatus DD-c of an embodiment may include the above-described polycyclic compound.

The light emitting device ED according to an embodiment of the present disclosure may include the above-described polycyclic compound in at least one of the hole transport region HTR disposed between the first electrode EL1 and the second electrode EL2, the emission layer EML, or the electron transport region ETR, or in a capping layer CPL.

For example, the light emitting device ED of an embodiment may include the polycyclic compound in the electron transport region ETR, thereby exhibiting excellent or suitable luminous efficiency and long service life characteristics.

For example, the electron transport layer ETL of the electron transport region ETR as described above may include the polycyclic compound to thereby have a refractive index of less than about 1.8 with respect to light having a wavelength of about 450 nm. In some embodiments, the thickness of the electron transport layer ETL satisfies a range of about 250 Å or more.

The light emitting device ED of the present disclosure may have a refractive index of less than about 1.8 with respect to light having a wavelength of about 450 nm and includes the electron transport layer ETL having a thickness of about 250 Å or more, and thus the luminous efficiency and device service life may be improved.

Hereinafter, with reference to Examples and Comparative Examples, a polycyclic compound according to an embodiment of the present disclosure and a light emitting device of an embodiment will be described in more detail. In some embodiments, Examples described are only illustrations to assist the understanding of the present disclosure, and the scope of the present disclosure is not limited thereto.

### Examples

### 1. Synthesis of Polycyclic Compound

First, a synthetic method of a polycyclic compound according to the present embodiment will be described in more detail by illustrating a synthetic method of Compound 1 and Compound 2. In some embodiments, in the following descriptions, the synthetic methods of the polycyclic compounds are provided as examples, but the synthetic method according to an embodiment of the present disclosure is not limited to Examples.

### 1) Synthesis of Compound 1

Compound 1 according to an example may be synthesized by, for example, the steps (e.g., acts or tasks) shown in Reaction Scheme 1:

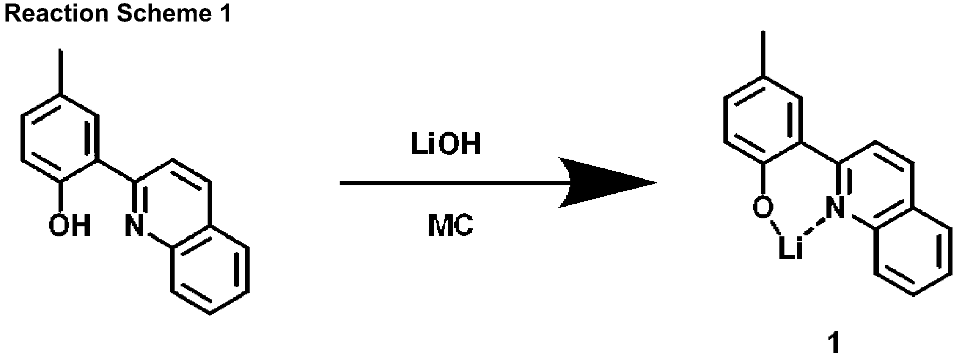

### (1) Synthetic Method of Compound 1

In an argon atmosphere, to a 500 mL round bottom flask, 4-methyl-2-(quinolin-2-yl)phenol (5.00 g, 0.0213 mol) was added and dissolved in 200 mL of methylene chloride (MC) with high purity, and LiOH (0.51 g, 0.0213 mol) was slowly injected thereto with a syringe. After the resulting mixture was stirred at room temperature for about 24 hours, a precipitate was filtered out with a filter, and then the mixture was washed several times with MC. Thereafter, the mixture was dried in a high-vacuum chamber for about two days to obtain Compound 1 (4.88 g, 95%).
H-NMR(DMSO-d6): 8.71(1H, d), 8.15(1H, d), 8.03-8.00 (2H, m), 7.86(1H, t), 7.70(1H, t), 7.29(1H, d), 7.10(1H, d), 6.95(1H, d), 2.40(3H, s),
m/z: 241.11

### 2) Synthesis of Compound 2

Compound 2 according to an example may be synthesized by, for example, the steps shown in Reaction Scheme 2:

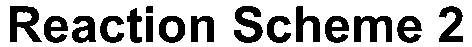

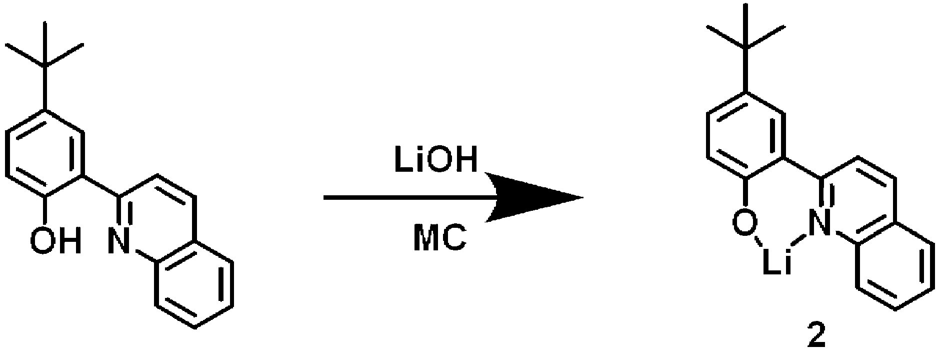

### (1) Synthetic Method of Compound 2

In an argon atmosphere, to a 500 mL round bottom flask, 4-(tert-butyl)-2-(quinolin-2-yl)phenol (5.00 g, 0.0180 mol) was added and dissolved in 200 mL of methylene chloride (MC) with high purity, and LiOH (0.43g, 0.0180 mol) was slowly injected thereto with a syringe. After the resulting mixture was stirred at room temperature for about 24 hours, a precipitate was filtered out with a filter, and then the mixture was washed several times with MC. Thereafter, the mixture was dried in a high-vacuum chamber for about two days to obtain Compound 2 (4.84 g, 95%).
H-NMR(DMSO-d6): 8.72(1H, d), 8.17-8.15 (2H, m), 8.06 (1H, d), 7.86(1H, t), 7.70(1H, t), 7.47(1H, d), 7.29(1H, d), 6.92(1H, d), 1.43(9H, s),
m/z: 283.15

### 2. Manufacture and Evaluation of Light Emitting Device Including Polycyclic Compound

### Manufacture of Light Emitting Device

An electron transport layer including a low refractive material and an electron transport layer material was formed to manufacture each of the light emitting devices of Examples 1 to 4 and Comparative Examples 1 to 8.

The polycyclic compound represented by Formula 1 or Comparative Example Compound 1 was utilized as a low refractive material, and the compound represented by Formula 2 or the compound represented by Formula 3 was utilized as a hole transport layer material.

Compound 1 or Compound 2 was utilized as the polycyclic compound represented by Formula 1. Compound A was utilized as the compound represented by Formula 2. Compound B was utilized as the compound represented by Formula 3.

Compound 1, Compound A, Compound B, and Comparative Example Compound C1 are listed in Table 1.

Each of the electron transport layers included in the light emitting devices of Examples 1 to 4 has a thickness of about 350 Å, and each of the electron transport layers included in the light emitting devices of Comparative Examples 1 to 8 has a thickness of about 350 Å or about 240Å.

**Table 1**

| | |
|---|---|
| Polycyclic compound represented by Formula 1 | 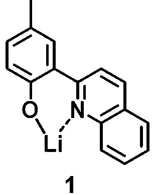 |
| | 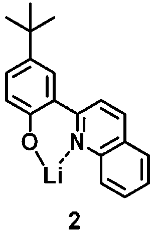 |
| Compound represented by Formula 2 | 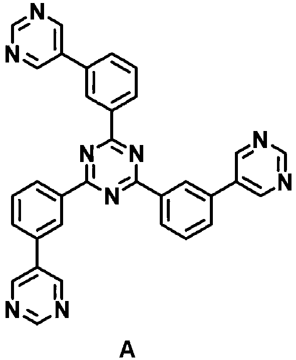 |
| Compound represented by Formula 3 | 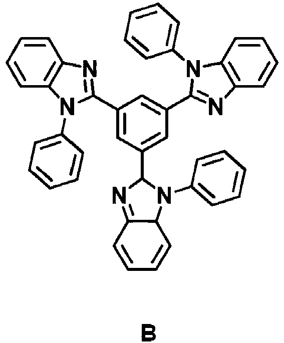 |
| Comparative Example Compound C1 | 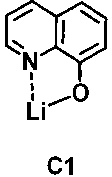 |

### Manufacture of Light Emitting Device

An ITO glass substrate was cut to a size of about 50 mm x 50 mm x 0.7 mm, washed by ultrasonic waves utilizing isopropyl alcohol for about 5 minutes and distilled water for about 5 minutes, respectively, and then irradiated with ultraviolet rays for about 30 minutes and cleansed by exposing to ozone, and then installed on a vacuum deposition apparatus. Thereafter, a hole injection layer having a thickness of about 50 Å was formed of HATCN, and a hole transport layer having a thickness of about 1,200 Å was formed of NPB on the hole injection layer. An electron blocking layer having a thickness of about 50 Å was formed of TCTA on the upper portion of the hole transport layer.

Compound H1 as a host material and Compound D1 as a dopant material were co-deposited at a weight ratio of 99:1 to form a 100 A-thick emission layer.

A hole blocking layer having a thickness of about 50 Å was formed of T2T on the upper portion of the emission layer. Next, any one among Compound 1, Compound 2, and Comparative Example Compound C1 and any one among Compound A and Compound B were co-deposited at a weight ratio of 1:1 to form an electron transport layer. The electron transport layer was formed to have a thickness of about 350 Å or about 240 Å.

A 10 Å-thick electron injection layer was formed of Yb on the upper portion of the electron transport layer. Thereafter, a 100 Å-thick electrode was formed of AgMg.

A 100 Å-thick capping layer was formed of Compound P4 on the upper portion of the electron injection layer.

Each layer was formed by a vacuum deposition method.

The compounds utilized in the manufacture of the light emitting devices are shown below. The following materials/compounds were utilized to manufacture the devices by subjecting commercial products to sublimation purification.

### Compounds Used to Manufacture Devices

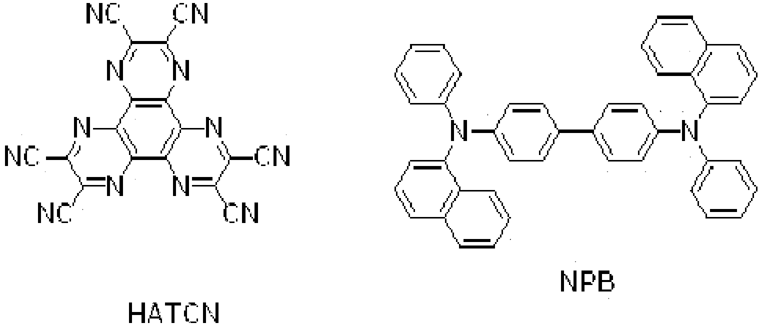

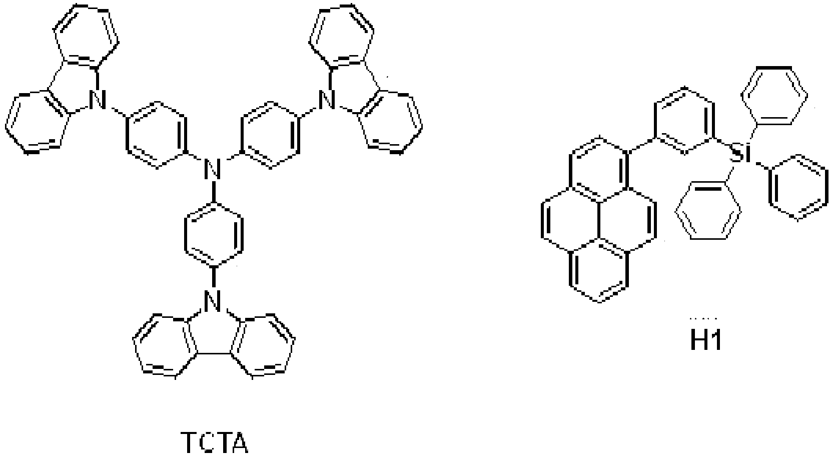

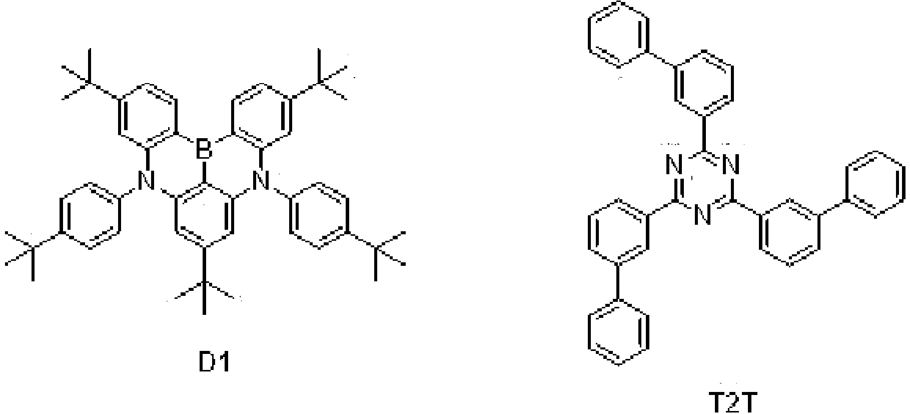

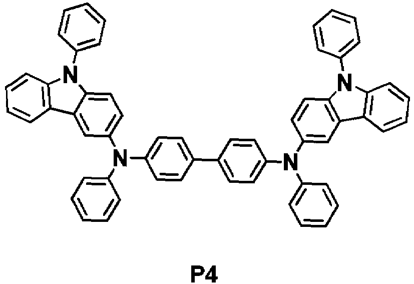

### Evaluation of Light Emitting Device Characteristics 1

Characteristics of the manufactured light emitting devices were evaluated utilizing a brightness light distribution characteristics measurement device. The characteristics of the light emitting devices of Examples 1 to 4 and Comparative Examples 1 and 2 were evaluated and listed in Table 2.

The refractive indices of the electron transport layers included in the light emitting devices and driving voltages, efficiencies, and service lives of the light emitting devices were measured and listed in Table 2.

The electron transport layer included in each light emitting device of Examples 1 to 4 and Comparative Examples 1 and 2 has a thickness of about 350 Å. For example, the thickness of the electron transport layer included in each light emitting device of Examples 1 to 4 and Comparative Examples 1 and 2 satisfies a value of about 250 Å or more.

The driving voltage (V) and efficiency (cd/A) in Table 2 were measured at a current density of 10 mA/cm² and a brightness of 1,000 cd/m². Service life ratio was indicated by measuring time for the brightness to be deteriorated to a brightness of 95% with respect to the initial brightness, and listed are relative values when the service life of Comparative Example 1 is 120.

**Table 2**

| Division | Electron transport layer Composition | | Thickness of electron transport layer (Å) | Refractive index of electron transport layer | Driving voltage (V) | Efficiency (cd/A) | Service life ratio (T95) |
|---|---|---|---|---|---|---|---|
| | Low refractive material | Electron transport material | | | | | |
| Example 1 | Compound 1 | Compound B | 350 | 1.78 | 3.8 | 7.0 | 120 |
| Example 2 | Compound 1 | Compound A | 350 | 1.74 | 3.9 | 7.2 | 128 |
| Example 3 | Compound 2 | Compound B | 350 | 1.73 | 3.9 | 7.2 | 130 |
| Example 4 | Compound 2 | Compound A | 350 | 1.72 | 4.0 | 7.4 | 131 |
| Comparative Example 1 | Comparative Example Compound C1 | Compound B | 350 | 1.83 | 3.8 | 6.8 | 120 |
| Comparative Example 2 | Comparative Example Compound C1 | Compound A | 350 | 1.8 | 4.0 | 6.7 | 115 |

Referring to the results of Table 2, for each light emitting device of Examples 1 to 4, the electron transport layer has a refractive index of less than about 1.8, and the thickness of the electron transport layer satisfies a range of about 250 Å or more. For each light emitting device of Comparative Examples 1 and 2, the electron transport layer has a refractive index of about 1.8 or more, and the thickness of the electron transport layer satisfies a range of about 250 Å or more.

Comparing the light emitting devices of Example 1, Example 3, and Comparative Example 1, the light emitting device of Comparative Example 1 includes Comparative Example Compound C1 as a low refractive material, and thus the electron transport layer has a refractive index of about 1.8 or more. Accordingly, the light emitting device of Comparative Example 1 exhibits lower efficiency than the light emitting device of Example 1, and exhibits shorter service life than the light emitting device of Example 3.

Comparing the light emitting devices of Example 2, Example 4, and Comparative Example 2, the light emitting device of Comparative Example 2 includes Comparative Example Compound C1 as a low refractive material, and thus the electron transport layer has a refractive index of about 1.8 or more. Accordingly, the light emitting device of Comparative Example 2 exhibits lower efficiency and shorter service life than the light emitting devices of Examples 2 and 4.

The refractive index of the polycyclic compound represented by Formula 1 of the present disclosure may satisfy a range of less than about 1.7. The electron transport layer of the present disclosure includes the polycyclic compound, and thus the refractive index may satisfy a range of less than about 1.8.

The light emitting device of the present disclosure includes the electron transport layer having a refractive index of less than about 1.8, and thus the efficiency and service life characteristics may be improved.

### Evaluation of Light Emitting Device Characteristics 2

Characteristics of the manufactured light emitting devices were evaluated utilizing a brightness light distribution characteristics measurement device. The characteristics of the light emitting devices of Examples 1 to 4 and Comparative Examples 3 to 8 are evaluated and listed in Table 3.

The refractive indices of the electron transport layers included in the light emitting devices and driving voltages, efficiencies, and service lives of the light emitting devices are measured and listed in Table 3.

The electron transport layer included in each light emitting device of Examples 1 to 4 has a thickness of about 350 Å. For example, the thickness of the electron transport layer included in each light emitting device of Examples 1 to 4 satisfies a value of about 250 Å or more. The evaluation items (properties) of the light emitting devices of Examples 1 to 4 listed in Table 3 may each independently be the same as the evaluation items (properties) of the light emitting devices of Examples 1 to 4 listed in Table 2.

The electron transport layer included in the light emitting device of each of Comparative Example 3 to 8 has a thickness of about 240 Å. For example, the thickness of the electron transport layer in each light emitting device of Comparative Examples 3 to 8 does not satisfy a range of about 250 Å or more.

The driving voltage (V) and efficiency (cd/A) in Table 3 were measured at a current density of 10 mA/cm² and a brightness of 1,000 cd/m². Service life ratio was determined by measuring the time for the brightness to deteriorate to a brightness of 95% with respect to the initial brightness, and listed are relative values when the service life of Comparative Example 1 is 120.

**Table 3**

| Division | Electron transport layer Composition | | Thickness of electron transport layer (Å) | Refractive index of electron transport layer | Driving voltage (V) | Efficiency (cd/A) | Service life ratio (T95) |
|---|---|---|---|---|---|---|---|
| | Low refractive material | Electron transport material | | | | | |
| Example 1 | Compound 1 | Compound B | 350 | 1.78 | 3.8 | 7.0 | 120 |
| Example 2 | Compound 1 | Compound | 350 | 1.74 | 3.9 | 7.2 | 128 |
| | | A | | | | | |
| Example 3 | Compound 2 | Compound B | 350 | 1.73 | 3.9 | 7.2 | 130 |
| Example 4 | Compound 2 | Compound A | 350 | 1.72 | 4.0 | 7.4 | 131 |
| Comparative Example 3 | Compound 1 | Compound B | 240 | 1.78 | 3.7 | 6.8 | 113 |
| Comparative Example 4 | Compound 1 | Compound A | 240 | 1.74 | 3.8 | 6.9 | 125 |
| Comparative Example 5 | Compound 2 | Compound B | 240 | 1.73 | 3.8 | 6.9 | 125 |
| Comparative Example 6 | Compound 2 | Compound A | 240 | 1.72 | 3.9 | 7.0 | 128 |
| Comparative Example 7 | Comparative Example Compound C1 | Compound B | 240 | 1.83 | 3.7 | 6.7 | 115 |
| Comparative Example 8 | Comparative Example Compound C1 | Compound A | 240 | 1.8 | 3.9 | 6.7 | 110 |

Referring to the results of Table 3, for each light emitting device of Examples 1 to 4, the electron transport layer has a refractive index of less than about 1.8, and the thickness of the electron transport layer satisfies a range of about 250 Å or more. Comparing the light emitting device of Example 1 to Comparative Example 3, for the light emitting device of Comparative Example 3, the electron transport layer has a thickness of less than about 250 Å. Accordingly, although the refractive index of the electron transport layer included in the light emitting device of Comparative Example 3 is less than about 1.8, the light emitting device of Comparative Example 3 exhibits lower efficiency and a shorter service life than the light emitting device of Example 1.

Comparing the light emitting device of Example 2 to Comparative Example 4, for the light emitting device of Comparative Example 4, the electron transport layer has a thickness of less than about 250 Å. Accordingly, although the refractive index of the electron transport layer included in the light emitting device of Comparative Example 4 is less than about 1.8, the light emitting device of Comparative Example 4 exhibits lower efficiency and shorter service life than the light emitting device of Example 2.

Comparing the light emitting devices of Example 3 to Comparative Example 5, for the light emitting device of Comparative Example 5, the electron transport layer has a thickness of less than about 250 Å. Accordingly, although the refractive index of the electron transport layer included in the light emitting device of Comparative Example 5 is less than about 1.8, the light emitting device of Comparative Example 5 exhibits lower efficiency and shorter service life than the light emitting device of Example 3.

Comparing the light emitting devices of Example 4 to Comparative Example 6, for the light emitting device of Comparative Example 6, the electron transport layer has a thickness of less than about 250 Å. Accordingly, although the refractive index of the electron transport layer included in the light emitting device of Comparative Example 6 is less than about 1.8, the light emitting device of Comparative Example 6 exhibits lower efficiency and a shorter service life than the light emitting device of Example 4.

Comparing the light emitting devices of Examples 1 to 4 to Comparative Examples 7 and 8, for the light emitting devices of Comparative Examples 7 and 8, the electron transport layer has a refractive index of about 1.8 or more, and the electron transport layer has a thickness of less than about 250 Å. Accordingly, the light emitting devices of Comparative Examples 7 and 8 exhibit lower efficiency and a shorter service life than the light emitting devices of Examples 1 to 4.

For the electron transport layer included in the light emitting device of the present disclosure, the refractive index satisfies a range of less than about 1.8, and the thickness satisfies a range of about 250 Å or more, and thus the efficiency and service life characteristics of the light emitting device may be improved.

### Evaluation of Light Emitting Device Efficiency

Mode analysis of the light emitting devices of Examples 1 to 4 and Comparative Examples 1 and 2 in Table 2 were evaluated, and the results are listed in Table 4. The mode analysis with respect to light having a wavelength of about 450 nm which is emitted from the emission layer of each light emitting device of Examples 1 to 4, and Comparative Examples 1 and 2 were evaluated by utilizing an in-house optical simulator, and the results are listed in Table 4.

In some embodiments, the electron transport layer composition and the thickness of the electron transport layer included in each light emitting device of Examples 1 to 4 and Comparative Examples 1 and 2 may each independently be the same as those defined in Table 2.

**Table 4**

| Division | Refractive index of electron transport layer | Outcoupling Efficiency (%) | Substrate (%) | Absorbed (%) | Waveguided modes (%) | Surface plasmon (%) |
|---|---|---|---|---|---|---|
| Example 1 | 1.78 | 27 | 25 | 5 | 23 | 20 |
| Example 2 | 1.74 | 28 | 25 | 5 | 22 | 20 |
| Example 3 | 1.73 | 29 | 25 | 5 | 21 | 20 |
| Example 4 | 1.72 | 30 | 25 | 5 | 20 | 20 |
| Comparative Example 1 | 1.83 | 25 | 25 | 5 | 25 | 20 |
| Comparative Example 2 | 1.8 | 26 | 25 | 5 | 24 | 20 |

Referring to the results of Table 4, it may be seen that the outcoupling efficiency of each light emitting device of Examples 1 to 4 is higher than that of each light emitting device of Comparative Examples 1 and 2. This suggests that the refractive index of the electron transport layer included in each light emitting device of Examples 1 to 4 satisfies a range of less than about 1.8, and thus the quantity of light which is lost in waveguided modes in the emitted light from the emission layer is reduced. Each light emitting device of Comparative Examples 1 and 2 includes the electron transport layer having a thickness of about 250 Å or more. However, it is thought that each light emitting device of Comparative Examples 1 and 2 includes the electron transport layer having a refractive index of about 1.8 or more, and thus the quantity of light which is lost in waveguided modes is more than that of the light emitting devices of Examples 1 to 4, and the outcoupling efficiency is reduced.

Each light emitting device of Examples 1 to 4 has the outcoupling efficiency higher than that of each light emitting device of Comparative Examples 1 and 2, and thus the luminous efficiency may be improved (increased).

The polycyclic compound represented by Formula 1 according to an embodiment of the present disclosure has a refractive index of less than about 1.7 with respect to light having a wavelength of about 450 nm, and thus when the polycyclic compound is included in the electron transport layer, the refractive index of the electron transport layer may be reduced to be less than about 1.8.

The electron transport layer of the present disclosure may include the polycyclic compound represented by Formula 1 and the electron transport material. The electron transport layer of the present disclosure has a refractive index of less than about 1.8, and a thickness of about 250 Å or more.

The light emitting device of the present disclosure includes the electron transport layer, and thus the efficiency and service life of the device may be improved (increased).

The light emitting device of an embodiment may exhibit improved device characteristics with high efficiency and a long service life.

The polycyclic compound of an embodiment may be included in an electron transport layer of the light emitting device to contribute to high efficiency and a long service life of the light emitting device.

The use of "may" when describing embodiments of the present disclosure refers to "one or more embodiments of the present disclosure."

As used herein, the term "substantially," "about," and similar terms are used as terms of approximation and not as terms of degree, and are intended to account for the inherent deviations in measured or calculated values that would be recognized by those of ordinary skill in the art. "About" or "approximately," as used herein, is inclusive of the stated value and means within an acceptable range of deviation for the particular value as determined by one of ordinary skill in the art, considering the measurement in question and the error associated with measurement of the particular quantity (i.e., the limitations of the measurement system). For example, "about" may mean within one or more standard deviations, or within ± 30%, 20%, 10%, 5% of the stated value.

Also, any numerical range recited herein is intended to include all sub-ranges of the same numerical precision subsumed within the recited range. For example, a range of "1.0 to 10.0" is intended to include all subranges between (and including) the recited minimum value of 1.0 and the recited maximum value of 10.0, that is, having a minimum value equal to or greater than 1.0 and a maximum value equal to or less than 10.0, such as, for example, 2.4 to 7.6. Any maximum numerical limitation recited herein is intended to include all lower numerical limitations subsumed therein and any minimum numerical limitation recited in this disclosure is intended to include all higher numerical limitations subsumed therein. Accordingly, Applicant reserves the right to amend this disclosure, including the claims, to expressly recite any sub-range subsumed within the ranges expressly recited herein.

The light emitting device or any other relevant devices or components according to embodiments of the present disclosure described herein may be implemented utilizing any suitable hardware, firmware (e.g., an application-specific integrated circuit), software, or a combination of software, firmware, and hardware. For example, the various components of the device may be formed on one integrated circuit (IC) chip or on separate IC chips. Further, the various components of the device may be implemented on a flexible printed circuit film, a tape carrier package (TCP), a printed circuit board (PCB), or formed on one substrate. Further, the various components of the device may be a process or thread, running on one or more processors, in one or more computing devices, executing computer program instructions and interacting with other system components for performing the various functionalities described herein. The computer program instructions are stored in a memory which may be implemented in a computing device using a standard memory device, such as, for example, a random access memory (RAM). The computer program instructions may also be stored in other non-transitory computer readable media such as, for example, a CD-ROM, flash drive, or the like. Also, a person of skill in the art should recognize that the functionality of various computing devices may be combined or integrated into a single computing device, or the functionality of a particular computing device may be distributed across one or more other computing devices without departing from the scope of the embodiments of the present disclosure.

Although the present disclosure has been described with reference to preferred embodiments of the present disclosure, it will be understood that the present disclosure should not be limited to these preferred embodiments but one or more suitable changes and modifications can be made by those skilled in the art without departing from the scope of the present disclosure as defined by the following claims.

Accordingly, the technical scope of the present disclosure is not intended to be limited to the contents set forth in the detailed description of the disclosure, but is intended to be defined by the appended claims.

The following are clauses describing embodiments of the invention. They are not claims.

Clause 1. A polycyclic compound represented by Formula 1: 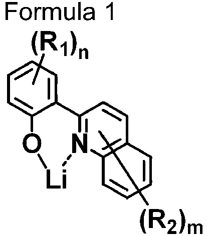 wherein, in Formula 1,
R₁ and R₂ are each independently a hydrogen atom, a deuterium atom, a halogen atom, a substituted or unsubstituted silyl group, a substituted or unsubstituted amine group, a substituted or unsubstituted alkyl group having 1 to 20 carbon atoms, a substituted or unsubstituted alkenyl group having 2 to 20 carbon atoms, a substituted or unsubstituted aryl group having 6 to 30 ring-forming carbon atoms, or a substituted or unsubstituted heteroaryl group having 2 to 30 ring-forming carbon atoms, and optionally may be bonded to an adjacent group to form a ring,
n is an integer of 0 to 4, and
m is an integer of 0 to 6.

Clause 2. The polycyclic compound of Clause 1, wherein the polycyclic compound is represented by Formula 1-1: 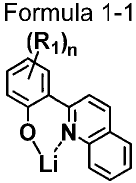 wherein, in Formula 1-1, R₁ and n are the same as defined in claim 1.

Clause 3. The polycyclic compound of Clause 1 or Clause 2, wherein the polycyclic compound is represented by Formula 1-2: 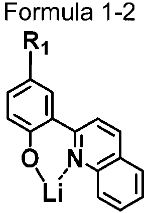 wherein, in Formula 1-2, R₁ is the same as defined in claim 1.

Clause 4. The polycyclic compound of any one of Clauses 1 to 3, wherein R₁ is a substituted or unsubstituted alkyl group having 1 to 20 carbon atoms.

Clause 5. The polycyclic compound of any one of Clauses 1 to 4, wherein the polycyclic compound comprises at least one compound represented in Compound Group 1:

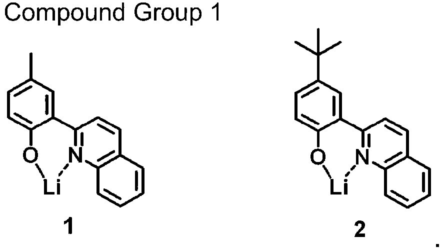

Clause 6. A light emitting device comprising:
a first electrode;
a hole transport region on the first electrode;
an emission layer on the hole transport region;
an electron transport region on the emission layer; and
a second electrode on the electron transport region,
wherein the electron transport region comprises a polycyclic compound according to any one of Clauses 1 to 5.

Clause 7. The light emitting device of Clause 6, further comprising at least one compound represented by Formula 2 and/or Formula 3: 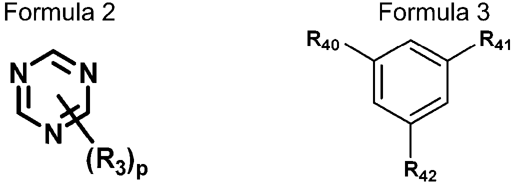 wherein, in Formula 2,
each R₃ is independently a hydrogen atom, a deuterium atom, a halogen atom, a substituted or unsubstituted silyl group, a substituted or unsubstituted amine group, a substituted or unsubstituted alkyl group having 1 to 20 carbon atoms, a substituted or unsubstituted alkenyl group having 2 to 20 carbon atoms, a substituted or unsubstituted aryl group having 6 to 30 ring-forming carbon atoms, or a substituted or unsubstituted heteroaryl group having 2 to 30 ring-forming carbon atoms, and optionally may be bonded to an adjacent group to form a ring, and
p is an integer of 0 to 3; and
wherein, in Formula 3,
R₄₀ to R₄₂ are each independently a hydrogen atom, a deuterium atom, a halogen atom, a cyano group, a substituted or unsubstituted silyl group, a substituted or unsubstituted thiol group, a substituted or unsubstituted oxy group, a substituted or unsubstituted amine group, a substituted or unsubstituted alkyl group having 1 to 20 carbon atoms, a substituted or unsubstituted alkenyl group having 2 to 20 carbon atoms, a substituted or unsubstituted aryl group having 6 to 30 ring-forming carbon atoms, or a substituted or unsubstituted heteroaryl group having 2 to 30 ring-forming carbon atoms, and optionally may be bonded to an adjacent group to form a ring.

Clause 8. The light emitting device of Clause 6 or 7, wherein the electron transport region comprises a hole blocking layer on the emission layer, an electron transport layer on the hole blocking layer, and an electron injection layer on the electron transport layer, and wherein the electron transport layer comprises the polycyclic compound according to any one of Clauses 1 to 5.

Clause 9. The light emitting device of Clause 8, wherein the electron transport layer has a thickness of about 250 Å or more.

Clause 10. The light emitting device of Clause 8 or Clause 9, wherein the electron transport layer has a refractive index of less than about 1.8.

Clause 11. The light emitting device of any one of Clauses 6 to 10, wherein the emission layer is configured to emit light having a center wavelength of about 430 nm to about 470 nm.

## Claims

1. A light emitting device (ED) comprising:
a first electrode (EL1);
a second electrode (EL2) on the first electrode; and
at least one functional layer between the first electrode and the second electrode,
wherein the at least one functional layer comprises a polycyclic compound represented by Formula 1: 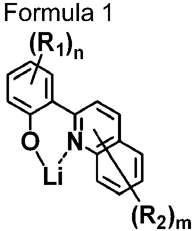
wherein, in Formula 1,
R₁ is a halogen atom, a substituted or unsubstituted silyl group, a substituted or unsubstituted amine group, a substituted or unsubstituted alkyl group having 1 to 20 carbon atoms, a substituted or unsubstituted alkenyl group having 2 to 20 carbon atoms, a substituted or unsubstituted aryl group having 6 to 30 ring-forming carbon atoms, or a substituted or unsubstituted heteroaryl group having 2 to 30 ring-forming carbon atoms,
R₂ is a hydrogen atom, a deuterium atom, a halogen atom, a substituted or unsubstituted silyl group, a substituted or unsubstituted amine group, a substituted or unsubstituted alkyl group having 1 to 20 carbon atoms, a substituted or unsubstituted alkenyl group having 2 to 20 carbon atoms, a substituted or unsubstituted aryl group having 6 to 30 ring-forming carbon atoms, or a substituted or unsubstituted heteroaryl group having 2 to 30 ring-forming carbon atoms,
n is 1, and
m is an integer of 0 to 6.

2. The light emitting device of claim 1, wherein the polycyclic compound is represented by Formula 1-1: 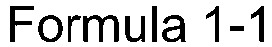 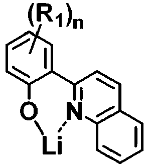 wherein, in Formula 1-1, R₁ and n are the same as defined in claim 1.

3. The light emitting device of claim 1 or claim 2, wherein the polycyclic compound is represented by Formula 1-2: 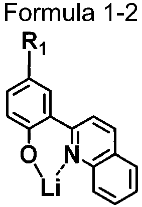 wherein, in Formula 1-2, R₁ is the same as defined in claim 1.

4. The light emitting device of any one of claims 1 to 3, wherein R₁ is a substituted or unsubstituted alkyl group having 1 to 20 carbon atoms.

5. The light emitting device of any one of claims 1 to 4, wherein the polycyclic compound comprises at least one compound represented in Compound Group 1:

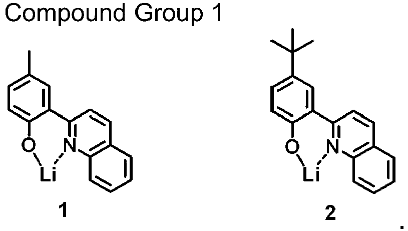

6. The light emitting device of any one of claims 1 to 5, wherein the at least one functional layer further comprises at least one compound represented by Formula 2 and/or Formula 3: 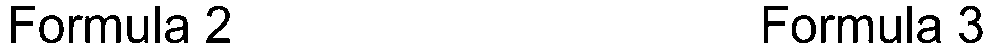 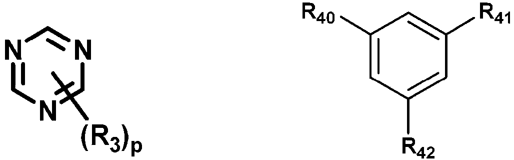 wherein, in Formula 2,
each R₃ is independently a hydrogen atom, a deuterium atom, a halogen atom, a substituted or unsubstituted silyl group, a substituted or unsubstituted amine group, a substituted or unsubstituted alkyl group having 1 to 20 carbon atoms, a substituted or unsubstituted alkenyl group having 2 to 20 carbon atoms, a substituted or unsubstituted aryl group having 6 to 30 ring-forming carbon atoms, or a substituted or unsubstituted heteroaryl group having 2 to 30 ring-forming carbon atoms, and optionally may be bonded to an adjacent group to form a ring, and
p is an integer of 0 to 3; and
wherein, in Formula 3,
R₄₀ to R₄₂ are each independently a hydrogen atom, a deuterium atom, a halogen atom, a cyano group, a substituted or unsubstituted silyl group, a substituted or unsubstituted thiol group, a substituted or unsubstituted oxy group, a substituted or unsubstituted amine group, a substituted or unsubstituted alkyl group having 1 to 20 carbon atoms, a substituted or unsubstituted alkenyl group having 2 to 20 carbon atoms, a substituted or unsubstituted aryl group having 6 to 30 ring-forming carbon atoms, or a substituted or unsubstituted heteroaryl group having 2 to 30 ring-forming carbon atoms, and optionally may be bonded to an adjacent group to form a ring.

7. The light emitting device of any claims 1 to 6, wherein the at least one functional layer comprises:
a first light emitting structure (OL-B3) on the first electrode;
a second light emitting structure (OL-B2) on the first light emitting structure;
a third light emitting structure (OL-B1) on the second light emitting structure; and
a fourth light emitting structure (OL-C1) on the third light emitting structure.

8. The light emitting device of claim 7, wherein at least one among the first to fourth light emitting structures comprises the polycyclic compound.

9. The light emitting device of claim 7 or 8, wherein the first to third light emitting structures emit blue light, and the fourth light emitting structure emit green light.

10. The light emitting device of any claims 7 to 9, wherein the at least one functional layer further comprises:
a first charge generation layer (CGL3) between the first light emitting structure and the second light emitting structure;
a second charge generation layer (CGL2) between the second light emitting structure and the third light emitting structure;
a third charge generation layer (CGL1) between the third light emitting structure and the fourth light emitting structure.

11. A display apparatus (DD-c) comprising:
a display panel (DP) comprising the light emitting device according to any one of claims 1 to 10, wherein a first light emitting region (PXA-R), a second light emitting region (PXA-G), a third light emitting region (PXA-B), and non-light emitting region (NPXA) surrounding the first to third light emitting regions are defined;
a light control layer (CCL) on the display panel; and
a color filter layer (CFL) on the light control layer.

12. The display apparatus of claim 11, wherein the light control layer comprises:
a plurality of light control parts (CCP1, CCP2, CCP3); and
divided patterns (BMP) between the light control parts.

13. The display apparatus of claim 12, wherein the plurality of light control parts comprises:
a first light control part (CCP1) overlapping the first light emitting region and comprising a first quantum dot (QD1) configured to convert first color light provided from the light emitting device into second color light;
a second light control part (CCP2) overlapping the second light emitting region and comprising a second quantum dot (QD2) configured to convert the first color light into third color light; and
a third light control part (CCP3) overlapping the third light emitting region and configured to transmit the first color light.

14. The display apparatus of claim 13, wherein the first quantum dot is a red quantum dot, and the second quantum dot is a green quantum dot.

15. The display apparatus of any claims 11 to 14, wherein the first light emitting region is a red light emitting region, the second light emitting region is a green light emitting region, and the third light emitting region is a blue light emitting region.
